# EUROPEAN PATENT SPECIFICATION

(11) **EP 0 980 429 B1**
(45) Date of publication and mention of the grant of the patent: **13.12.2006**
(21) Application number: 98918934.5
(22) Date of filing: 07.05.1998
(51) Int. Cl.: C12N 15/12, C12N 15/62, C07K 14/705, C07K 16/28, C12N 15/70

(54) **HUMAN TOLL-LIKE RECEPTOR PROTEINS, RELATED REAGENTS AND METHODS**
MENSCHLICHE TOLL-ÄHNLICHE REZEPTORPROTEINE, ZUGEHÖRIGE REAGENZIEN UND VERFAHREN
RECEPTEUR PROTEIQUE HUMAIN DU TYPE TOLL, REACTIFS CONNEXES ET TECHNIQUES AFFERENTES

(30) Priority: 07.05.1997 US 44293 P; 22.01.1998 US 72212 P; 05.03.1998 US 76947 P
(43) Date of publication of application: 23.02.2000
(73) Proprietor: SCHERING CORPORATION, Kenilworth, New Jersey 07033 (US)
(72) Inventor: HARDIMAN, Gerard, T., Watertown, MA 02172 (US); ROCK, Fernando, L., Foster City, CA 94404 (US); BAZAN, J., Fernando, Menlo Park, CA 94025 (US); KASTELEIN, Robert, A., Redwood City, CA 94062 (US)
(74) Representative: Naylor, Kathryn May
(86) International application number: PCT/US1998/008979
(87) International publication number: WO 1998/050547

(56) References cited:
- WO-A-98/02557
- NOMURA N ET AL: "PREDICTION OF THE CODING SEQUENCES OF UNIDENTIFIED HUMAN GENES I. THE CODING SEQUENCES OF 40 NEW GENES (KIAA0001-KIAA0040) DEDUCEDBY ANALYSIS OF RANDOMLY SAMPLED CDNA CLONES FROM HUMAN IMMATURE MYELOID CELL LINE KG-1" DNA RESEARCH, vol. 1, no. 1, 1994, pages 27-35, XP002049267 cited in the application -& DATABASE EMBL - EMHUM2 Entry HSRSC786, Acc.No. D13637, 31 March 1993 NOMURA, N.: "Human mRNA for KIAA0012 gene, complete cds." XP002082890
- MEDZHITOV R ET AL: "A HUMAN HOMOLOGUE OF THE DROSOPHILA TOLL PROTEIN SIGNALS ACTIVATION OF ADAPTIVE IMMUNITY" NATURE, vol. 388, 24 July 1997, pages 394-396, XP002056592 & DATABASE EMBL - EMHUM2 Entry HSU93091, Acc.No. U93091, 11 April 1997 "Human Toll protein homolog mRNA, complete cds and LINE-1 reverse transcriptase homolog, pseudogene." XP002073841
- DATABASE EMBL - EMEST11 Entry HSA05049, Acc.No. AA005049, 25 July 1996 HILLIER, L. ET AL.: "zh96c04.r1 Soares fetal liver spleen 1NFLS S1 Homo sapiens cDNA clone 429126 5'." XP002073842
- DATABASE EMBL - EMEST8 Entry HS150139, Acc.No. R76150, 10 June 1995 HILLIER, L. ET AL.: "yi71d02.r1 Homo sapiens cDNA clone 144675 5'." XP002082885
- DATABASE EMBL - EMEST7 Entry HS021274, Acc.No. N41021, 27 January 1996 HILLIER, L. ET AL.: "yy53b03.s1 Homo sapiens cDNA clone 277229 3'." XP002082886
- DATABASE EMBL - EMEST13 Entry HSC3991, Acc.No. C01399, 17 July 1996 OKUBO, K.: "HUMGS0008381, Human Gene Signature, 3'-direction cDNA sequence." XP002082887
- ADAMS M D ET AL: "INITIAL ASSESSMENT OF HUMAN GENE DIVERSITY AND EXPRESSION PATTERNS BASED UPON 83 MILLION NUCLEOTIDES OF CDNA SEQUENCE" NATURE, vol. 377, 28 September 1995, pages 3-17, XP002042918 -& DATABASE EMBL - EMEST14 Entry HSZZ86976, Acc.No. AA381849, 18 April 1997 ADAMS, M.D. ET AL.: "EST94973 Activated T-cells I Homo sapiens cDNA 5' end similar to H. sapiens hypothetical protein (GP:D13637_1)." XP002082891
- DATABASE EMBL - EMEST7 Entry HS1167131, Acc.No. AA252405, 15 March 1997 STRAUSBERG, R.: "zs12e09.r1 NCI_CGAP_GCB1 Homo sapiens cDNA clone IMAGE:685000 5'." XP002082888
- HARDIMAN, G. ET AL.: "Molecular characterization and modular analysis of human MyD88." ONCOGENE, vol. 13, no. 11, 1996, pages 2467-75, XP002073838
- TAGUCHI, T. ET AL.: "Chromosomal localization of TIL, a gene encoding a protein related to the Drosophila transmembrane receptor Toll, to human chromosome 4p14" GENOMICS, vol. 32, 1996, pages 486-8, XP002073839 cited in the application
- ROCK, F.L. ET AL.: "A family of human receptors structurally related to Drosophila Toll." PROC.NATL.ACAD.SCI.USA, vol. 95, January 1998, pages 588-93, XP002073840
- DATABASE EMBL - EMHUM1 Entry AC003046, Acc.No. AC003046, 8 November 1997 MUNZY, D. ET AL.: "Homo sapiens Xp22 PACs RPC11-263P4 and RPC11-164K3 complete sequence." XP002082889

### SEQUENCE LISTING

(1) GENERAL INFORMATION:
   (i) APPLICANT: (A) NAME: Schering Corporation
      (B) STREET: 2000 Galloping Hill Road
      (C) CITY: Kenilworth
      (D) STATE: New Jersey
      (E) COUNTRY: USA
      (F) POSTAL CODE: 07033
      (G) TELEPHONE: (908) 298-4000
      (H) TELEFAX: (908) 298-5388
   (ii) TITLE OF INVENTION: HUMAN RECEPTOR PROTEINS; RELATED REAGENTS AND METHODS
   (iii) NUMBER OF SEQUENCES: 35
   (iv) COMPUTER READABLE FORM:
      (A) MEDIUM TYPE: Floppy disk
      (B) COMPUTER: Macintosh Power PC
      (C) OPERATING SYSTEM: 8.0
      (D) SOFTWARE: Microsoft Word 6.0
   (v) CURRENT APPLICATION DATA:
      (A) APPLICATION NUMBER:
      (B) FILING DATE:
      (C) CLASSIFICATION:
   (vi) PRIOR APPLICATION DATA:
      (A) APPLICATION NO.: USSN 60/044,293
      (B) FILING DATE: 07-MAY-1997

      (A) APPLICATION NO.: USSN 60/072,212
      (B) FILING DATE: 22-JAN-1998

      (A) APPLICATION NO.: USSN 60/076,947
      (B) FILING DATE: 05-MAR-1998
(2) INFORMATION FOR SEQ ID NO:1:
   (i) SEQUENCE CHARACTERISTICS:
      (A) LENGTH: 2367 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..2358
   (ix) FEATURE:
      (A) NAME/KEY: mat_peptide
      (B) LOCATION: 67..2358
   (xi) SEQUENCE DESCRIPTION: SEQ ID NO:1:
   ATG ACT AGC ATC TTC CAT TTT GCC ATT ATC TTC ATG TTA ATA CTT CAG         48
   Met Thr Ser Ile Phe His Phe Ala Ile Ile Phe Met Leu Ile Leu Gln
   -22     -20                 -15                 -10
   ATC AGA ATA CAA TTA TCT GAA GAA AGT GAA TTT TTA GTT GAT AGG TCA         96
   Ile Arg Ile Gln Leu Ser Glu Glu Ser Glu Phe Leu Val Asp Arg Ser
        -5                   1               5                  10
   AAA AAC GGT CTC ATC CAC GTT CCT AAA GAC CTA TCC CAG AAA ACA ACA        144
   Lys Asn Gly Leu Ile His Val Pro Lys Asp Leu Ser Gln Lys Thr Thr
                    15                  20                  25
   ATC TTA AAT ATA TCG CAA AAT TAT ATA TCT GAG CTT TGG ACT TCT GAC        192
   Ile Leu Asn Ile Ser Gln Asn Tyr Ile Ser Glu Leu Trp Thr Ser Asp
                30                  35                  40
   ATC TTA TCA CTG TCA AAA CTG AGG ATT TTG ATA ATT TCT CAT AAT AGA        240
   Ile Leu Ser Leu Ser Lys Leu Arg Ile Leu Ile Ile Ser His Asn Arg
            45                  50                  55
   ATC CAG TAT CTT GAT ATC AGT GTT TTC AAA TTC AAC CAG GAA TTG GAA        288
   Ile Gln Tyr Leu Asp Ile Ser Val Phe Lys Phe Asn Gln Glu Leu Glu
        60                  65                  70
   TAC TTG GAT TTG TCC CAC AAC AAG TTG GTG AAG ATT TCT TGC CAC CCT        336
   Tyr Leu Asp Leu Ser His Asn Lys Leu Val Lys Ile Ser Cys His Pro
    75                  80                  85                  90
   ACT GTG AAC CTC AAG CAC TTG GAC CTG TCA TTT AAT GCA TTT GAT GCC        384
   Thr Val Asn Leu Lys His Leu Asp Leu Ser Phe Asn Ala Phe Asp Ala
                    95                 100                 105
   CTG CCT ATA TGC AAA GAG TTT GGC AAT ATG TCT CAA CTA AAA TTT CTG        432
   Leu Pro Ile Cys Lys Glu Phe Gly Asn Met Ser Gln Leu Lys Phe Leu
               110                 115                 120
   GGG TTG AGC ACC ACA CAC TTA GAA AAA TCT AGT GTG CTG CCA ATT GCT        480
   Gly Leu Ser Thr Thr His Leu Glu Lys Ser Ser Val Leu Pro Ile Ala
           125                 130                 135
   CAT TTG AAT ATC AGC AAG GTC TTG CTG GTC TTA GGA GAG ACT TAT GGG        528
   His Leu Asn Ile Ser Lys Val Leu Leu Val Leu Gly Glu Thr Tyr Gly
       140                 145                 150
   GAA AAA GAA GAC CCT GAG GGC CTT CAA GAC TTT AAC ACT GAG AGT CTG        576
   Glu Lys Glu Asp Pro Glu Gly Leu Gln Asp Phe Asn Thr Glu Ser Leu
   155                 160                 165                 170
   CAC ATT GTG TTC CCC ACA AAC AAA GAA TTC CAT TTT ATT TTG GAT GTG        624
   His Ile Val Phe Pro Thr Asn Lys Glu Phe His Phe Ile Leu Asp Val
                   175                 180                 185
   TCA GTC AAG ACT GTA GCA AAT CTG GAA CTA TCT AAT ATC AAA TGT GTG        672
   Ser Val Lys Thr Val Ala Asn Leu Glu Leu Ser Asn Ile Lys Cys Val
               190                 195                 200
   CTA GAA GAT AAC AAA TGT TCT TAC TTC CTA AGT ATT CTG GCG AAA CTT        720
   Leu Glu Asp Asn Lys Cys Ser Tyr Phe Leu Ser Ile Leu Ala Lys Leu
           205                 210                 215
   CAA ACA AAT CCA AAG TTA TCA AGT CTT ACC TTA AAC AAC ATT GAA ACA     768
   Gln Thr Asn Pro Lys Leu Ser Ser Leu Thr Leu Asn Asn Ile Glu Thr
       220                 225                 230
   ACT TGG AAT TCT TTC ATT AGG ATC CTC CAA CTA GTT TGG CAT ACA ACT     816
   Thr Trp Asn Ser Phe Ile Arg Ile Leu Gln Leu Val Trp His Thr Thr
   235                 240                 245                 250
   GTA TGG TAT TTC TCA ATT TCA AAC GTG AAG CTA CAG GGT CAG CTG GAC     864
   Val Trp Tyr Phe Ser Ile Ser Asn Val Lys Leu Gln Gly Gln Leu Asp
                   255                 260                 265
   TTC AGA GAT TTT GAT TAT TCT GGC ACT TCC TTG AAG GCC TTG TCT ATA     912
   Phe Arg Asp Phe Asp Tyr Ser Gly Thr Ser Leu Lys Ala Leu Ser Ile
               270                 275                 280
   CAC CAA GTT GTC AGC GAT GTG TTC GGT TTT CCG CAA AGT TAT ATC TAT     960
   His Gln Val Val Ser Asp Val Phe Gly Phe Pro Gln Ser Tyr Ile Tyr
           285                 290                 295
   GAA ATC TTT TCG AAT ATG AAC ATC AAA AAT TTC ACA GTG TCT GGT ACA    1008
   Glu Ile Phe Ser Asn Met Asn Ile Lys Asn Phe Thr Val Ser Gly Thr
       300                 305                 310
   CGC ATG GTC CAC ATG CTT TGC CCA TCC AAA ATT AGC CCG TTC CTG CAT    1056
   Arg Met Val His Met Leu Cys Pro Ser Lys Ile Ser Pro Phe Leu His
   315                 320                 325                 330
   TTG GAT TTT TCC AAT AAT CTC TTA ACA GAC ACG GTT TTT GAA AAT TGT    1104
   Leu Asp Phe Ser Asn Asn Leu Leu Thr Asp Thr Val Phe Glu Asn Cys
                   335                 340                 345
   GGG CAC CTT ACT GAG TTG GAG ACA CTT ATT TTA CAA ATG AAT CAA TTA    1152
   Gly His Leu Thr Glu Leu Glu Thr Leu Ile Leu Gln Met Asn Gln Leu
               350                 355                 360
   AAA GAA CTT TCA AAA ATA GCT GAA ATG ACT ACA CAG ATG AAG TCT CTG    1200
   Lys Glu Leu Ser Lys Ile Ala Glu Met Thr Thr Gln Met Lys Ser Leu
           365                 370                 375
   CAA CAA TTG GAT ATT AGC CAG AAT TCT GTA AGC TAT GAT GAA AAG AAA    1248
   Gln Gln Leu Asp Ile Ser Gln Asn Ser Val Ser Tyr Asp Glu Lys Lys
       380                 385                 390
   GGA GAC TGT TCT TGG ACT AAA AGT TTA TTA AGT TTA AAT ATG TCT TCA    1296
   Gly Asp Cys Ser Trp Thr Lys Ser Leu Leu Ser Leu Asn Met Ser Ser
   395                 400                 405                 410
   AAT ATA CTT ACT GAC ACT ATT TTC AGA TGT TTA CCT CCC AGG ATC AAG    1344
   Asn Ile Leu Thr Asp Thr Ile Phe Arg Cys Leu Pro Pro Arg Ile Lys
                   415                 420                 425
   GTA CTT GAT CTT CAC AGC AAT AAA ATA AAG AGC ATT CCT AAA CAA GTC    1392
   Val Leu Asp Leu His Ser Asn Lys Ile Lys Ser Ile Pro Lys Gln Val
               430                 435                 440
   GTA AAA CTG GAA GCT TTG CAA GAA CTC AAT GTT GCT TTC AAT TCT TTA    1440
   Val Lys Leu Glu Ala Leu Gln Glu Leu Asn Val Ala Phe Asn Ser Leu
           445                 450                 455
   ACT GAC CTT CCT GGA TGT GGC AGC TTT AGC AGC CTT TCT GTA TTG ATC     1488
   Thr Asp Leu Pro Gly Cys Gly Ser Phe Ser Ser Leu Ser Val Leu Ile
       460                 465                 470
   ATT GAT CAC AAT TCA GTT TCC CAC CCA TCA GCT GAT TTC TTC CAG AGC     1536
   Ile Asp His Asn Ser Val Ser His Pro Ser Ala Asp Phe Phe Gln Ser
   475                 480                 485                 490
   TGC CAG AAG ATG AGG TCA ATA AAA GCA GGG GAC AAT CCA TTC CAA TGT     1584
   Cys Gln Lys Met Arg Ser Ile Lys Ala Gly Asp Asn Pro Phe Gln Cys
                   495                 500                 505
   ACC TGT GAG CTC GGA GAA TTT GTC AAA AAT ATA GAC CAA GTA TCA AGT     1632
   Thr Cys Glu Leu Gly Glu Phe Val Lys Asn Ile Asp Gln Val Ser Ser
               510                 515                 520
   GAA GTG TTA GAG GGC TGG CCT GAT TCT TAT AAG TGT GAC TAC CCG GAA     1680
   Glu Val Leu Glu Gly Trp Pro Asp Ser Tyr Lys Cys Asp Tyr Pro Glu
           525                 530                 535
   AGT TAT AGA GGA ACC CTA CTA AAG GAC TTT CAC ATG TCT GAA TTA TCC     1728
   Ser Tyr Arg Gly Thr Leu Leu Lys Asp Phe His Met Ser Glu Leu Ser
       540                 545                 550
   TGC AAC ATA ACT CTG CTG ATC GTC ACC ATC GTT GCC ACC ATG CTG GTG     1776
   Cys Asn Ile Thr Leu Leu Ile Val Thr Ile Val Ala Thr Met Leu Val
   555                 560                 565                 570
   TTG GCT GTG ACT GTG ACC TCC CTC TGC ATC TAC TTG GAT CTG CCC TGG     1824
   Leu Ala Val Thr Val Thr Ser Leu Cys Ile Tyr Leu Asp Leu Pro Trp
                   575                 580                 585
   TAT CTC AGG ATG GTG TGC CAG TGG ACC CAG ACC CGG CGC AGG GCC AGG     1872
   Tyr Leu Arg Met Val Cys Gln Trp Thr Gln Thr Arg Arg Arg Ala Arg
               590                 595                 600
   AAC ATA CCC TTA GAA GAA CTC CAA AGA AAT CTC CAG TTT CAT GCA TTT     1920
   Asn Ile Pro Leu Glu Glu Leu Gln Arg Asn Leu Gln Phe His Ala Phe
           605                 610                 615
   ATT TCA TAT AGT GGG CAC GAT TCT TTC TGG GTG AAG AAT GAA TTA TTG     1968
   Ile Ser Tyr Ser Gly His Asp Ser Phe Trp Val Lys Asn Glu Leu Leu
       620                 625                 630
   CCA AAC CTA GAG AAA GAA GGT ATG CAG ATT TGC CTT CAT GAG AGA AAC     2016
   Pro Asn Leu Glu Lys Glu Gly Met Gln Ile Cys Leu His Glu Arg Asn
   635                 640                 645                 650
   TTT GTT CCT GGC AAG AGC ATT GTG GAA AAT ATC ATC ACC TGC ATT GAG     2064
   Phe Val Pro Gly Lys Ser Ile Val Glu Asn Ile Ile Thr Cys Ile Glu
                   655                 660                 665
   AAG AGT TAC AAG TCC ATC TTT GTT TTG TCT CCC AAC TTT GTC CAG AGT     2112
   Lys Ser Tyr Lys Ser Ile Phe Val Leu Ser Pro Asn Phe Val Gln Ser
               670                 675                 680
   GAA TGG TGC CAT TAT GAA CTC TAC TTT GCC CAT CAC AAT CTC TTT CAT     2160
   Glu Trp Cys His Tyr Glu Leu Tyr Phe Ala His His Asn Leu Phe His
           685                 690                 695
   GAA GGA TCT AAT AGC TTA ATC CTG ATC TTG CTG GAA CCC ATT CCG CAG        2208
   Glu Gly Ser Asn Ser Leu Ile Leu Ile Leu Leu Glu Pro Ile Pro Gln
       700                 705                 710
   TAC TCC ATT CCT AGC AGT TAT CAC AAG CTC AAA AGT CTC ATG GCC AGG        2256
   Tyr Ser Ile Pro Ser Ser Tyr His Lys Leu Lys Ser Leu Met Ala Arg
   715                 720                 725                 730
   AGG ACT TAT TTG GAA TGG CCC AAG GAA AAG AGC AAA CGT GGC CTT TTT        2304
   Arg Thr Tyr Leu Glu Trp Pro Lys Glu Lys Ser Lys Arg Gly Leu Phe
                   735                 740                 745
   TGG GCT AAC TTA AGG GCA GCC ATT AAT ATT AAG CTG ACA GAG CAA GCA        2352
   Trp Ala Asn Leu Arg Ala Ala Ile Asn Ile Lys Leu Thr Glu Gln Ala
               750                 755                 760
   AAG AAA TAGTCTAGA                                                      2367
   Lys Lys
(2) INFORMATION FOR SEQ ID NO:2:
   (i) SEQUENCE CHARACTERISTICS:
      (A) LENGTH: 786 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Thr Ser Ile Phe His Phe Ala Ile Ile Phe Met Leu Ile Leu Gln
   -22     -20                 -15                 -10
   Ile Arg Ile Gln Leu Ser Glu Glu Ser Glu Phe Leu Val Asp Arg Ser
        -5                  1               5                  10
   Lys Asn Gly Leu Ile His Val Pro Lys Asp Leu Ser Gln Lys Thr Thr
                    15                  20                  25
   Ile Leu Asn Ile Ser Gln Asn Tyr Ile Ser Glu Leu Trp Thr Ser Asp
                30                  35                  40
   Ile Leu Ser Leu Ser Lys Leu Arg Ile Leu Ile Ile Ser His Asn Arg
            45                  50                  55
   Ile Gln Tyr Leu Asp Ile Ser Val Phe Lys Phe Asn Gln Glu Leu Glu
        60                  65                  70
   Tyr Leu Asp Leu Ser His Asn Lys Leu Val Lys Ile Ser Cys His Pro
    75                  80                  85                  90
   Thr Val Asn Leu Lys His Leu Asp Leu Ser Phe Asn Ala Phe Asp Ala
                    95                 100                 105
   Leu Pro Ile Cys Lys Glu Phe Gly Asn Met Ser Gln Leu Lys Phe Leu
               110                 115                 120
   Gly Leu Ser Thr Thr His Leu Glu Lys Ser Ser Val Leu Pro Ile Ala
           125                 130                 135
   His Leu Asn Ile Ser Lys Val Leu Leu Val Leu Gly Glu Thr Tyr Gly
       140                 145                 150
   Glu Lys Glu Asp Pro Glu Gly Leu Gln Asp Phe Asn Thr Glu Ser Leu
   155                 160                 165                 170
   His Ile Val Phe Pro Thr Asn Lys Glu Phe His Phe Ile Leu Asp Val
                   175                 180                 185
   Ser Val Lys Thr Val Ala Asn Leu Glu Leu Ser Asn Ile Lys Cys Val
               190                 195                 200
   Leu Glu Asp Asn Lys Cys Ser Tyr Phe Leu Ser Ile Leu Ala Lys Leu
           205                 210                 215
   Gln Thr Asn Pro Lys Leu Ser Ser Leu Thr Leu Asn Asn Ile Glu Thr
       220                 225                 230
   Thr Trp Asn Ser Phe Ile Arg Ile Leu Gln Leu Val Trp His Thr Thr
   235                 240                 245                 250
   Val Trp Tyr Phe Ser Ile Ser Asn Val Lys Leu Gln Gly Gln Leu Asp
                   255                 260                 265
   Phe Arg Asp Phe Asp Tyr Ser Gly Thr Ser Leu Lys Ala Leu Ser Ile
               270                 275                 280
   His Gln Val Val Ser Asp Val Phe Gly Phe Pro Gln Ser Tyr Ile Tyr
           285                 290                 295
   Glu Ile Phe Ser Asn Met Asn Ile Lys Asn Phe Thr Val Ser Gly Thr
       300                 305                 310
   Arg Met Val His Met Leu Cys Pro Ser Lys Ile Ser Pro Phe Leu His
   315                 320                 325                 330
   Leu Asp Phe Ser Asn Asn Leu Leu Thr Asp Thr Val Phe Glu Asn Cys
                   335                 340                 345
   Gly His Leu Thr Glu Leu Glu Thr Leu Ile Leu Gln Met Asn Gln Leu
               350                 355                 360
   Lys Glu Leu Ser Lys Ile Ala Glu Met Thr Thr Gln Met Lys Ser Leu
           365                 370                 375
   Gln Gln Leu Asp Ile Ser Gln Asn Ser Val Ser Tyr Asp Glu Lys Lys
       380                 385                 390
   Gly Asp Cys Ser Trp Thr Lys Ser Leu Leu Ser Leu Asn Met Ser Ser
   395                 400                 405                 410
   Asn Ile Leu Thr Asp Thr Ile Phe Arg Cys Leu Pro Pro Arg Ile Lys
                   415                 420                 425
   Val Leu Asp Leu His Ser Asn Lys Ile Lys Ser Ile Pro Lys Gln Val
               430                 435                 440
   Val Lys Leu Glu Ala Leu Gln Glu Leu Asn Val Ala Phe Asn Ser Leu
           445                 450                 455
   Thr Asp Leu Pro Gly Cys Gly Ser Phe Ser Ser Leu Ser Val Leu Ile
       460                 465                 470
   Ile Asp His Asn Ser Val Ser His Pro Ser Ala Asp Phe Phe Gln Ser
   475                 480                 485                     490
   Cys Gln Lys Met Arg Ser Ile Lys Ala Gly Asp Asn Pro Phe Gln Cys
                   495                 500                     505
   Thr Cys Glu Leu Gly Glu Phe Val Lys Asn Ile Asp Gln Val Ser Ser
               510                 515                 520
   Glu Val Leu Glu Gly Trp Pro Asp Ser Tyr Lys Cys Asp Tyr Pro Glu
           525                 530                 535
   Ser Tyr Arg Gly Thr Leu Leu Lys Asp Phe His Met Ser Glu Leu Ser
       540                 545                 550
   Cys Asn Ile Thr Leu Leu Ile Val Thr Ile Val Ala Thr Met Leu Val
   555                 560                 565                     570
   Leu Ala Val Thr Val Thr Ser Leu Cys Ile Tyr Leu Asp Leu Pro Trp
                   575                 580                     585
   Tyr Leu Arg Met Val Cys Gln Trp Thr Gln Thr Arg Arg Arg Ala Arg
               590                 595                 600
   Asn Ile Pro Leu Glu Glu Leu Gln Arg Asn Leu Gln Phe His Ala Phe
           605                 610                 615
   Ile Ser Tyr Ser Gly His Asp Ser Phe Trp Val Lys Asn Glu Leu Leu
       620                 625                 630
   Pro Asn Leu Glu Lys Glu Gly Met Gln Ile Cys Leu His Glu Arg Asn
   635                 640                 645                     650
   Phe Val Pro Gly Lys Ser Ile Val Glu Asn Ile Ile Thr Cys Ile Glu
                   655                 660                     665
   Lys Ser Tyr Lys Ser Ile Phe Val Leu Ser Pro Asn Phe Val Gln Ser
               670                 675                 680
   Glu Trp Cys His Tyr Glu Leu Tyr Phe Ala His His Asn Leu Phe His
           685                 690                 695
   Glu Gly Ser Asn Ser Leu Ile Leu Ile Leu Leu Glu Pro Ile Pro Gln
       700                 705                 710
   Tyr Ser Ile Pro Ser Ser Tyr His Lys Leu Lys Ser Leu Met Ala Arg
   715                 720                 725                     730
   Arg Thr Tyr Leu Glu Trp Pro Lys Glu Lys Ser Lys Arg Gly Leu Phe
                   735                 740                     745
   Trp Ala Asn Leu Arg Ala Ala Ile Asn Ile Lys Leu Thr Glu Gln Ala
               750                 755                 760
   Lys Lys
(2) INFORMATION FOR SEQ ID NO:3:
   (i) SEQUENCE CHARACTERISTICS:
      (A) LENGTH: 2355 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..2352
   (ix) FEATURE:
      (A) NAME/KEY: mat_peptide
      (B) LOCATION: 67..2352
   (xi) SEQUENCE DESCRIPTION: SEQ ID NO:3:
   ATG CCA CAT ACT TTG TGG ATG GTG TGG GTC TTG GGG GTC ATC ATC AGC       48
   CTC TCC AAG GAA GAA TCC TCC AAT CAG GCT TCT CTG TCT TGT GAC CGC       96
   AAT GGT ATC TGC AAG GGC AGC TCA GGA TCT TTA AAC TCC ATT CCC TCA      144
   GGG CTC ACA GAA GCT GTA AAA AGC CTT GAC CTG TCC AAC AAC AGG ATC      192
   ACC TAC ATT AGC AAC AGT GAC CTA CAG AGG TGT GTG AAC CTC CAG GCT      240
   CTG GTG CTG ACA TCC AAT GGA ATT AAC ACA ATA GAG GAA GAT TCT TTT      288
   TCT TCC CTG GGC AGT CTT GAA CAT TTA GAC TTA TCC TAT AAT TAC TTA      336
   TCT AAT TTA TCG TCT TCC TGG TTC AAG CCC CTT TCT TCT TTA ACA TTC      384
   TTA AAC TTA CTG GGA AAT CCT TAC AAA ACC CTA GGG GAA ACA TCT CTT      432
   TTT TCT CAT CTC ACA AAA TTG CAA ATC CTG AGA GTG GGA AAT ATG GAC      480
   ACC TTC ACT AAG ATT CAA AGA AAA GAT TTT GCT GGA CTT ACC TTC CTT      528
   GAG GAA CTT GAG ATT GAT GCT TCA GAT CTA CAG AGC TAT GAG CCA AAA      576
   AGT TTG AAG TCA ATT CAG AAC GTA AGT CAT CTG ATC CTT CAT ATG AAG      624
   CAG CAT ATT TTA CTG CTG GAG ATT TTT GTA GAT GTT ACA AGT TCC GTG      672
   GAA TGT TTG GAA CTG CGA GAT ACT GAT TTG GAC ACT TTC CAT TTT TCA      720
   GAA CTA TCC ACT GGT GAA ACA AAT TCA TTG ATT AAA AAG TTT ACA TTT      768
   AGA AAT GTG AAA ATC ACC GAT GAA AGT TTG TTT CAG GTT ATG AAA CTT      816
   TTG AAT CAG ATT TCT GGA TTG TTA GAA TTA GAG TTT GAT GAC TGT ACC      864
   CTT AAT GGA GTT GGT AAT TTT AGA GCA TCT GAT AAT GAC AGA GTT ATA      912
   GAT CCA GGT AAA GTG GAA ACG TTA ACA ATC CGG AGG CTG CAT ATT CCA      960
   AGG TTT TAC TTA TTT TAT GAT CTG AGC ACT TTA TAT TCA CTT ACA GAA     1008
   AGA GTT AAA AGA ATC ACA GTA GAA AAC AGT AAA GTT TTT CTG GTT CCT     1056
   TGT TTA CTT TCA CAA CAT TTA AAA TCA TTA GAA TAC TTG GAT CTC AGT     1104
   GAA AAT TTG ATG GTT GAA GAA TAC TTG AAA AAT TCA GCC TGT GAG GAT     1152
   GCC TGG CCC TCT CTA CAA ACT TTA ATT TTA AGG CAA AAT CAT TTG GCA     1200
   TCA TTG GAA AAA ACC GGA GAG ACT TTG CTC ACT CTG AAA AAC TTG ACT     1248
   AAC ATT GAT ATC AGT AAG AAT AGT TTT CAT TCT ATG CCT GAA ACT TGT       1296
   CAG TGG CCA GAA AAG ATG AAA TAT TTG AAC TTA TCC AGC ACA CGA ATA       1344
   CAC AGT GTA ACA GGC TGC ATT CCC AAG ACA CTG GAA ATT TTA GAT GTT       1392
   AGC AAC AAC AAT CTC AAT TTA TTT TCT TTG AAT TTG CCG CAA CTC AAA       1440
   GAA CTT TAT ATT TCC AGA AAT AAG TTG ATG ACT CTA CCA GAT GCC TCC       1488
   CTC TTA CCC ATG TTA CTA GTA TTG AAA ATC AGT AGG AAT GCA ATA ACT       1536
   ACG TTT TCT AAG GAG CAA CTT GAC TCA TTT CAC ACA CTG AAG ACT TTG       1584
   GAA GCT GGT GGC AAT AAC TTC ATT TGC TCC TGT GAA TTC CTC TCC TTC       1632
   ACT CAG GAG CAG CAA GCA CTG GCC AAA GTC TTG ATT GAT TGG CCA GCA       1680
   AAT TAC CTG TGT GAC TCT CCA TCC CAT GTG CGT GGC CAG CAG GTT CAG       1728
   GAT GTC CGC CTC TCG GTG TCG GAA TGT CAC AGG ACA GCA CTG GTG TCT       1776
   GGC ATG TGC TGT GCT CTG TTC CTG CTG ATC CTG CTC ACG GGG GTC CTG       1824
   TGC CAC CGT TTC CAT GGC CTG TGG TAT ATG AAA ATG ATG TGG GCC TGG       1872
   CTC CAG GCC AAA AGG AAG CCC AGG AAA GCT CCC AGC AGG AAC ATC TGC       1920
   TAT GAT GCA TTT GTT TCT TAC AGT GAG CGG GAT GCC TAC TGG GTG GAG       1968
   AAC CTT ATG GTC CAG GAG CTG GAG AAC TTC AAT CCC CCC TTC AAG TTG        2016
   TGT CTT CAT AAG CGG GAC TTC ATT CCT GGC AAG TGG ATC ATT GAC AAT        2064
   ATC ATT GAC TCC ATT GAA AAG AGC CAC AAA ACT GTC TTT GTG CTT TCT        2112
   GAA AAC TTT GTG AAG AGT GAG TGG TGC AAG TAT GAA CTG GAC TTC TCC        2160
   CAT TTC CGT CTT TTT GAA GAG AAC AAT GAT GCT GCC ATT CTC ATT CTT        2208
   CTG GAG CCC ATT GAG AAA AAA GCC ATT CCC CAG CGC TTC TGC AAG CTG        2256
   CGG AAG ATA ATG AAC ACC AAG ACC TAC CTG GAG TGG CCC ATG GAC GAG        2304
   GCT CAG CGG GAA GGA TTT TGG GTA AAT CTG AGA GCT GCG ATA AAG TCC        2352
   TAG                                                                    2355
(2) INFORMATION FOR SEQ ID NO:4:
   (i) SEQUENCE CHARACTERISTICS:
      (A) LENGTH: 784 amino acids
      (B) TYPE: amino acid
      (D) TOPOLOGY: linear
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 2715 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..2712
   (ix) FEATURE:
      (A) NAME/KEY: mat_peptide
      (B) LOCATION: 64..2712
   (xi) SEQUENCE DESCRIPTION: SEQ ID NO:5:
   ATG AGA CAG ACT TTG CCT TGT ATC TAC TTT TGG GGG GGC CTT TTG CCC      48
   Met Arg Gln Thr Leu Pro Cys Ile Tyr Phe Trp Gly Gly Leu Leu Pro
   -21 -20                 -15                 -10
   TTT GGG ATG CTG TGT GCA TCC TCC ACC ACC AAG TGC ACT GTT AGC CAT      96
   Phe Gly Met Leu Cys Ala Ser Ser Thr Thr Lys Cys Thr Val Ser His
    -5                   1               5                  10
   GAA GTT GCT GAC TGC AGC CAC CTG AAG TTG ACT CAG GTA CCC GAT GAT     144
   Glu Val Ala Asp Cys Ser His Leu Lys Leu Thr Gln Val Pro Asp Asp
                15                  20                  25
   CTA CCC ACA AAC ATA ACA GTG TTG AAC CTT ACC CAT AAT CAA CTC AGA     192
   Leu Pro Thr Asn Ile Thr Val Leu Asn Leu Thr His Asn Gln Leu Arg
            30                  35                  40
   AGA TTA CCA GCC GCC AAC TTC ACA AGG TAT AGC CAG CTA ACT AGC TTG     240
   Arg Leu Pro Ala Ala Asn Phe Thr Arg Tyr Ser Gln Leu Thr Ser Leu
        45                  50                  55
   GAT GTA GGA TTT AAC ACC ATC TCA AAA CTG GAG CCA GAA TTG TGC CAG     288
   Asp Val Gly Phe Asn Thr Ile Ser Lys Leu Glu Pro Glu Leu Cys Gln
    60                  65                  70                  75
   AAA CTT CCC ATG TTA AAA GTT TTG AAC CTC CAG CAC AAT GAG CTA TCT     336
   Lys Leu Pro Met Leu Lys Val Leu Asn Leu Gln His Asn Glu Leu Ser
                       80                      85                      90
   CAA CTT TCT GAT AAA ACC TTT GCC TTC TGC ACG AAT TTG ACT GAA CTC      384
   Gln Leu Ser Asp Lys Thr Phe Ala Phe Cys Thr Asn Leu Thr Glu Leu
                95                  100                 105
   CAT CTC ATG TCC AAC TCA ATC CAG AAA ATT AAA AAT AAT CCC TTT GTC      432
   His Leu Met Ser Asn Ser Ile Gln Lys Ile Lys Asn Asn Pro Phe Val
           110                 115                 120
   AAG CAG AAG AAT TTA ATC ACA TTA GAT CTG TCT CAT AAT GGC TTG TCA      480
   Lys Gln Lys Asn Leu Ile Thr Leu Asp Leu Ser His Asn Gly Leu Ser
       125                 130                 135
   TCT ACA AAA TTA GGA ACT CAG GTT CAG CTG GAA AAT CTC CAA GAG CTT      528
   Ser Thr Lys Leu Gly Thr Gln Val Gln Leu Glu Asn Leu Gln Glu Leu
   140                 145                 150                 155
   CTA TTA TCA AAC AAT AAA ATT CAA GCG CTA AAA AGT GAA GAA CTG GAT      576
   Leu Leu Ser Asn Asn Lys Ile Gln Ala Leu Lys Ser Glu Glu Leu Asp
                   160                 165                 170
   ATC TTT GCC AAT TCA TCT TTA AAA AAA TTA GAG TTG TCA TCG AAT CAA      624
   Ile Phe Ala Asn Ser Ser Leu Lys Lys Leu Glu Leu Ser Ser Asn Gln
               175                 180                 185
   ATT AAA GAG TTT TCT CCA GGG TGT TTT CAC GCA ATT GGA AGA TTA TTT      672
   Ile Lys Glu Phe Ser Pro Gly Cys Phe His Ala Ile Gly Arg Leu Phe
           190                 195                 200
   GGC CTC TTT CTG AAC AAT GTC CAG CTG GGT CCC AGC CTT ACA GAG AAG      720
   Gly Leu Phe Leu Asn Asn Val Gln Leu Gly Pro Ser Leu Thr Glu Lys
       205                 210                 215
   CTA TGT TTG GAA TTA GCA AAC ACA AGC ATT CGG AAT CTG TCT CTG AGT      768
   Leu Cys Leu Glu Leu Ala Asn Thr Ser Ile Arg Asn Leu Ser Leu Ser
   220                 225                 230                 235
   AAC AGC CAG CTG TCC ACC ACC AGC AAT ACA ACT TTC TTG GGA CTA AAG      816
   Asn Ser Gln Leu Ser Thr Thr Ser Asn Thr Thr Phe Leu Gly Leu Lys
                   240                 245                 250
   TGG ACA AAT CTC ACT ATG CTC GAT CTT TCC TAC AAC AAC TTA AAT GTG      864
   Trp Thr Asn Leu Thr Met Leu Asp Leu Ser Tyr Asn Asn Leu Asn Val
               255                 260                 265
   GTT GGT AAC GAT TCC TTT GCT TGG CTT CCA CAA CTA GAA TAT TTC TTC      912
   Val Gly Asn Asp Ser Phe Ala Trp Leu Pro Gln Leu Glu Tyr Phe Phe
           270                 275                 280
   CTA GAG TAT AAT AAT ATA CAG CAT TTG TTT TCT CAC TCT TTG CAC GGG      960
   Leu Glu Tyr Asn Asn Ile Gln His Leu Phe Ser His Ser Leu His Gly
       285                 290                 295
   CTT TTC AAT GTG AGG TAC CTG AAT TTG AAA CGG TCT TTT ACT AAA CAA     1008
   Leu Phe Asn Val Arg Tyr Leu Asn Leu Lys Arg Ser Phe Thr Lys Gln
   300                 305                 310                 315
   AGT ATT TCC CTT GCC TCA CTC CCC AAG ATT GAT GAT TTT TCT TTT CAG     1056
   Ser Ile Ser Leu Ala Ser Leu Pro Lys Ile Asp Asp Phe Ser Phe Gln
                   320                 325                 330
   TGG CTA AAA TGT TTG GAG CAC CTT AAC ATG GAA GAT AAT GAT ATT CCA      1104
   Trp Leu Lys Cys Leu Glu His Leu Asn Met Glu Asp Asn Asp Ile Pro
               335                 340                 345
   GGC ATA AAA AGC AAT ATG TTC ACA GGA TTG ATA AAC CTG AAA TAC TTA      1152
   Gly Ile Lys Ser Asn Met Phe Thr Gly Leu Ile Asn Leu Lys Tyr Leu
           350                 355                 360
   AGT CTA TCC AAC TCC TTT ACA AGT TTG CGA ACT TTG ACA AAT GAA ACA      1200
   Ser Leu Ser Asn Ser Phe Thr Ser Leu Arg Thr Leu Thr Asn Glu Thr
       365                 370                 375
   TTT GTA TCA CTT GCT CAT TCT CCC TTA CAC ATA CTC AAC CTA ACC AAG      1248
   Phe Val Ser Leu Ala His Ser Pro Leu His Ile Leu Asn Leu Thr Lys
   380                 385                 390                 395
   AAT AAA ATC TCA AAA ATA GAG AGT GAT GCT TTC TCT TGG TTG GGC CAC      1296
   Asn Lys Ile Ser Lys Ile Glu Ser Asp Ala Phe Ser Trp Leu Gly His
                   400                 405                 410
   CTA GAA GTA CTT GAC CTG GGC CTT AAT GAA ATT GGG CAA GAA CTC ACA      1344
   Leu Glu Val Leu Asp Leu Gly Leu Asn Glu Ile Gly Gln Glu Leu Thr
               415                 420                 425
   GGC CAG GAA TGG AGA GGT CTA GAA AAT ATT TTC GAA ATC TAT CTT TCC      1392
   Gly Gln Glu Trp Arg Gly Leu Glu Asn Ile Phe Glu Ile Tyr Leu Ser
           430                 435                 440
   TAC AAC AAG TAC CTG CAG CTG ACT AGG AAC TCC TTT GCC TTG GTC CCA      1440
   Tyr Asn Lys Tyr Leu Gln Leu Thr Arg Asn Ser Phe Ala Leu Val Pro
       445                 450                 455
   AGC CTT CAA CGA CTG ATG CTC CGA AGG GTG GCC CTT AAA AAT GTG GAT      1488
   Ser Leu Gln Arg Leu Met Leu Arg Arg Val Ala Leu Lys Asn Val Asp
   460                 465                 470                 475
   AGC TCT CCT TCA CCA TTC CAG CCT CTT CGT AAC TTG ACC ATT CTG GAT      1536
   Ser Ser Pro Ser Pro Phe Gln Pro Leu Arg Asn Leu Thr Ile Leu Asp
                   480                 485                 490
   CTA AGC AAC AAC AAC ATA GCC AAC ATA AAT GAT GAC ATG TTG GAG GGT      1584
   Leu Ser Asn Asn Asn Ile Ala Asn Ile Asn Asp Asp Met Leu Glu Gly
               495                 500                 505
   CTT GAG AAA CTA GAA ATT CTC GAT TTG CAG CAT AAC AAC TTA GCA CGG      1632
   Leu Glu Lys Leu Glu Ile Leu Asp Leu Gln His Asn Asn Leu Ala Arg
           510                 515                 520
   CTC TGG AAA CAC GCA AAC CCT GGT GGT CCC ATT TAT TTC CTA AAG GGT      1680
   Leu Trp Lys His Ala Asn Pro Gly Gly Pro Ile Tyr Phe Leu Lys Gly
       525                 530                 535
   CTG TCT CAC CTC CAC ATC CTT AAC TTG GAG TCC AAC GGC TTT GAC GAG      1728
   Leu Ser His Leu His Ile Leu Asn Leu Glu Ser Asn Gly Phe Asp Glu
   540                 545                 550                 555
   ATC CCA GTT GAG GTC TTC AAG GAT TTA TTT GAA CTA AAG ATC ATC GAT      1776
   Ile Pro Val Glu Val Phe Lys Asp Leu Phe Glu Leu Lys Ile Ile Asp
                   560                 565                 570
   TTA GGA TTG AAT AAT TTA AAC ACA CTT CCA GCA TCT GTC TTT AAT AAT      1824
   Leu Gly Leu Asn Asn Leu Asn Thr Leu Pro Ala Ser Val Phe Asn Asn
               575                 580                 585
   CAG GTG TCT CTA AAG TCA TTG AAC CTT CAG AAG AAT CTC ATA ACA TCC      1872
   Gln Val Ser Leu Lys Ser Leu Asn Leu Gln Lys Asn Leu Ile Thr Ser
           590                 595                 600
   GTT GAG AAG AAG GTT TTC GGG CCA GCT TTC AGG AAC CTG ACT GAG TTA      1920
   Val Glu Lys Lys Val Phe Gly Pro Ala Phe Arg Asn Leu Thr Glu Leu
       605                 610                 615
   GAT ATG CGC TTT AAT CCC TTT GAT TGC ACG TGT GAA AGT ATT GCC TGG      1968
   Asp Met Arg Phe Asn Pro Phe Asp Cys Thr Cys Glu Ser Ile Ala Trp
   620                 625                 630                 635
   TTT GTT AAT TGG ATT AAC GAG ACC CAT ACC AAC ATC CCT GAG CTG TCA      2016
   Phe Val Asn Trp Ile Asn Glu Thr His Thr Asn Ile Pro Glu Leu Ser
                   640                 645                 650
   AGC CAC TAC CTT TGC AAC ACT CCA CCT CAC TAT CAT GGG TTC CCA GTG      2064
   Ser His Tyr Leu Cys Asn Thr Pro Pro His Tyr His Gly Phe Pro Val
               655                 660                 665
   AGA CTT TTT GAT ACA TCA TCT TGC AAA GAC AGT GCC CCC TTT GAA CTC      2112
   Arg Leu Phe Asp Thr Ser Ser Cys Lys Asp Ser Ala Pro Phe Glu Leu
           670                 675                 680
   TTT TTC ATG ATC AAT ACC AGT ATC CTG TTG ATT TTT ATC TTT ATT GTA      2160
   Phe Phe Met Ile Asn Thr Ser Ile Leu Leu Ile Phe Ile Phe Ile Val
       685                 690                 695
   CTT CTC ATC CAC TTT GAG GGC TGG AGG ATA TCT TTT TAT TGG AAT GTT      2208
   Leu Leu Ile His Phe Glu Gly Trp Arg Ile Ser Phe Tyr Trp Asn Val
   700                 705                 710                 715
   TCA GTA CAT CGA GTT CTT GGT TTC AAA GAA ATA GAC AGA CAG ACA GAA      2256
   Ser Val His Arg Val Leu Gly Phe Lys Glu Ile Asp Arg Gln Thr Glu
                   720                 725                 730
   CAG TTT GAA TAT GCA GCA TAT ATA ATT CAT GCC TAT AAA GAT AAG GAT      2304
   Gln Phe Glu Tyr Ala Ala Tyr Ile Ile His Ala Tyr Lys Asp Lys Asp
               735                 740                 745
   TGG GTC TGG GAA CAT TTC TCT TCA ATG GAA AAG GAA GAC CAA TCT CTC      2352
   Trp Val Trp Glu His Phe Ser Ser Met Glu Lys Glu Asp Gln Ser Leu
           750                 755                 760
   AAA TTT TGT CTG GAA GAA AGG GAC TTT GAG GCG GGT GTT TTT GAA CTA      2400
   Lys Phe Cys Leu Glu Glu Arg Asp Phe Glu Ala Gly Val Phe Glu Leu
       765                 770                 775
   GAA GCA ATT GTT AAC AGC ATC AAA AGA AGC AGA AAA ATT ATT TTT GTT      2448
   Glu Ala Ile Val Asn Ser Ile Lys Arg Ser Arg Lys Ile Ile Phe Val
   780                 785                 790                 795
   ATA ACA CAC CAT CTA TTA AAA GAC CCA TTA TGC AAA AGA TTC AAG GTA      2496
   Ile Thr His His Leu Leu Lys Asp Pro Leu Cys Lys Arg Phe Lys Val
                   800                 805                 810
   CAT CAT GCA GTT CAA CAA GCT ATT GAA CAA AAT CTG GAT TCC ATT ATA      2544
   His His Ala Val Gln Gln Ala Ile Glu Gln Asn Leu Asp Ser Ile Ile
               815                 820                 825
   TTG GTT TTC CTT GAG GAG ATT CCA GAT TAT AAA CTG AAC CAT GCA CTC        2592
   Leu Val Phe Leu Glu Glu Ile Pro Asp Tyr Lys Leu Asn His Ala Leu
           830                 835                 840
   TGT TTG CGA AGA GGA ATG TTT AAA TCT CAC TGC ATC TTG AAC TGG CCA        2640
   Cys Leu Arg Arg Gly Met Phe Lys Ser His Cys Ile Leu Asn Trp Pro
       845                 850                 855
   GTT CAG AAA GAA CGG ATA GGT GCC TTT CGT CAT AAA TTG CAA GTA GCA        2688
   Val Gln Lys Glu Arg Ile Gly Ala Phe Arg His Lys Leu Gln Val Ala
   860                 865                 870                 875
   CTT GGA TCC AAA AAC TCT GTA CAT TAA                                    2715
   Leu Gly Ser Lys Asn Ser Val His
                   880
(2) INFORMATION FOR SEQ ID NO:6:
   (i) SEQUENCE CHARACTERISTICS:
      (A) LENGTH: 904 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Arg Gln Thr Leu Pro Cys Ile Tyr Phe Trp Gly Gly Leu Leu Pro
   -21 -20                 -15                 -10
   Phe Gly Met Leu Cys Ala Ser Ser Thr Thr Lys Cys Thr Val Ser His
    -5                   1               5                  10
   Glu Val Ala Asp Cys Ser His Leu Lys Leu Thr Gln Val Pro Asp Asp
                15                  20                  25
   Leu Pro Thr Asn Ile Thr Val Leu Asn Leu Thr His Asn Gln Leu Arg
            30                  35                  40
   Arg Leu Pro Ala Ala Asn Phe Thr Arg Tyr Ser Gln Leu Thr Ser Leu
        45                  50                  55
   Asp Val Gly Phe Asn Thr Ile Ser Lys Leu Glu Pro Glu Leu Cys Gln
    60                  65                  70                  75
   Lys Leu Pro Met Leu Lys Val Leu Asn Leu Gln His Asn Glu Leu Ser
                    80                  85                  90
   Gln Leu Ser Asp Lys Thr Phe Ala Phe Cys Thr Asn Leu Thr Glu Leu
                95                 100                 105
   His Leu Met Ser Asn Ser Ile Gln Lys Ile Lys Asn Asn Pro Phe Val
           110                 115                 120
   Lys Gln Lys Asn Leu Ile Thr Leu Asp Leu Ser His Asn Gly Leu Ser
       125                 130                 135
   Ser Thr Lys Leu Gly Thr Gln Val Gln Leu Glu Asn Leu Gln Glu Leu
   140 145 150 155
   Leu Leu Ser Asn Asn Lys Ile Gln Ala Leu Lys Ser Glu Glu Leu Asp
   160 165 170
   Ile Phe Ala Asn Ser Ser Leu Lys Lys Leu Glu Leu Ser Ser Asn Gln
   175 180 185
   Ile Lys Glu Phe Ser Pro Gly Cys Phe His Ala Ile Gly Arg Leu Phe
   190 195 200
   Gly Leu Phe Leu Asn Asn Val Gln Leu Gly Pro Ser Leu Thr Glu Lys
   205 210 215
   Leu Cys Leu Glu Leu Ala Asn Thr Ser Ile Arg Asn Leu Ser Leu Ser
   220 225 230 235
   Asn Ser Gln Leu Ser Thr Thr Ser Asn Thr Thr Phe Leu Gly Leu Lys
   240 245 250
   Trp Thr Asn Leu Thr Met Leu Asp Leu Ser Tyr Asn Asn Leu Asn Val
   255 260 265
   Val Gly Asn Asp Ser Phe Ala Trp Leu Pro Gln Leu Glu Tyr Phe Phe
   270 275 280
   Leu Glu Tyr Asn Asn Ile Gln His Leu Phe Ser His Ser Leu His Gly
   285 290 295
   Leu Phe Asn Val Arg Tyr Leu Asn Leu Lys Arg Ser Phe Thr Lys Gln
   300 305 310 315
   Ser Ile Ser Leu Ala Ser Leu Pro Lys Ile Asp Asp Phe Ser Phe Gln
   320 325 330
   Trp Leu Lys Cys Leu Glu His Leu Asn Met Glu Asp Asn Asp Ile Pro
   335 340 345
   Gly Ile Lys Ser Asn Met Phe Thr Gly Leu Ile Asn Leu Lys Tyr Leu
   350 355 360
   Ser Leu Ser Asn Ser Phe Thr Ser Leu Arg Thr Leu Thr Asn Glu Thr
   365 370 375
   Phe Val Ser Leu Ala His Ser Pro Leu His Ile Leu Asn Leu Thr Lys
   380 385 390 395
   Asn Lys Ile Ser Lys Ile Glu Ser Asp Ala Phe Ser Trp Leu Gly His
   400 405 410
   Leu Glu Val Leu Asp Leu Gly Leu Asn Glu Ile Gly Gln Glu Leu Thr
   415 420 425
   Gly Gln Glu Trp Arg Gly Leu Glu Asn Ile Phe Glu Ile Tyr Leu Ser
   430 435 440
   Tyr Asn Lys Tyr Leu Gln Leu Thr Arg Asn Ser Phe Ala Leu Val Pro
   445 450 455
   Ser Leu Gln Arg Leu Met Leu Arg Arg Val Ala Leu Lys Asn Val Asp
   460 465 470 475
   Ser Ser Pro Ser Pro Phe Gln Pro Leu Arg Asn Leu Thr Ile Leu Asp
   480 485 490
   Leu Ser Asn Asn Asn Ile Ala Asn Ile Asn Asp Asp Met Leu Glu Gly
   495 500 505
   Leu Glu Lys Leu Glu Ile Leu Asp Leu Gln His Asn Asn Leu Ala Arg
   510 515 520
   Leu Trp Lys His Ala Asn Pro Gly Gly Pro Ile Tyr Phe Leu Lys Gly
   525 530 535
   Leu Ser His Leu His Ile Leu Asn Leu Glu Ser Asn Gly Phe Asp Glu
   540 545 550 555
   Ile Pro Val Glu Val Phe Lys Asp Leu Phe Glu Leu Lys Ile Ile Asp
   560 565 570
   Leu Gly Leu Asn Asn Leu Asn Thr Leu Pro Ala Ser Val Phe Asn Asn
   575 580 585
   Gln Val Ser Leu Lys Ser Leu Asn Leu Gln Lys Asn Leu Ile Thr Ser
   590 595 600
   Val Glu Lys Lys Val Phe Gly Pro Ala Phe Arg Asn Leu Thr Glu Leu
   605 610 615
   Asp Met Arg Phe Asn Pro Phe Asp Cys Thr Cys Glu Ser Ile Ala Trp
   620 625 630 635
   Phe Val Asn Trp Ile Asn Glu Thr His Thr Asn Ile Pro Glu Leu Ser
   640 645 650
   Ser His Tyr Leu Cys Asn Thr Pro Pro His Tyr His Gly Phe Pro Val
   655 660 665
   Arg Leu Phe Asp Thr Ser Ser Cys Lys Asp Ser Ala Pro Phe Glu Leu
   670 675 680
   Phe Phe Met Ile Asn Thr Ser Ile Leu Leu Ile Phe Ile Phe Ile Val
   685 690 695
   Leu Leu Ile His Phe Glu Gly Trp Arg Ile Ser Phe Tyr Trp Asn Val
   700 705 710 715
   Ser Val His Arg Val Leu Gly Phe Lys Glu Ile Asp Arg Gln Thr Glu
   720 725 730
   Gln Phe Glu Tyr Ala Ala Tyr Ile Ile His Ala Tyr Lys Asp Lys Asp
   735 740 745
   Trp Val Trp Glu His Phe Ser Ser Met Glu Lys Glu Asp Gln Ser Leu
   750 755 760
   Lys Phe Cys Leu Glu Glu Arg Asp Phe Glu Ala Gly Val Phe Glu Leu
   765 770 775
   Glu Ala Ile Val Asn Ser Ile Lys Arg Ser Arg Lys Ile Ile Phe Val
   780 785 790 795
   Ile Thr His His Leu Leu Lys Asp Pro Leu Cys Lys Arg Phe Lys Val
                   800                 805                 810
   His His Ala Val Gln Gln Ala Ile Glu Gln Asn Leu Asp Ser Ile Ile
               815                 820                 825
   Leu Val Phe Leu Glu Glu Ile Pro Asp Tyr Lys Leu Asn His Ala Leu
           830                 835                 840
   Cys Leu Arg Arg Gly Met Phe Lys Ser His Cys Ile Leu Asn Trp Pro
       845                 850                 855
   Val Gln Lys Glu Arg Ile Gly Ala Phe Arg His Lys Leu Gln Val Ala
   860                 865                 870                 875
   Leu Gly Ser Lys Asn Ser Val His
                   880
(2) INFORMATION FOR SEQ ID NO:7:
   (i) SEQUENCE CHARACTERISTICS:
      (A) LENGTH: 2400 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..2397
   (xi) SEQUENCE DESCRIPTION: SEQ ID NO:7:
   ATG GAG CTG AAT TTC TAC AAA ATC CCC GAC AAC CTC CCC TTC TCA ACC        48
   Met Glu Leu Asn Phe Tyr Lys Ile Pro Asp Asn Leu Pro Phe Ser Thr
    1               5                  10                  15
   AAG AAC CTG GAC CTG AGC TTT AAT CCC CTG AGG CAT TTA GGC AGC TAT        96
   Lys Asn Leu Asp Leu Ser Phe Asn Pro Leu Arg His Leu Gly Ser Tyr
               20                  25                  30
   AGC TTC TTC AGT TTC CCA GAA CTG CAG GTG CTG GAT TTA TCC AGG TGT       144
   Ser Phe Phe Ser Phe Pro Glu Leu Gln Val Leu Asp Leu Ser Arg Cys
           35                  40                  45
   GAA ATC CAG ACA ATT GAA GAT GGG GCA TAT CAG AGC CTA AGC CAC CTC       192
   Glu Ile Gln Thr Ile Glu Asp Gly Ala Tyr Gln Ser Leu Ser His Leu
       50                  55                  60
   TCT ACC TTA ATA TTG ACA GGA AAC CCC ATC CAG AGT TTA GCC CTG GGA       240
   Ser Thr Leu Ile Leu Thr Gly Asn Pro Ile Gln Ser Leu Ala Leu Gly
   65                  70                  75                  80
   GCC TTT TCT GGA CTA TCA AGT TTA CAG AAG CTG GTG GCT GTG GAG ACA       288
   Ala Phe Ser Gly Leu Ser Ser Leu Gln Lys Leu Val Ala Val Glu Thr
                   85                  90                  95
   AAT CTA GCA TCT CTA GAG AAC TTC CCC ATT GGA CAT CTC AAA ACT TTG      336
   Asn Leu Ala Ser Leu Glu Asn Phe Pro Ile Gly His Leu Lys Thr Leu
               100                 105                 110
   AAA GAA CTT AAT GTG GCT CAC AAT CTT ATC CAA TCT TTC AAA TTA CCT      384
   Lys Glu Leu Asn Val Ala His Asn Leu Ile Gln Ser Phe Lys Leu Pro
           115                 120                 125
   GAG TAT TTT TCT AAT CTG ACC AAT CTA GAG CAC TTG GAC CTT TCC AGC      432
   Glu Tyr Phe Ser Asn Leu Thr Asn Leu Glu His Leu Asp Leu Ser Ser
       130                 135                 140
   AAC AAG ATT CAA AGT ATT TAT TGC ACA GAC TTG CGG GTT CTA CAT CAA      480
   Asn Lys Ile Gln Ser Ile Tyr Cys Thr Asp Leu Arg Val Leu His Gln
   145                 150                 155                 160
   ATG CCC CTA CTC AAT CTC TCT TTA GAC CTG TCC CTG AAC CCT ATG AAC      528
   Met Pro Leu Leu Asn Leu Ser Leu Asp Leu Ser Leu Asn Pro Met Asn
                   165                 170                 175
   TTT ATC CAA CCA GGT GCA TTT AAA GAA ATT AGG CTT CAT AAG CTG ACT      576
   Phe Ile Gln Pro Gly Ala Phe Lys Glu Ile Arg Leu His Lys Leu Thr
               180                 185                 190
   TTA AGA AAT AAT TTT GAT AGT TTA AAT GTA ATG AAA ACT TGT ATT CAA      624
   Leu Arg Asn Asn Phe Asp Ser Leu Asn Val Met Lys Thr Cys Ile Gln
           195                 200                 205
   GGT CTG GCT GGT TTA GAA GTC CAT CGT TTG GTT CTG GGA GAA TTT AGA      672
   Gly Leu Ala Gly Leu Glu Val His Arg Leu Val Leu Gly Glu Phe Arg
       210                 215                 220
   AAT GAA GGA AAC TTG GAA AAG TTT GAC AAA TCT GCT CTA GAG GGC CTG      720
   Asn Glu Gly Asn Leu Glu Lys Phe Asp Lys Ser Ala Leu Glu Gly Leu
   225                 230                 235                 240
   TGC AAT TTG ACC ATT GAA GAA TTC CGA TTA GCA TAC TTA GAC TAC TAC      768
   Cys Asn Leu Thr Ile Glu Glu Phe Arg Leu Ala Tyr Leu Asp Tyr Tyr
                   245                 250                 255
   CTC GAT GAT ATT ATT GAC TTA TTT AAT TGT TTG ACA AAT GTT TCT TCA      816
   Leu Asp Asp Ile Ile Asp Leu Phe Asn Cys Leu Thr Asn Val Ser Ser
               260                 265                 270
   TTT TCC CTG GTG AGT GTG ACT ATT GAA AGG GTA AAA GAC TTT TCT TAT      864
   Phe Ser Leu Val Ser Val Thr Ile Glu Arg Val Lys Asp Phe Ser Tyr
           275                 280                 285
   AAT TTC GGA TGG CAA CAT TTA GAA TTA GTT AAC TGT AAA TTT GGA CAG      912
   Asn Phe Gly Trp Gln His Leu Glu Leu Val Asn Cys Lys Phe Gly Gln
       290                 295                 300
   TTT CCC ACA TTG AAA CTC AAA TCT CTC AAA AGG CTT ACT TTC ACT TCC      960
   Phe Pro Thr Leu Lys Leu Lys Ser Leu Lys Arg Leu Thr Phe Thr Ser
   305                 310                 315                 320
   AAC AAA GGT GGG AAT GCT TTT TCA GAA GTT GAT CTA CCA AGC CTT GAG     1008
   Asn Lys Gly Gly Asn Ala Phe Ser Glu Val Asp Leu Pro Ser Leu Glu
                   325                 330                 335
   TTT CTA GAT CTC AGT AGA AAT GGC TTG AGT TTC AAA GGT TGC TGT TCT     1056
   Phe Leu Asp Leu Ser Arg Asn Gly Leu Ser Phe Lys Gly Cys Cys Ser
               340                 345                 350
   CAA AGT GAT TTT GGG ACA ACC AGC CTA AAG TAT TTA GAT CTG AGC TTC      1104
   Gln Ser Asp Phe Gly Thr Thr Ser Leu Lys Tyr Leu Asp Leu Ser Phe
           355                 360                 365
   AAT GGT GTT ATT ACC ATG AGT TCA AAC TTC TTG GGC TTA GAA CAA CTA      1152
   Asn Gly Val Ile Thr Met Ser Ser Asn Phe Leu Gly Leu Glu Gln Leu
       370                 375                 380
   GAA CAT CTG GAT TTC CAG CAT TCC AAT TTG AAA CAA ATG AGT GAG TTT      1200
   Glu His Leu Asp Phe Gln His Ser Asn Leu Lys Gln Met Ser Glu Phe
   385                 390                 395                 400
   TCA GTA TTC CTA TCA CTC AGA AAC CTC ATT TAC CTT GAC ATT TCT CAT      1248
   Ser Val Phe Leu Ser Leu Arg Asn Leu Ile Tyr Leu Asp Ile Ser His
                   405                 410                 415
   ACT CAC ACC AGA GTT GCT TTC AAT GGC ATC TTC AAT GGC TTG TCC AGT      1296
   Thr His Thr Arg Val Ala Phe Asn Gly Ile Phe Asn Gly Leu Ser Ser
               420                 425                 430
   CTC GAA GTC TTG AAA ATG GCT GGC AAT TCT TTC CAG GAA AAC TTC CTT      1344
   Leu Glu Val Leu Lys Met Ala Gly Asn Ser Phe Gln Glu Asn Phe Leu
           435                 440                 445
   CCA GAT ATC TTC ACA GAG CTG AGA AAC TTG ACC TTC CTG GAC CTC TCT      1392
   Pro Asp Ile Phe Thr Glu Leu Arg Asn Leu Thr Phe Leu Asp Leu Ser
       450                 455                 460
   CAG TGT CAA CTG GAG CAG TTG TCT CCA ACA GCA TTT AAC TCA CTC TCC      1440
   Gln Cys Gln Leu Glu Gln Leu Ser Pro Thr Ala Phe Asn Ser Leu Ser
   465                 470                 475                 480
   AGT CTT CAG GTA CTA AAT ATG AGC CAC AAC AAC TTC TTT TCA TTG GAT      1488
   Ser Leu Gln Val Leu Asn Met Ser His Asn Asn Phe Phe Ser Leu Asp
                   485                 490                 495
   ACG TTT CCT TAT AAG TGT CTG AAC TCC CTC CAG GTT CTT GAT TAC AGT      1536
   Thr Phe Pro Tyr Lys Cys Leu Asn Ser Leu Gln Val Leu Asp Tyr Ser
               500                 505                 510
   CTC AAT CAC ATA ATG ACT TCC AAA AAA CAG GAA CTA CAG CAT TTT CCA      1584
   Leu Asn His Ile Met Thr Ser Lys Lys Gln Glu Leu Gln His Phe Pro
           515                 520                 525
   AGT AGT CTA GCT TTC TTA AAT CTT ACT CAG AAT GAC TTT GCT TGT ACT      1632
   Ser Ser Leu Ala Phe Leu Asn Leu Thr Gln Asn Asp Phe Ala Cys Thr
       530                 535                 540
   TGT GAA CAC CAG AGT TTC CTG CAA TGG ATC AAG GAC CAG AGG CAG CTC      1680
   Cys Glu His Gln Ser Phe Leu Gln Trp Ile Lys Asp Gln Arg Gln Leu
   545                 550                 555                 560
   TTG GTG GAA GTT GAA CGA ATG GAA TGT GCA ACA CCT TCA GAT AAG CAG      1728
   Leu Val Glu Val Glu Arg Met Glu Cys Ala Thr Pro Ser Asp Lys Gln
                   565                 570                 575
   GGC ATG CCT GTG CTG AGT TTG AAT ATC ACC TGT CAG ATG AAT AAG ACC      1776
   Gly Met Pro Val Leu Ser Leu Asn Ile Thr Cys Gln Met Asn Lys Thr
                   580                 585                 590
   ATC ATT GGT GTG TCG GTC CTC AGT GTG CTT GTA GTA TCT GTT GTA GCA      1824
   Ile Ile Gly Val Ser Val Leu Ser Val Leu Val Val Ser Val Val Ala
           595                 600                 605
   GTT CTG GTC TAT AAG TTC TAT TTT CAC CTG ATG CTT CTT GCT GGC TGC      1872
   Val Leu Val Tyr Lys Phe Tyr Phe His Leu Met Leu Leu Ala Gly Cys
       610                 615                 620
   ATA AAG TAT GGT AGA GGT GAA AAC ATC TAT GAT GCC TTT GTT ATC TAC      1920
   Ile Lys Tyr Gly Arg Gly Glu Asn Ile Tyr Asp Ala Phe Val Ile Tyr
   625                 630                 635                 640
   TCA AGC CAG GAT GAG GAC TGG GTA AGG AAT GAG CTA GTA AAG AAT TTA      1968
   Ser Ser Gln Asp Glu Asp Trp Val Arg Asn Glu Leu Val Lys Asn Leu
                   645                 650                 655
   GAA GAA GGG GTG CCT CCA TTT CAG CTC TGC CTT CAC TAC AGA GAC TTT      2016
   Glu Glu Gly Val Pro Pro Phe Gln Leu Cys Leu His Tyr Arg Asp Phe
               660                 665                 670
   ATT CCC GGT GTG GCC ATT GCT GCC AAC ATC ATC CAT GAA GGT TTC CAT      2064
   Ile Pro Gly Val Ala Ile Ala Ala Asn Ile Ile His Glu Gly Phe His
           675                 680                 685
   AAA AGC CGA AAG GTG ATT GTT GTG GTG TCC CAG CAC TTC ATC CAG AGC      2112
   Lys Ser Arg Lys Val Ile Val Val Val Ser Gln His Phe Ile Gln Ser
       690                 695                 700
   CGC TGG TGT ATC TTT GAA TAT GAG ATT GCT CAG ACC TGG CAG TTT CTG      2160
   Arg Trp Cys Ile Phe Glu Tyr Glu Ile Ala Gln Thr Trp Gln Phe Leu
   705                 710                 715                 720
   AGC AGT CGT GCT GGT ATC ATC TTC ATT GTC CTG CAG AAG GTG GAG AAG      2208
   Ser Ser Arg Ala Gly Ile Ile Phe Ile Val Leu Gln Lys Val Glu Lys
                   725                 730                 735
   ACC CTG CTC AGG CAG CAG GTG GAG CTG TAC CGC CTT CTC AGC AGG AAC      2256
   Thr Leu Leu Arg Gln Gln Val Glu Leu Tyr Arg Leu Leu Ser Arg Asn
               740                 745                 750
   ACT TAC CTG GAG TGG GAG GAC AGT GTC CTG GGG CGG CAC ATC TTC TGG      2304
   Thr Tyr Leu Glu Trp Glu Asp Ser Val Leu Gly Arg His Ile Phe Trp
           755                 760                 765
   AGA CGA CTC AGA AAA GCC CTG CTG GAT GGT AAA TCA TGG AAT CCA GAA      2352
   Arg Arg Leu Arg Lys Ala Leu Leu Asp Gly Lys Ser Trp Asn Pro Glu
       770                 775                 780
   GGA ACA GTG GGT ACA GGA TGC AAT TGG CAG GAA GCA ACA TCT ATC          2397
   Gly Thr Val Gly Thr Gly Cys Asn Trp Gln Glu Ala Thr Ser Ile
   785                 790                 795
   TGA                                                                  2400
(2) INFORMATION FOR SEQ ID NO:8:
   (i) SEQUENCE CHARACTERISTICS:
      (A) LENGTH: 799 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Glu Leu Asn Phe Tyr Lys Ile Pro Asp Asn Leu Pro Phe Ser Thr
    1               5                  10                  15
   Lys Asn Leu Asp Leu Ser Phe Asn Pro Leu Arg His Leu Gly Ser Tyr
               20                  25                  30
   Ser Phe Phe Ser Phe Pro Glu Leu Gln Val Leu Asp Leu Ser Arg Cys
           35                  40                  45
   Glu Ile Gln Thr Ile Glu Asp Gly Ala Tyr Gln Ser Leu Ser His Leu
       50                  55                  60
   Ser Thr Leu Ile Leu Thr Gly Asn Pro Ile Gln Ser Leu Ala Leu Gly
   65                  70                  75                  80
   Ala Phe Ser Gly Leu Ser Ser Leu Gln Lys Leu Val Ala Val Glu Thr
                   85                  90                  95
   Asn Leu Ala Ser Leu Glu Asn Phe Pro Ile Gly His Leu Lys Thr Leu
               100                 105                 110
   Lys Glu Leu Asn Val Ala His Asn Leu Ile Gln Ser Phe Lys Leu Pro
           115                 120                 125
   Glu Tyr Phe Ser Asn Leu Thr Asn Leu Glu His Leu Asp Leu Ser Ser
       130                 135                 140
   Asn Lys Ile Gln Ser Ile Tyr Cys Thr Asp Leu Arg Val Leu His Gln
   145                 150                 155                 160
   Met Pro Leu Leu Asn Leu Ser Leu Asp Leu Ser Leu Asn Pro Met Asn
                   165                 170                 175
   Phe Ile Gln Pro Gly Ala Phe Lys Glu Ile Arg Leu His Lys Leu Thr
               180                 185                 190
   Leu Arg Asn Asn Phe Asp Ser Leu Asn Val Met Lys Thr Cys Ile Gln
           195                 200                 205
   Gly Leu Ala Gly Leu Glu Val His Arg Leu Val Leu Gly Glu Phe Arg
       210                 215                 220
   Asn Glu Gly Asn Leu Glu Lys Phe Asp Lys Ser Ala Leu Glu Gly Leu
   225                 230                 235                 240
   Cys Asn Leu Thr Ile Glu Glu Phe Arg Leu Ala Tyr Leu Asp Tyr Tyr
                   245                 250                 255
   Leu Asp Asp Ile Ile Asp Leu Phe Asn Cys Leu Thr Asn Val Ser Ser
               260                 265                 270
   Phe Ser Leu Val Ser Val Thr Ile Glu Arg Val Lys Asp Phe Ser Tyr
           275                 280                 285
   Asn Phe Gly Trp Gln His Leu Glu Leu Val Asn Cys Lys Phe Gly Gln
       290                 295                 300
   Phe Pro Thr Leu Lys Leu Lys Ser Leu Lys Arg Leu Thr Phe Thr Ser
   305                 310                 315                 320
   Asn Lys Gly Gly Asn Ala Phe Ser Glu Val Asp Leu Pro Ser Leu Glu
                   325                 330                 335
   Phe Leu Asp Leu Ser Arg Asn Gly Leu Ser Phe Lys Gly Cys Cys Ser
               340                 345                 350
   Gln Ser Asp Phe Gly Thr Thr Ser Leu Lys Tyr Leu Asp Leu Ser Phe
           355                 360                 365
   Asn Gly Val Ile Thr Met Ser Ser Asn Phe Leu Gly Leu Glu Gln Leu
       370                 375                 380
   Glu His Leu Asp Phe Gln His Ser Asn Leu Lys Gln Met Ser Glu Phe
   385                 390                 395                 400
   Ser Val Phe Leu Ser Leu Arg Asn Leu Ile Tyr Leu Asp Ile Ser His
                   405                 410                 415
   Thr His Thr Arg Val Ala Phe Asn Gly Ile Phe Asn Gly Leu Ser Ser
               420                 425                 430
   Leu Glu Val Leu Lys Met Ala Gly Asn Ser Phe Gln Glu Asn Phe Leu
           435                 440                 445
   Pro Asp Ile Phe Thr Glu Leu Arg Asn Leu Thr Phe Leu Asp Leu Ser
       450                 455                 460
   Gln Cys Gln Leu Glu Gln Leu Ser Pro Thr Ala Phe Asn Ser Leu Ser
   465                 470                 475                 480
   Ser Leu Gln Val Leu Asn Met Ser His Asn Asn Phe Phe Ser Leu Asp
                   485                 490                 495
   Thr Phe Pro Tyr Lys Cys Leu Asn Ser Leu Gln Val Leu Asp Tyr Ser
               500                 505                 510
   Leu Asn His Ile Met Thr Ser Lys Lys Gln Glu Leu Gln His Phe Pro
           515                 520                 525
   Ser Ser Leu Ala Phe Leu Asn Leu Thr Gln Asn Asp Phe Ala Cys Thr
       530                 535                 540
   Cys Glu His Gln Ser Phe Leu Gln Trp Ile Lys Asp Gln Arg Gln Leu
   545                 550                 555                 560
   Leu Val Glu Val Glu Arg Met Glu Cys Ala Thr Pro Ser Asp Lys Gln
                   565                 570                 575
   Gly Met Pro Val Leu Ser Leu Asn Ile Thr Cys Gln Met Asn Lys Thr
               580                 585                 590
   Ile Ile Gly Val Ser Val Leu Ser Val Leu Val Val Ser Val Val Ala
           595                 600                 605
   Val Leu Val Tyr Lys Phe Tyr Phe His Leu Met Leu Leu Ala Gly Cys
           610                 615                 620
   Ile Lys Tyr Gly Arg Gly Glu Asn Ile Tyr Asp Ala Phe Val Ile Tyr
   625                 630                 635                 640
   Ser Ser Gln Asp Glu Asp Trp Val Arg Asn Glu Leu Val Lys Asn Leu
                   645                 650                 655
   Glu Glu Gly Val Pro Pro Phe Gln Leu Cys Leu His Tyr Arg Asp Phe
               660                 665                 670
   Ile Pro Gly Val Ala Ile Ala Ala Asn Ile Ile His Glu Gly Phe His
           675                 680                 685
   Lys Ser Arg Lys Val Ile Val Val Val Ser Gln His Phe Ile Gln Ser
       690                 695                 700
   Arg Trp Cys Ile Phe Glu Tyr Glu Ile Ala Gln Thr Trp Gln Phe Leu
   705                 710                 715                 720
   Ser Ser Arg Ala Gly Ile Ile Phe Ile Val Leu Gln Lys Val Glu Lys
                   725                 730                 735
   Thr Leu Leu Arg Gln Gln Val Glu Leu Tyr Arg Leu Leu Ser Arg Asn
               740                 745                 750
   Thr Tyr Leu Glu Trp Glu Asp Ser Val Leu Gly Arg His Ile Phe Trp
           755                 760                 765
   Arg Arg Leu Arg Lys Ala Leu Leu Asp Gly Lys Ser Trp Asn Pro Glu
       770                 775                 780
   Gly Thr Val Gly Thr Gly Cys Asn Trp Gln Glu Ala Thr Ser Ile
   785                 790                 795
(2) INFORMATION FOR SEQ ID NO:9:
   (i) SEQUENCE CHARACTERISTICS:
      (A) LENGTH: 1275 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1095
   (xi) SEQUENCE DESCRIPTION: SEQ ID NO:9:
   TGT TGG GAT GTT TTT GAG GGA CTT TCT CAT CTT CAA GTT CTG TAT TTG     48
   Cys Trp Asp Val Phe Glu Gly Leu Ser His Leu Gln Val Leu Tyr Leu
    1               5                  10                  15
   AAT CAT AAC TAT CTT AAT TCC CTT CCA CCA GGA GTA TTT AGC CAT CTG     96
   Asn His Asn Tyr Leu Asn Ser Leu Pro Pro Gly Val Phe Ser His Leu
               20                  25                  30
   ACT GCA TTA AGG GGA CTA AGC CTC AAC TCC AAC AGG CTG ACA GTT CTT     144
   Thr Ala Leu Arg Gly Leu Ser Leu Asn Ser Asn Arg Leu Thr Val Leu
            35                  40                  45
   TCT CAC AAT GAT TTA CCT GCT AAT TTA GAG ATC CTG GAC ATA TCC AGG     192
   Ser His Asn Asp Leu Pro Ala Asn Leu Glu Ile Leu Asp Ile Ser Arg
        50                  55                  60
   AAC CAG CTC CTA GCT CCT AAT CCT GAT GTA TTT GTA TCA CTT AGT GTC     240
   Asn Gln Leu Leu Ala Pro Asn Pro Asp Val Phe Val Ser Leu Ser Val
    65                  70                  75                  80
   TTG GAT ATA ACT CAT AAC AAG TTC ATT TGT GAA TGT GAA CTT AGC ACT     288
   Leu Asp Ile Thr His Asn Lys Phe Ile Cys Glu Cys Glu Leu Ser Thr
                    85                  90                  95
   TTT ATC AAT TGG CTT AAT CAC ACC AAT GTC ACT ATA GCT GGG CCT CCT     336
   Phe Ile Asn Trp Leu Asn His Thr Asn Val Thr Ile Ala Gly Pro Pro
               100                 105                 110
   GCA GAC ATA TAT TGT GTG TAC CCT GAC TCG TTC TCT GGG GTT TCC CTC     384
   Ala Asp Ile Tyr Cys Val Tyr Pro Asp Ser Phe Ser Gly Val Ser Leu
           115                 120                 125
   TTC TCT CTT TCC ACG GAA GGT TGT GAT GAA GAG GAA GTC TTA AAG TCC     432
   Phe Ser Leu Ser Thr Glu Gly Cys Asp Glu Glu Glu Val Leu Lys Ser
       130                 135                 140
   CTA AAG TTC TCC CTT TTC ATT GTA TGC ACT GTC ACT CTG ACT CTG TTC     480
   Leu Lys Phe Ser Leu Phe Ile Val Cys Thr Val Thr Leu Thr Leu Phe
   145                 150                 155                 160
   CTC ATG ACC ATC CTC ACA GTC ACA AAG TTC CGG GGC TTC TGT TTT ATC     528
   Leu Met Thr Ile Leu Thr Val Thr Lys Phe Arg Gly Phe Cys Phe Ile
                   165                 170                 175
   TGT TAT AAG ACA GCC CAG AGA CTG GTG TTC AAG GAC CAT CCC CAG GGC     576
   Cys Tyr Lys Thr Ala Gln Arg Leu Val Phe Lys Asp His Pro Gln Gly
               180                 185                 190
   ACA GAA CCT GAT ATG TAC AAA TAT GAT GCC TAT TTG TGC TTC AGC AGC     624
   Thr Glu Pro Asp Met Tyr Lys Tyr Asp Ala Tyr Leu Cys Phe Ser Ser
           195                 200                 205
   AAA GAC TTC ACA TGG GTG CAG AAT GCT TTG CTC AAA CAC CTG GAC ACT     672
   Lys Asp Phe Thr Trp Val Gln Asn Ala Leu Leu Lys His Leu Asp Thr
       210                 215                 220
   CAA TAC AGT GAC CAA AAC AGA TTC AAC CTG TGC TTT GAA GAA AGA GAC     720
   Gln Tyr Ser Asp Gln Asn Arg Phe Asn Leu Cys Phe Glu Glu Arg Asp
   225                 230                 235                 240
   TTT GTC CCA GGA GAA AAC CGC ATT GCC AAT ATC CAG GAT GCC ATC TGG     768
   Phe Val Pro Gly Glu Asn Arg Ile Ala Asn Ile Gln Asp Ala Ile Trp
                   245                 250                 255
   AAC AGT AGA AAG ATC GTT TGT CTT GTG AGC AGA CAC TTC CTT AGA GAT     816
   Asn Ser Arg Lys Ile Val Cys Leu Val Ser Arg His Phe Leu Arg Asp
               260                 265                 270
   GGC TGG TGC CTT GAA GCC TTC AGT TAT GCC CAG GGC AGG TGC TTA TCT     864
   Gly Trp Cys Leu Glu Ala Phe Ser Tyr Ala Gln Gly Arg Cys Leu Ser
           275                 280                 285
   GAC CTT AAC AGT GCT CTC ATC ATG GTG GTG GTT GGG TCC TTG TCC CAG        912
   Asp Leu Asn Ser Ala Leu Ile Met Val Val Val Gly Ser Leu Ser Gln
       290                 295                 300
   TAC CAG TTG ATG AAA CAT CAA TCC ATC AGA GGC TTT GTA CAG AAA CAG        960
   Tyr Gln Leu Met Lys His Gln Ser Ile Arg Gly Phe Val Gln Lys Gln
   305                 310                 315                 320
   CAG TAT TTG AGG TGG CCT GAG GAT CTC CAG GAT GTT GGC TGG TTT CTT       1008
   Gln Tyr Leu Arg Trp Pro Glu Asp Leu Gln Asp Val Gly Trp Phe Leu
                   325                 330                 335
   CAT AAA CTC TCT CAA CAG ATA CTA AAG AAA GAA AAG GAA AAG AAG AAA       1056
   His Lys Leu Ser Gln Gln Ile Leu Lys Lys Glu Lys Glu Lys Lys Lys
               340                 345                 350
   GAC AAT AAC ATT CCG TTG CAA ACT GTA GCA ACC ATC TCC TAATCAAAGG        1105
   Asp Asn Asn Ile Pro Leu Gln Thr Val Ala Thr Ile Ser
           355                 360                 365
   AGCAATTTCC AACTTATCTC AAGCCACAAA TAACTCTTCA CTTTGTATTT GCACCAAGTT    1165
   ATCATTTTGG GGTCCTCTCT GGAGGTTTTT TTTTTCTTTT TGCTACTATG AAAACAACAT    1225
   AAATCTCTCA ATTTTCGTAT CAAAAAAAAA AAAAAAAAAA TGGCGGCCGC               1275
(2) INFORMATION FOR SEQ ID NO:10:
   (i) SEQUENCE CHARACTERISTICS:
      (A) LENGTH: 365 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Cys Trp Asp Val Phe Glu Gly Leu Ser His Leu Gln Val Leu Tyr Leu
    1               5                  10                  15
   Asn His Asn Tyr Leu Asn Ser Leu Pro Pro Gly Val Phe Ser His Leu
               20                  25                  30
   Thr Ala Leu Arg Gly Leu Ser Leu Asn Ser Asn Arg Leu Thr Val Leu
           35                  40                  45
   Ser His Asn Asp Leu Pro Ala Asn Leu Glu Ile Leu Asp Ile Ser Arg
       50                  55                  60
   Asn Gln Leu Leu Ala Pro Asn Pro Asp Val Phe Val Ser Leu Ser Val
   65                  70                  75                  80
   Leu Asp Ile Thr His Asn Lys Phe Ile Cys Glu Cys Glu Leu Ser Thr
                   85                  90                  95
   Phe Ile Asn Trp Leu Asn His Thr Asn Val Thr Ile Ala Gly Pro Pro
               100                 105                 110
   Ala Asp Ile Tyr Cys Val Tyr Pro Asp Ser Phe Ser Gly Val Ser Leu
           115                 120                 125
   Phe Ser Leu Ser Thr Glu Gly Cys Asp Glu Glu Glu Val Leu Lys Ser
       130                 135                 140
   Leu Lys Phe Ser Leu Phe Ile Val Cys Thr Val Thr Leu Thr Leu Phe
   145                 150                 155                 160
   Leu Met Thr Ile Leu Thr Val Thr Lys Phe Arg Gly Phe Cys Phe Ile
                   165                 170                 175
   Cys Tyr Lys Thr Ala Gln Arg Leu Val Phe Lys Asp His Pro Gln Gly
               180                 185                 190
   Thr Glu Pro Asp Met Tyr Lys Tyr Asp Ala Tyr Leu Cys Phe Ser Ser
           195                 200                 205
   Lys Asp Phe Thr Trp Val Gln Asn Ala Leu Leu Lys His Leu Asp Thr
       210                 215                 220
   Gln Tyr Ser Asp Gln Asn Arg Phe Asn Leu Cys Phe Glu Glu Arg Asp
   225                 230                 235                 240
   Phe Val Pro Gly Glu Asn Arg Ile Ala Asn Ile Gln Asp Ala Ile Trp
                   245                 250                 255
   Asn Ser Arg Lys Ile Val Cys Leu Val Ser Arg His Phe Leu Arg Asp
               260                 265                 270
   Gly Trp Cys Leu Glu Ala Phe Ser Tyr Ala Gln Gly Arg Cys Leu Ser
           275                 280                 285
   Asp Leu Asn Ser Ala Leu Ile Met Val Val Val Gly Ser Leu Ser Gln
       290                 295                 300
   Tyr Gln Leu Met Lys His Gln Ser Ile Arg Gly Phe Val Gln Lys Gln
   305                 310                 315                 320
   Gln Tyr Leu Arg Trp Pro Glu Asp Leu Gln Asp Val Gly Trp Phe Leu
                   325                 330                 335
   His Lys Leu Ser Gln Gln Ile Leu Lys Lys Glu Lys Glu Lys Lys Lys
               340                 345                 350
   Asp Asn Asn Ile Pro Leu Gln Thr Val Ala Thr Ile Ser
           355                 360                 365
(2) INFORMATION FOR SEQ ID NO:11:
   (i) SEQUENCE CHARACTERISTICS:
      (A) LENGTH: 3138 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..3135
   (ix) FEATURE:
      (A) NAME/KEY: mat_peptide
      (B) LOCATION: 67..3135
   (xi) SEQUENCE DESCRIPTION: SEQ ID NO:11:
   ATG TGG ACA CTG AAG AGA CTA ATT CTT ATC CTT TTT AAC ATA ATC CTA      48
   ATT TCC AAA CTC CTT GGG GCT AGA TGG TTT CCT AAA ACT CTG CCC TGT      96
   GAT GTC ACT CTG GAT GTT CCA AAG AAC CAT GTG ATC GTG GAC TGC ACA     144
   GAC AAG CAT TTG ACA GAA ATT CCT GGA GGT ATT CCC ACG AAC ACC ACG     192
   AAC CTC ACC CTC ACC ATT AAC CAC ATA CCA GAC ATC TCC CCA GCG TCC     240
   TTT CAC AGA CTG GAC CAT CTG GTA GAG ATC GAT TTC AGA TGC AAC TGT     288
   GTA CCT ATT CCA CTG GGG TCA AAA AAC AAC ATG TGC ATC AAG AGG CTG     336
   CAG ATT AAA CCC AGA AGC TTT AGT GGA CTC ACT TAT TTA AAA TCC CTT     384
   TAC CTG GAT GGA AAC CAG CTA CTA GAG ATA CCG CAG GGC CTC CCG CCT     432
   AGC TTA CAG CTT CTC AGC CTT GAG GCC AAC AAC ATC TTT TCC ATC AGA     480
   AAA GAG AAT CTA ACA GAA CTG GCC AAC ATA GAA ATA CTC TAC CTG GGC     528
   CAA AAC TGT TAT TAT CGA AAT CCT TGT TAT GTT TCA TAT TCA ATA GAG     576
   AAA GAT GCC TTC CTA AAC TTG ACA AAG TTA AAA GTG CTC TCC CTG AAA     624
   GAT AAC AAT GTC ACA GCC GTC CCT ACT GTT TTG CCA TCT ACT TTA ACA      672
   GAA CTA TAT CTC TAC AAC AAC ATG ATT GCA AAA ATC CAA GAA GAT GAT      720
   TTT AAT AAC CTC AAC CAA TTA CAA ATT CTT GAC CTA AGT GGA AAT TGC      768
   CCT CGT TGT TAT AAT GCC CCA TTT CCT TGT GCG CCG TGT AAA AAT AAT      816
   TCT CCC CTA CAG ATC CCT GTA AAT GCT TTT GAT GCG CTG ACA GAA TTA      864
   AAA GTT TTA CGT CTA CAC AGT AAC TCT CTT CAG CAT GTG CCC CCA AGA      912
   TGG TTT AAG AAC ATC AAC AAA CTC CAG GAA CTG GAT CTG TCC CAA AAC      960
   TTC TTG GCC AAA GAA ATT GGG GAT GCT AAA TTT CTG CAT TTT CTC CCC     1008
   AGC CTC ATC CAA TTG GAT CTG TCT TTC AAT TTT GAA CTT CAG GTC TAT     1056
   CGT GCA TCT ATG AAT CTA TCA CAA GCA TTT TCT TCA CTG AAA AGC CTG     1104
   AAA ATT CTG CGG ATC AGA GGA TAT GTC TTT AAA GAG TTG AAA AGC TTT     1152
   AAC CTC TCG CCA TTA CAT AAT CTT CAA AAT CTT GAA GTT CTT GAT CTT     1200
   GGC ACT AAC TTT ATA AAA ATT GCT AAC CTC AGC ATG TTT AAA CAA TTT     1248
   AAA AGA CTG AAA GTC ATA GAT CTT TCA GTG AAT AAA ATA TCA CCT TCA     1296
   GGA GAT TCA AGT GAA GTT GGC TTC TGC TCA AAT GCC AGA ACT TCT GTA     1344
   GAA AGT TAT GAA CCC CAG GTC CTG GAA CAA TTA CAT TAT TTC AGA TAT     1392
   GAT AAG TAT GCA AGG AGT TGC AGA TTC AAA AAC AAA GAG GCT TCT TTC     1440
   ATG TCT GTT AAT GAA AGC TGC TAC AAG TAT GGG CAG ACC TTG GAT CTA     1488
   AGT AAA AAT AGT ATA TTT TTT GTC AAG TCC TCT GAT TTT CAG CAT CTT     1536
   TCT TTC CTC AAA TGC CTG AAT CTG TCA GGA AAT CTC ATT AGC CAA ACT     1584
   CTT AAT GGC AGT GAA TTC CAA CCT TTA GCA GAG CTG AGA TAT TTG GAC     1632
   TTC TCC AAC AAC CGG CTT GAT TTA CTC CAT TCA ACA GCA TTT GAA GAG     1680
   CTT CAC AAA CTG GAA GTT CTG GAT ATA AGC AGT AAT AGC CAT TAT TTT     1728
   CAA TCA GAA GGA ATT ACT CAT ATG CTA AAC TTT ACC AAG AAC CTA AAG     1776
   GTT CTG CAG AAA CTG ATG ATG AAC GAC AAT GAC ATC TCT TCC TCC ACC     1824
   AGC AGG ACC ATG GAG AGT GAG TCT CTT AGA ACT CTG GAA TTC AGA GGA     1872
   AAT CAC TTA GAT GTT TTA TGG AGA GAA GGT GAT AAC AGA TAC TTA CAA     1920
   TTA TTC AAG AAT CTG CTA AAA TTA GAG GAA TTA GAC ATC TCT AAA AAT     1968
   TCC CTA AGT TTC TTG CCT TCT GGA GTT TTT GAT GGT ATG CCT CCA AAT     2016
   CTA AAG AAT CTC TCT TTG GCC AAA AAT GGG CTC AAA TCT TTC AGT TGG     2064
   AAG AAA CTC CAG TGT CTA AAG AAC CTG GAA ACT TTG GAC CTC AGC CAC     2112
   AAC CAA CTG ACC ACT GTC CCT GAG AGA TTA TCC AAC TGT TCC AGA AGC     2160
   CTC AAG AAT CTG ATT CTT AAG AAT AAT CAA ATC AGG AGT CTG ACG AAG     2208
   TAT TTT CTA CAA GAT GCC TTC CAG TTG CGA TAT CTG GAT CTC AGC TCA     2256
   AAT AAA ATC CAG ATG ATC CAA AAG ACC AGC TTC CCA GAA AAT GTC CTC     2304
   AAC AAT CTG AAG ATG TTG CTT TTG CAT CAT AAT CGG TTT CTG TGC ACC     2352
   TGT GAT GCT GTG TGG TTT GTC TGG TGG GTT AAC CAT ACG GAG GTG ACT     2400
   ATT CCT TAC CTG GCC ACA GAT GTG ACT TGT GTG GGG CCA GGA GCA CAC     2448
   AAG GGC CAA AGT GTG ATC TCC CTG GAT CTG TAC ACC TGT GAG TTA GAT     2496
   CTG ACT AAC CTG ATT CTG TTC TCA CTT TCC ATA TCT GTA TCT CTC TTT     2544
   CTC ATG GTG ATG ATG ACA GCA AGT CAC CTC TAT TTC TGG GAT GTG TGG     2592
   TAT ATT TAC CAT TTC TGT AAG GCC AAG ATA AAG GGG TAT CAG CGT CTA     2640
   ATA TCA CCA GAC TGT TGC TAT GAT GCT TTT ATT GTG TAT GAC ACT AAA     2688
   GAC CCA GCT GTG ACC GAG TGG GTT TTG GCT GAG CTG GTG GCC AAA CTG     2736
   GAA GAC CCA AGA GAG AAA CAT TTT AAT TTA TGT CTC GAG GAA AGG GAC     2784
   TGG TTA CCA GGG CAG CCA GTT CTG GAA AAC CTT TCC CAG AGC ATA CAG     2832
   CTT AGC AAA AAG ACA GTG TTT GTG ATG ACA GAC AAG TAT GCA AAG ACT       2880
   GAA AAT TTT AAG ATA GCA TTT TAC TTG TCC CAT CAG AGG CTC ATG GAT       2928
   GAA AAA GTT GAT GTG ATT ATC TTG ATA TTT CTT GAG AAG CCC TTT CAG       2976
   AAG TCC AAG TTC CTC CAG CTC CGG AAA AGG CTC TGT GGG AGT TCT GTC       3024
   CTT GAG TGG CCA ACA AAC CCG CAA GCT CAC CCA TAC TTC TGG CAG TGT       3072
   CTA AAG AAC GCC CTG GCC ACA GAC AAT CAT GTG GCC TAT AGT CAG GTG       3120
   TTC AAG GAA ACG GTC TAG                                               3138
(2) INFORMATION FOR SEQ ID NO:12:
   (i) SEQUENCE CHARACTERISTICS:
      (A) LENGTH: 1045 amino acids
      (B) TYPE: amino acid
      (D) TOPOLOGY: linear
   (ii) MOLECULE TYPE: protein
   (xi) SEQUENCE DESCRIPTION: SEQ ID NO:12:
(2) INFORMATION FOR SEQ ID NO:13:
   (i) SEQUENCE CHARACTERISTICS:
      (A) LENGTH: 180 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..177
   (xi) SEQUENCE DESCRIPTION: SEQ ID NO:13:
   CTT GGA AAA CCT CTT CAG AAG TCT AAG TTT CTT CAG CTC AGG AAG AGA          48
   Leu Gly Lys Pro Leu Gln Lys Ser Lys Phe Leu Gln Leu Arg Lys Arg
     1               5                  10                  15
   CTC TGC AGG AGC TCT GTC CTT GAG TGG CCT GCA AAT CCA CAG GCT CAC          96
   Leu Cys Arg Ser Ser Val Leu Glu Trp Pro Ala Asn Pro Gln Ala His
                20                  25                  30
   CCA TAC TTC TGG CAG TGC CTG AAA AAT GCC CTG ACC ACA GAC AAT CAT         144
   Pro Tyr Phe Trp Gln Cys Leu Lys Asn Ala Leu Thr Thr Asp Asn His
            35                  40                  45
   GTG GCT TAT AGT CAA ATG TTC AAG GAA ACA GTC TAG                         180
   Val Ala Tyr Ser Gln Met Phe Lys Glu Thr Val
        50                  55
(2) INFORMATION FOR SEQ ID NO:14:
   (i) SEQUENCE CHARACTERISTICS:
      (A) LENGTH: 59 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Leu Gly Lys Pro Leu Gln Lys Ser Lys Phe Leu Gln Leu Arg Lys Arg
     1               5                  10                  15
   Leu Cys Arg Ser Ser Val Leu Glu Trp Pro Ala Asn Pro Gln Ala His
                20                  25                  30
   Pro Tyr Phe Trp Gln Cys Leu Lys Asn Ala Leu Thr Thr Asp Asn His
            35                  40                  45
   Val Ala Tyr Ser Gln Met Phe Lys Glu Thr Val
        50                  55
(2) INFORMATION FOR SEQ ID NO:15:
   (i) SEQUENCE CHARACTERISTICS:
      (A) LENGTH: 990 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 2..988
   (xi) SEQUENCE DESCRIPTION: SEQ ID NO:15:
   G AAT TCC AGA CTT ATA AAC TTG AAA AAT CTC TAT TTG GCC TGG AAC      46
     Asn Ser Arg Leu Ile Asn Leu Lys Asn Leu Tyr Leu Ala Trp Asn
       1           5                  10                  15
   TGC TAT TTT AAC AAA GTT TGC GAG AAA ACT AAC ATA GAA GAT GGA GTA    94
   Cys Tyr Phe Asn Lys Val Cys Glu Lys Thr Asn Ile Glu Asp Gly Val
                        20                  25                  30
   TTT GAA ACG CTG ACA AAT TTG GAG TTG CTA TCA CTA TCT TTC AAT TCT   142
   Phe Glu Thr Leu Thr Asn Leu Glu Leu Leu Ser Leu Ser Phe Asn Ser
                   35                  40                  45
   CTT TCA CAT GTG CCA CCC AAA CTG CCA AGC TCC CTA CGC AAA CTT TTT   190
   Leu Ser His Val Pro Pro Lys Leu Pro Ser Ser Leu Arg Lys Leu Phe
               50                  55                  60
   CTG AGC AAC ACC CAG ATC AAA TAC ATT AGT GAA GAA GAT TTC AAG GGA   238
   Leu Ser Asn Thr Gln Ile Lys Tyr Ile Ser Glu Glu Asp Phe Lys Gly
           65                  70                  75
   TTG ATA AAT TTA ACA TTA CTA GAT TTA AGC GGG AAC TGT CCG AGG TGC   286
   Leu Ile Asn Leu Thr Leu Leu Asp Leu Ser Gly Asn Cys Pro Arg Cys
    80                  85                  90                  95
   TTC AAT GCC CCA TTT CCA TGC GTG CCT TGT GAT GGT GGT GCT TCA ATT   334
   Phe Asn Ala Pro Phe Pro Cys Val Pro Cys Asp Gly Gly Ala Ser Ile
                   100                 105                 110
   AAT ATA GAT CGT TTT GCT TTT CAA AAC TTG ACC CAA CTT CGA TAC CTA   382
   Asn Ile Asp Arg Phe Ala Phe Gln Asn Leu Thr Gln Leu Arg Tyr Leu
               115                 120                 125
   AAC CTC TCT AGC ACT TCC CTC AGG AAG ATT AAT GCT GCC TGG TTT AAA   430
   Asn Leu Ser Ser Thr Ser Leu Arg Lys Ile Asn Ala Ala Trp Phe Lys
           130                 135                 140
   AAT ATG CCT CAT CTG AAG GTG CTG GAT CTT GAA TTC AAC TAT TTA GTG   478
   Asn Met Pro His Leu Lys Val Leu Asp Leu Glu Phe Asn Tyr Leu Val
       145                 150                 155
   GGA GAA ATA GCC TCT GGG GCA TTT TTA ACG ATG CTG CCC CGC TTA GAA   526
   Gly Glu Ile Ala Ser Gly Ala Phe Leu Thr Met Leu Pro Arg Leu Glu
   160                 165                 170                 175
   ATA CTT GAC TTG TCT TTT AAC TAT ATA AAG GGG AGT TAT CCA CAG CAT   574
   Ile Leu Asp Leu Ser Phe Asn Tyr Ile Lys Gly Ser Tyr Pro Gln His
                   180                 185                 190
   ATT AAT ATT TCC AGA AAC TTC TCT AAA CTT TTG TCT CTA CGG GCA TTG   622
   Ile Asn Ile Ser Arg Asn Phe Ser Lys Leu Leu Ser Leu Arg Ala Leu
                   195                     200                     205
   CAT TTA AGA GGT TAT GTG TTC CAG GAA CTC AGA GAA GAT GAT TTC CAG          670
   His Leu Arg Gly Tyr Val Phe Gln Glu Leu Arg Glu Asp Asp Phe Gln
           210                     215                     220
   CCC CTG ATG CAG CTT CCA AAC TTA TCG ACT ATC AAC TTG GGT ATT AAT          718
   Pro Leu Met Gln Leu Pro Asn Leu Ser Thr Ile Asn Leu Gly Ile Asn
       225                     230                     235
   TTT ATT AAG CAA ATC GAT TTC AAA CTT TTC CAA AAT TTC TCC AAT CTG          766
   Phe Ile Lys Gln Ile Asp Phe Lys Leu Phe Gln Asn Phe Ser Asn Leu
   240                     245                     250                     255
   GAA ATT ATT TAC TTG TCA GAA AAC AGA ATA TCA CCG TTG GTA AAA GAT          814
   Glu Ile Ile Tyr Leu Ser Glu Asn Arg Ile Ser Pro Leu Val Lys Asp
                   260                     265                     270
   ACC CGG CAG AGT TAT GCA AAT AGT TCC TCT TTT CAA CGT CAT ATC CGG          862
   Thr Arg Gln Ser Tyr Ala Asn Ser Ser Ser Phe Gln Arg His Ile Arg
               275                     280                     285
   AAA CGA CGC TCA ACA GAT TTT GAG TTT GAC CCA CAT TCG AAC TTT TAT          910
   Lys Arg Arg Ser Thr Asp Phe Glu Phe Asp Pro His Ser Asn Phe Tyr
           290                     295                     300
   CAT TTC ACC CGT CCT TTA ATA AAG CCA CAA TGT GCT GCT TAT GGA AAA          958
   His Phe Thr Arg Pro Leu Ile Lys Pro Gln Cys Ala Ala Tyr Gly Lys
       305                     310                     315
   GCC TTA GAT TTA AGC CTC AAC AGT ATT TTC TT                               990
   Ala Leu Asp Leu Ser Leu Asn Ser Ile Phe
   320                     325
(2) INFORMATION FOR SEQ ID NO:16:
   (i) SEQUENCE CHARACTERISTICS:
      (A) LENGTH: 329 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Asn Ser Arg Leu Ile Asn Leu Lys Asn Leu Tyr Leu Ala Trp Asn Cys
   1 5 10 15
   Tyr Phe Asn Lys Val Cys Glu Lys Thr Asn Ile Glu Asp Gly Val Phe
   20 25 30
   Glu Thr Leu Thr Asn Leu Glu Leu Leu Ser Leu Ser Phe Asn Ser Leu
   35 40 45
   Ser His Val Pro Pro Lys Leu Pro Ser Ser Leu Arg Lys Leu Phe Leu
   50 55 60
   Ser Asn Thr Gln Ile Lys Tyr Ile Ser Glu Glu Asp Phe Lys Gly Leu
   65 70 75 80
   Ile Asn Leu Thr Leu Leu Asp Leu Ser Gly Asn Cys Pro Arg Cys Phe
                   85                  90                  95
   Asn Ala Pro Phe Pro Cys Val Pro Cys Asp Gly Gly Ala Ser Ile Asn
               100                 105                 110
   Ile Asp Arg Phe Ala Phe Gln Asn Leu Thr Gln Leu Arg Tyr Leu Asn
           115                 120                 125
   Leu Ser Ser Thr Ser Leu Arg Lys Ile Asn Ala Ala Trp Phe Lys Asn
       130                 135                 140
   Met Pro His Leu Lys Val Leu Asp Leu Glu Phe Asn Tyr Leu Val Gly
   145                 150                 155                 160
   Glu Ile Ala Ser Gly Ala Phe Leu Thr Met Leu Pro Arg Leu Glu Ile
                   165                 170                 175
   Leu Asp Leu Ser Phe Asn Tyr Ile Lys Gly Ser Tyr Pro Gln His Ile
               180                 185                 190
   Asn Ile Ser Arg Asn Phe Ser Lys Leu Leu Ser Leu Arg Ala Leu His
           195                 200                 205
   Leu Arg Gly Tyr Val Phe Gln Glu Leu Arg Glu Asp Asp Phe Gln Pro
       210                 215                 220
   Leu Met Gln Leu Pro Asn Leu Ser Thr Ile Asn Leu Gly Ile Asn Phe
   225                 230                 235                 240
   Ile Lys Gln Ile Asp Phe Lys Leu Phe Gln Asn Phe Ser Asn Leu Glu
                   245                 250                 255
   Ile Ile Tyr Leu Ser Glu Asn Arg Ile Ser Pro Leu Val Lys Asp Thr
               260                 265                 270
   Arg Gln Ser Tyr Ala Asn Ser Ser Ser Phe Gln Arg His Ile Arg Lys
           275                 280                 285
   Arg Arg Ser Thr Asp Phe Glu Phe Asp Pro His Ser Asn Phe Tyr His
       290                 295                 300
   Phe Thr Arg Pro Leu Ile Lys Pro Gln Cys Ala Ala Tyr Gly Lys Ala
   305                 310                 315                 320
   Leu Asp Leu Ser Leu Asn Ser Ile Phe
                   325
(2) INFORMATION FOR SEQ ID NO:17:
   (i) SEQUENCE CHARACTERISTICS:
      (A) LENGTH: 1557 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..513
   (ix) FEATURE:
      (A) NAME/KEY: misc_feature
      (B) LOCATION: 278
      (D) OTHER INFORMATION: /note= "nucleotide 278 designated G, may be G or C"
   (ix) FEATURE:
      (A) NAME/KEY: misc_feature
      (B) LOCATION: 445
      (D) OTHER INFORMATION: /note= "nucleotide 445 designated A, may be A or T"
   (ix) FEATURE:
      (A) NAME/KEY: misc_feature
      (B) LOCATION: 572
      (D) OTHER INFORMATION: /note= "nucleotides 572, 593, 600, 607, 617, 622, 625, 631, 640, 646, 653, 719, 775, and 861 are designated C; each may be A, C, G, or T"
   (xi) SEQUENCE DESCRIPTION: SEQ ID NO:17:
   CAG TCT CTT TCC ACA TCC CAA ACT TTC TAT GAT GCT TAC ATT TCT TAT        48
   Gln Ser Leu Ser Thr Ser Gln Thr Phe Tyr Asp Ala Tyr Ile Ser Tyr
     1               5                  10                  15
   GAC ACC AAA GAT GCC TCT GTT ACT GAC TGG GTG ATA AAT GAG CTG CGC        96
   Asp Thr Lys Asp Ala Ser Val Thr Asp Trp Val Ile Asn Glu Leu Arg
                20                  25                  30
   TAC CAC CTT GAA GAG AGC CGA GAC AAA AAC GTT CTC CTT TGT CTA GAG       144
   Tyr His Leu Glu Glu Ser Arg Asp Lys Asn Val Leu Leu Cys Leu Glu
            35                  40                  45
   GAG AGG GAT TGG GAC CCG GGA TTG GCC ATC ATC GAC AAC CTC ATG CAG       192
   Glu Arg Asp Trp Asp Pro Gly Leu Ala Ile Ile Asp Asn Leu Met Gln
        50                  55                  60
   AGC ATC AAC CAA AGC AAG AAA ACA GTA TTT GTT TTA ACC AAA AAA TAT       240
   Ser Ile Asn Gln Ser Lys Lys Thr Val Phe Val Leu Thr Lys Lys Tyr
    65                  70                  75                  80
   GCA AAA AGC TGG AAC TTT AAA ACA GCT TTT TAC TTG GGC TTG CAG AGG       288
   Ala Lys Ser Trp Asn Phe Lys Thr Ala Phe Tyr Leu Gly Leu Gln Arg
                    85                  90                  95
   CTA ATG GGT GAG AAC ATG GAT GTG ATT ATA TTT ATC CTG CTG GAG CCA       336
   Leu Met Gly Glu Asn Met Asp Val Ile Ile Phe Ile Leu Leu Glu Pro
               100                 105                 110
   GTG TTA CAG CAT TCT CCG TAT TTG AGG CTA CGG CAG CGG ATC TGT AAG       384
   Val Leu Gln His Ser Pro Tyr Leu Arg Leu Arg Gln Arg Ile Cys Lys
           115                 120                 125
   AGC TCC ATC CTC CAG TGG CCT GAC AAC CCG AAG GCA GAA AGG TTG TTT       432
   Ser Ser Ile Leu Gln Trp Pro Asp Asn Pro Lys Ala Glu Arg Leu Phe
       130                 135                 140
   TGG CAA ACT CTG AGA AAT GTG GTC TTG ACT GAA AAT GAT TCA CGG TAT       480
   Trp Gln Thr Leu Arg Asn Val Val Leu Thr Glu Asn Asp Ser Arg Tyr
   145                 150                 155                 160
   AAC AAT ATG TAT GTC GAT TCC ATT AAG CAA TAC TAACTGACGT TAAGTCATGA    533
   Asn Asn Met Tyr Val Asp Ser Ile Lys Gln Tyr
                   165                 170
   TTTCGCGCCA TAATAAAGAT GCAAAGGAAT GACATTTCCG TATTAGTTAT CTATTGCTAC    593
   GGTAACCAAA TTACTCCCAA AAACCTTACG TCGGTTTCAA AACAACCACA TTCTGCTGGC    653
   CCCACAGTTT TTGAGGGTCA GGAGTCCAGG CCCAGCATAA CTGGGTCTTC TGCTTCAGGG    713
   TGTCTCCAGA GGCTGCAATG TAGGTGTTCA CCAGAGACAT AGGCATCACT GGGGTCACAC    773
   TCCATGTGGT TGTTTTCTGG ATTCAATTCC TCCTGGGCTA TTGGCCAAAG GCTATACTCA    833
   TGTAAGCCAT GCGAGCCTAT CCCACAACGG CAGCTTGCTT CATCAGAGCT AGCAAAAAAG    893
   AGAGGTTGCT AGCAAGATGA AGTCACAATC TTTTGTAATC GAATCAAAAA AGTGATATCT    953
   CATCACTTTG GCCATATTCT ATTTGTTAGA AGTAAACCAC AGGTCCCACC AGCTCCATGG   1013
   GAGTGACCAC CTCAGTCCAG GGAAAACAGC TGAAGACCAA GATGGTGAGC TCTGATTGCT   1073
   TCAGTTGGTC ATCAACTATT TTCCCTTGAC TGCTGTCCTG GGATGGCCGG CTATCTTGAT   1133
   GGATAGATTG TGAATATCAG GAGGCCAGGG ATCACTGTGG ACCATCTTAG CAGTTGACCT   1193
   AACACATCTT CTTTTCAATA TCTAAGAACT TTTGCCACTG TGACTAATGG TCCTAATATT   1253
   AAGCTGTTGT TTATATTTAT CATATATCTA TGGCTACATG GTTATATTAT GCTGTGGTTG   1313
   CGTTCGGTTT TATTTACAGT TGCTTTTACA AATATTTGCT GTAACATTTG ACTTCTAAGG   1373
   TTTAGATGCC ATTTAAGAAC TGAGATGGAT AGCTTTTAAA GCATCTTTTA CTTCTTACCA   1433
   TTTTTTAAAA GTATGCAGCT AAATTCGAAG CTTTTGGTCT ATATTGTTAA TTGCCATTGC   1493
   TGTAAATCTT AAAATGAATG AATAAAAATG TTTCATTTTA AAAAAAAAAA AAAAAAAAAA   1553
   AAAA                                                                1557
(2) INFORMATION FOR SEQ ID NO:18:
   (i) SEQUENCE CHARACTERISTICS:
      (A) LENGTH: 171 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Gln Ser Leu Ser Thr Ser Gln Thr Phe Tyr Asp Ala Tyr Ile Ser Tyr
     1               5                  10                  15
   Asp Thr Lys Asp Ala Ser Val Thr Asp Trp Val Ile Asn Glu Leu Arg
               20                  25                  30
   Tyr His Leu Glu Glu Ser Arg Asp Lys Asn Val Leu Leu Cys Leu Glu
           35                  40                  45
   Glu Arg Asp Trp Asp Pro Gly Leu Ala Ile Ile Asp Asn Leu Met Gln
       50                  55                  60
   Ser Ile Asn Gln Ser Lys Lys Thr Val Phe Val Leu Thr Lys Lys Tyr
   65                  70                  75                  80
   Ala Lys Ser Trp Asn Phe Lys Thr Ala Phe Tyr Leu Gly Leu Gln Arg
                   85                  90                  95
   Leu Met Gly Glu Asn Met Asp Val Ile Ile Phe Ile Leu Leu Glu Pro
               100                 105                 110
   Val Leu Gln His Ser Pro Tyr Leu Arg Leu Arg Gln Arg Ile Cys Lys
           115                 120                 125
   Ser Ser Ile Leu Gln Trp Pro Asp Asn Pro Lys Ala Glu Arg Leu Phe
       130                 135                 140
   Trp Gln Thr Leu Arg Asn Val Val Leu Thr Glu Asn Asp Ser Arg Tyr
   145                 150                 155                 160
   Asn Asn Met Tyr Val Asp Ser Ile Lys Gln Tyr
                   165                 170
(2) INFORMATION FOR SEQ ID NO:19:
   (i) SEQUENCE CHARACTERISTICS:
      (A) LENGTH: 629 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..486
   (ix) FEATURE:
      (A) NAME/KEY: misc_feature
      (B) LOCATION: 144
      (D) OTHER INFORMATION: /note= "nucleotides 144 and 225 designated C; may be C or T"
   (xi) SEQUENCE DESCRIPTION: SEQ ID NO:19:
   AAT GAA TTG ATC CCC AAT CTA GAG AAG GAA GAT GGT TCT ATC TTG ATT       48
   Asn Glu Leu Ile Pro Asn Leu Glu Lys Glu Asp Gly Ser Ile Leu Ile
     1               5                  10                  15
   TGC CTT TAT GAA AGC TAC TTT GAC CCT GGC AAA AGC ATT AGT GAA AAT       96
   Cys Leu Tyr Glu Ser Tyr Phe Asp Pro Gly Lys Ser Ile Ser Glu Asn
                20                  25                  30
   ATT GTA AGC TTC ATT GAG AAA AGC TAT AAG TCC ATC TTT GTT TTG TCC      144
   Ile Val Ser Phe Ile Glu Lys Ser Tyr Lys Ser Ile Phe Val Leu Ser
            35                  40                  45
   CCC AAC TTT GTC CAG AAT GAG TGG TGC CAT TAT GAA TTC TAC TTT GCC      192
   Pro Asn Phe Val Gln Asn Glu Trp Cys His Tyr Glu Phe Tyr Phe Ala
        50                  55                  60
   CAC CAC AAT CTC TTC CAT GAA AAT TCT GAT CAC ATA ATT CTT ATC TTA      240
   His His Asn Leu Phe His Glu Asn Ser Asp His Ile Ile Leu Ile Leu
    65                  70                  75                  80
   CTG GAA CCC ATT CCA TTC TAT TGC ATT CCC ACC AGG TAT CAT AAA CTG      288
   Leu Glu Pro Ile Pro Phe Tyr Cys Ile Pro Thr Arg Tyr His Lys Leu
                    85                  90                  95
   GAA GCT CTC CTG GAA AAA AAA GCA TAC TTG GAA TGG CCC AAG GAT AGG      336
   Glu Ala Leu Leu Glu Lys Lys Ala Tyr Leu Glu Trp Pro Lys Asp Arg
               100                 105                 110
   CGT AAA TGT GGG CTT TTC TGG GCA AAC CTT CGA GCT GCT GTT AAT GTT      384
   Arg Lys Cys Gly Leu Phe Trp Ala Asn Leu Arg Ala Ala Val Asn Val
           115                 120                 125
   AAT GTA TTA GCC ACC AGA GAA ATG TAT GAA CTG CAG ACA TTC ACA GAG      432
   Asn Val Leu Ala Thr Arg Glu Met Tyr Glu Leu Gln Thr Phe Thr Glu
       130                 135                 140
   TTA AAT GAA GAG TCT CGA GGT TCT ACA ATC TCT CTG ATG AGA ACA GAC      480
   Leu Asn Glu Glu Ser Arg Gly Ser Thr Ile Ser Leu Met Arg Thr Asp
   145                 150                 155                 160
   TGT CTA TAAAATCCCA CAGTCCTTGG GAAGTTGGGG ACCACATACA CTGTTGGGAT       536
   Cys Leu
   GTACATTGAT ACAACCTTTA TGATGGCAAT TTGACAATAT TTATTAAAAT AAAAAATGGT    596
   TATTCCCTTC AAAAAAAAAA AAAAAAAAAA AAA                                 629
(2) INFORMATION FOR SEQ ID NO:20:
   (i) SEQUENCE CHARACTERISTICS:
      (A) LENGTH: 162 amino acids
      (B) TYPE: amino acid
      (D) TOPOLOGY: linear
   (ii) MOLECULE TYPE: protein
   (xi) SEQUENCE DESCRIPTION: SEQ ID NO:20:
   Asn Glu Leu Ile Pro Asn Leu Glu Lys Glu Asp Gly Ser Ile Leu Ile
    1               5                  10                  15
   Cys Leu Tyr Glu Ser Tyr Phe Asp Pro Gly Lys Ser Ile Ser Glu Asn
               20                  25                  30
   Ile Val Ser Phe Ile Glu Lys Ser Tyr Lys Ser Ile Phe Val Leu Ser
           35                  40                  45
   Pro Asn Phe Val Gln Asn Glu Trp Cys His Tyr Glu Phe Tyr Phe Ala
       50                  55                  60
   His His Asn Leu Phe His Glu Asn Ser Asp His Ile Ile Leu Ile Leu
   65                  70                  75                  80
   Leu Glu Pro Ile Pro Phe Tyr Cys Ile Pro Thr Arg Tyr His Lys Leu
                   85                  90                  95
   Glu Ala Leu Leu Glu Lys Lys Ala Tyr Leu Glu Trp Pro Lys Asp Arg
               100                 105                 110
   Arg Lys Cys Gly Leu Phe Trp Ala Asn Leu Arg Ala Ala Val Asn Val
           115                 120                 125
   Asn Val Leu Ala Thr Arg Glu Met Tyr Glu Leu Gln Thr Phe Thr Glu
       130                 135                 140
   Leu Asn Glu Glu Ser Arg Gly Ser Thr Ile Ser Leu Met Arg Thr Asp
   145                 150                 155                 160
   Cys Leu
(2) INFORMATION FOR SEQ ID NO:21:
   (i) SEQUENCE CHARACTERISTICS:
      (A) LENGTH: 427 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:21:
   AAG AAC TCC AAA GAA AAC CTC CAG TTT CAT GCT TTT ATT TCA TAT AGT      48
   Lys Asn Ser Lys Glu Asn Leu Gln Phe His Ala Phe Ile Ser Tyr Ser
     1               5                  10                  15
   GAA CAT GAT TCT GCC TGG GTG AAA AGT GAA TTG GTA CCT TAC CTA GAA      96
   Glu His Asp Ser Ala Trp Val Lys Ser Glu Leu Val Pro Tyr Leu Glu
                20                  25                  30
   AAA GAA GAT ATA CAG ATT TGT CTT CAT GAG AGA AAC TTT GTC CCT GGC     144
   Lys Glu Asp Ile Gln Ile Cys Leu His Glu Arg Asn Phe Val Pro Gly
            35                  40                  45
   AAG AGC ATT GTG GAA AAT ATC ATC AAC TGC ATT GAG AAG AGT TAC AAG     192
   Lys Ser Ile Val Glu Asn Ile Ile Asn Cys Ile Glu Lys Ser Tyr Lys
        50                  55                  60
   TCC ATC TTT GTT TTG TCT CCC AAC TTT GTC CAG AGT GAG TGG TGC CAT     240
   Ser Ile Phe Val Leu Ser Pro Asn Phe Val Gln Ser Glu Trp Cys His
    65                  70                  75                  80
   TAC GAA CTC TAT TTT GCC CAT CAC AAT CTC TTT CAT GAA GGA TCT AAT     288
   Tyr Glu Leu Tyr Phe Ala His His Asn Leu Phe His Glu Gly Ser Asn
                    85                  90                  95
   AAC TTA ATC CTC ATC TTA CTG GAA CCC ATT CCA CAG AAC AGC ATT CCC     336
   Asn Leu Ile Leu Ile Leu Leu Glu Pro Ile Pro Gln Asn Ser Ile Pro
               100                 105                 110
   AAC AAG TAC CAC AAG CTG AAG GCT CTC ATG ACG CAG CGG ACT TAT TTG     384
   Asn Lys Tyr His Lys Leu Lys Ala Leu Met Thr Gln Arg Thr Tyr Leu
           115                 120                 125
   CAG TGG CCC AAG GAG AAA AGC AAA CGT GGG CTC TTT TGG GCT             426
   Gln Trp Pro Lys Glu Lys Ser Lys Arg Gly Leu Phe Trp Ala
       130                 135                 140
   A                                                                   427
(2) INFORMATION FOR SEQ ID NO:22:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:22:
   Lys Asn Ser Lys Glu Asn Leu Gln Phe His Ala Phe Ile Ser Tyr Ser
     1               5                  10                  15
   Glu His Asp Ser Ala Trp Val Lys Ser Glu Leu Val Pro Tyr Leu Glu
                20                  25                  30
   Lys Glu Asp Ile Gln Ile Cys Leu His Glu Arg Asn Phe Val Pro Gly
            35                  40                  45
   Lys Ser Ile Val Glu Asn Ile Ile Asn Cys Ile Glu Lys Ser Tyr Lys
        50                  55                  60
   Ser Ile Phe Val Leu Ser Pro Asn Phe Val Gln Ser Glu Trp Cys His
    65                  70                  75                      80
   Tyr Glu Leu Tyr Phe Ala His His Asn Leu Phe His Glu Gly Ser Asn
                    85                  90                  95
   Asn Leu Ile Leu Ile Leu Leu Glu Pro Ile Pro Gln Asn Ser Ile Pro
               100                 105                 110
   Asn Lys Tyr His Lys Leu Lys Ala Leu Met Thr Gln Arg Thr Tyr Leu
           115                 120                 125
   Gln Trp Pro Lys Glu Lys Ser Lys Arg Gly Leu Phe Trp Ala
       130                 135                 140
(2) INFORMATION FOR SEQ ID NO:23:
   (i) SEQUENCE CHARACTERISTICS:
      (A) LENGTH: 662 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..627
   (ix) FEATURE:
      (A) NAME/KEY: misc_feature
      (B) LOCATION: 54
      (D) OTHER INFORMATION: /note= "nucleotides 54, 103, and 345 are designated A; each may be A or G"
   (ix) FEATURE:
      (A) NAME/KEY: misc_feature
      (B) LOCATION: 313
      (D) OTHER INFORMATION: /note= "nucleotide 313 designated G, may be G or T"
   (ix) FEATURE:
      (A) NAME/KEY: misc_feature
      (B) LOCATION: 316
      (D) OTHER INFORMATION: /note= "nucleotides 316, 380, 407, and 408 designated C; each may be A, C, G, or T"
   (xi) SEQUENCE DESCRIPTION: SEQ ID NO:23:
   GCT TCC ACC TGT GCC TGG CCT GGC TTC CCT GGC GGG GGC GGC AAA GTG       48
   GGC GAA ATG AGG ATG CCC TGC CCT ACG ATG CCT TCG TGG TCT TCG ACA       96
   AAA CGC AGA GCG CAG TGG CAG ACT GGG TGT ACA ACG AGC TTC GGG GGC      144
   AGC TGG AGG AGT GCC GTG GGC GCT GGG CAC TCC GCC TGT GCC TGG AGG      192
   AAC GCG ACT GGC TGC CTG GCA AAA CCC TCT TTG AGA ACC TGT GGG CCT      240
   CGG TCT ATG GCA GCC GCA AGA CGC TGT TTG TGC TGG CCC ACA CGG ACC      288
   GGG TCA GTG GTC TCT TGC GCG CCA GTT CTC CTG CTG GCC CAG CAG CGC      336
   CTG CTG GAA GAC CGC AAG GAC GTC GTG GTG CTG GTG ATC CTA ACG CCT      384
   GAC GGC CAA GCC TCC CGA CTA CCC GAT GCG CTG ACC AGC GCC TCT GCC        432
   GCC AGA GTG TCC TCC TCT GGC CCC ACC AGC CCA GTG GTC GCG CAG CTT        480
   CTG AGG CCA GCA TGC ATG GCC CTG ACC AGG GAC AAC CAC CAC TTC TAT        528
   AAC CGG AAC TTC TGC CAG GGA ACC CAC GGC CGA ATA GCC GTG AGC CGG        576
   AAT CCT GCA CGG TGC CAC CTC CAC ACA CAC CTA ACA TAT GCC TGC CTG        624
   ATC TGACCAACAC ATGCTCGCCA CCCTCACCAC ACACC                             662
(2) INFORMATION FOR SEQ ID NO:24:
   (i) SEQUENCE CHARACTERISTICS:
      (A) LENGTH: 209 amino acids
      (B) TYPE: amino acid
      (D) TOPOLOGY: linear
   (ii) MOLECULE TYPE: protein
   (xi) SEQUENCE DESCRIPTION: SEQ ID NO:24:
(2) INFORMATION FOR SEQ ID NO:25:
   (i) SEQUENCE CHARACTERISTICS:
      (A) LENGTH: 4865 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 107..2617
   (ix) FEATURE:
      (A) NAME/KEY: mat_peptide
      (B) LOCATION: 173..2617
   (ix) FEATURE:
      (A) NAME/KEY: misc_feature
      (B) LOCATION: 81
      (D) OTHER INFORMATION: /note= "nucleotides 81, 3144, 3205, and 3563 designated A, each may be A, C, G, or T"
   (ix) FEATURE:
      (A) NAME/KEY: misc_feature
      (B) LOCATION: 84
      (D) OTHER INFORMATION: /note= "nucleotide 84 designated C, may be C or G"
   (ix) FEATURE:
      (A) NAME/KEY: misc_feature
      (B) LOCATION: 739
      (D) OTHER INFORMATION: /note= "nucleotide 739 designated C, may be C or T"
   (ix) FEATURE:
      (A) NAME/KEY: misc_feature
      (B) LOCATION: 3132
      (D) OTHER INFORMATION: /note= "nucleotides 3132, 3532, 3538, and 3553 designated G, each may be G or T"
   (ix) FEATURE:
      (A) NAME/KEY: misc_feature
      (B) LOCATION: 3638
      (D) OTHER INFORMATION: /note= "nucleotide 3638 designated A, may be A or T"
   (ix) FEATURE:
      (A) NAME/KEY: misc_feature
      (B) LOCATION: 3677
      (D) OTHER INFORMATION: /note= "nucleotides 3677, 3685, and 3736 designated C, each may be A or C"
   (xi) SEQUENCE DESCRIPTION: SEQ ID NO:25:
   AAAATACTCC CTTGCCTCAA AAACTGCTCG GTCAAACGGT GATAGCAAAC CACGCATTCA      60
   CAGGGCCACT GCTGCTCACA AAACCAGTGA GGATGATGCC AGGATG ATG TCT GCC        115
                                                      Met Ser Ala
                                                      -22     -20
   TCG CGC CTG GCT GGG ACT CTG ATC CCA GCC ATG GCC TTC CTC TCC TGC       163
   Ser Arg Leu Ala Gly Thr Leu Ile Pro Ala Met Ala Phe Leu Ser Cys
                   -15                 -10                 -5
   GTG AGA CCA GAA AGC TGG GAG CCC TGC GTG GAG GTT CCT AAT ATT ACT       211
   Val Arg Pro Glu Ser Trp Glu Pro Cys Val Glu Val Pro Asn Ile Thr
               1               5                   10
   TAT CAA TGC ATG GAG CTG AAT TTC TAC AAA ATC CCC GAC AAC CTC CCC       259
   Tyr Gln Cys Met Glu Leu Asn Phe Tyr Lys Ile Pro Asp Asn Leu Pro
       15                  20                  25
   TTC TCA ACC AAG AAC CTG GAC CTG AGC TTT AAT CCC CTG AGG CAT TTA       307
   Phe Ser Thr Lys Asn Leu Asp Leu Ser Phe Asn Pro Leu Arg His Leu
   30                  35                  40                  45
   GGC AGC TAT AGC TTC TTC AGT TTC CCA GAA CTG CAG GTG CTG GAT TTA       355
   Gly Ser Tyr Ser Phe Phe Ser Phe Pro Glu Leu Gln Val Leu Asp Leu
                   50                  55                  60
   TCC AGG TGT GAA ATC CAG ACA ATT GAA GAT GGG GCA TAT CAG AGC CTA       403
   Ser Arg Cys Glu Ile Gln Thr Ile Glu Asp Gly Ala Tyr Gln Ser Leu
               65                  70                  75
   AGC CAC CTC TCT ACC TTA ATA TTG ACA GGA AAC CCC ATC CAG AGT TTA       451
   Ser His Leu Ser Thr Leu Ile Leu Thr Gly Asn Pro Ile Gln Ser Leu
           80                  85                  90
   GCC CTG GGA GCC TTT TCT GGA CTA TCA AGT TTA CAG AAG CTG GTG GCT       499
   Ala Leu Gly Ala Phe Ser Gly Leu Ser Ser Leu Gln Lys Leu Val Ala
       95                  100                 105
   GTG GAG ACA AAT CTA GCA TCT CTA GAG AAC TTC CCC ATT GGA CAT CTC       547
   Val Glu Thr Asn Leu Ala Ser Leu Glu Asn Phe Pro Ile Gly His Leu
   110                 115                 120                 125
   AAA ACT TTG AAA GAA CTT AAT GTG GCT CAC AAT CTT ATC CAA TCT TTC       595
   Lys Thr Leu Lys Glu Leu Asn Val Ala His Asn Leu Ile Gln Ser Phe
                   130                 135                 140
   AAA TTA CCT GAG TAT TTT TCT AAT CTG ACC AAT CTA GAG CAC TTG GAC        643
   Lys Leu Pro Glu Tyr Phe Ser Asn Leu Thr Asn Leu Glu His Leu Asp
               145                 150                 155
   CTT TCC AGC AAC AAG ATT CAA AGT ATT TAT TGC ACA GAC TTG CGG GTT        691
   Leu Ser Ser Asn Lys Ile Gln Ser Ile Tyr Cys Thr Asp Leu Arg Val
           160                 165                 170
   CTA CAT CAA ATG CCC CTA CTC AAT CTC TCT TTA GAC CTG TCC CTG AAC        739
   Leu His Gln Met Pro Leu Leu Asn Leu Ser Leu Asp Leu Ser Leu Asn
       175                 180                 185
   CCT ATG AAC TTT ATC CAA CCA GGT GCA TTT AAA GAA ATT AGG CTT CAT        787
   Pro Met Asn Phe Ile Gln Pro Gly Ala Phe Lys Glu Ile Arg Leu His
   190                 195                 200                 205
   AAG CTG ACT TTA AGA AAT AAT TTT GAT AGT TTA AAT GTA ATG AAA ACT        835
   Lys Leu Thr Leu Arg Asn Asn Phe Asp Ser Leu Asn Val Met Lys Thr
                   210                 215                 220
   TGT ATT CAA GGT CTG GCT GGT TTA GAA GTC CAT CGT TTG GTT CTG GGA        883
   Cys Ile Gln Gly Leu Ala Gly Leu Glu Val His Arg Leu Val Leu Gly
               225                 230                 235
   GAA TTT AGA AAT GAA GGA AAC TTG GAA AAG TTT GAC AAA TCT GCT CTA        931
   Glu Phe Arg Asn Glu Gly Asn Leu Glu Lys Phe Asp Lys Ser Ala Leu
           240                 245                 250
   GAG GGC CTG TGC AAT TTG ACC ATT GAA GAA TTC CGA TTA GCA TAC TTA        979
   Glu Gly Leu Cys Asn Leu Thr Ile Glu Glu Phe Arg Leu Ala Tyr Leu
       255                 260                 265
   GAC TAC TAC CTC GAT GAT ATT ATT GAC TTA TTT AAT TGT TTG ACA AAT       1027
   Asp Tyr Tyr Leu Asp Asp Ile Ile Asp Leu Phe Asn Cys Leu Thr Asn
   270                 275                 280                 285
   GTT TCT TCA TTT TCC CTG GTG AGT GTG ACT ATT GAA AGG GTA AAA GAC       1075
   Val Ser Ser Phe Ser Leu Val Ser Val Thr Ile Glu Arg Val Lys Asp
                   290                 295                 300
   TTT TCT TAT AAT TTC GGA TGG CAA CAT TTA GAA TTA GTT AAC TGT AAA       1123
   Phe Ser Tyr Asn Phe Gly Trp Gln His Leu Glu Leu Val Asn Cys Lys
               305                 310                 315
   TTT GGA CAG TTT CCC ACA TTG AAA CTC AAA TCT CTC AAA AGG CTT ACT       1171
   Phe Gly Gln Phe Pro Thr Leu Lys Leu Lys Ser Leu Lys Arg Leu Thr
           320                 325                 330
   TTC ACT TCC AAC AAA GGT GGG AAT GCT TTT TCA GAA GTT GAT CTA CCA       1219
   Phe Thr Ser Asn Lys Gly Gly Asn Ala Phe Ser Glu Val Asp Leu Pro
       335                 340                 345
   AGC CTT GAG TTT CTA GAT CTC AGT AGA AAT GGC TTG AGT TTC AAA GGT       1267
   Ser Leu Glu Phe Leu Asp Leu Ser Arg Asn Gly Leu Ser Phe Lys Gly
   350                 355                 360                 365
   TGC TGT TCT CAA AGT GAT TTT GGG ACA ACC AGC CTA AAG TAT TTA GAT       1315
   Cys Cys Ser Gln Ser Asp Phe Gly Thr Thr Ser Leu Lys Tyr Leu Asp
                   370                 375                 380
   CTG AGC TTC AAT GGT GTT ATT ACC ATG AGT TCA AAC TTC TTG GGC TTA    1363
   Leu Ser Phe Asn Gly Val Ile Thr Met Ser Ser Asn Phe Leu Gly Leu
               385                 390                 395
   GAA CAA CTA GAA CAT CTG GAT TTC CAG CAT TCC AAT TTG AAA CAA ATG    1411
   Glu Gln Leu Glu His Leu Asp Phe Gln His Ser Asn Leu Lys Gln Met
           400                 405                 410
   AGT GAG TTT TCA GTA TTC CTA TCA CTC AGA AAC CTC ATT TAC CTT GAC    1459
   Ser Glu Phe Ser Val Phe Leu Ser Leu Arg Asn Leu Ile Tyr Leu Asp
       415                 420                 425
   ATT TCT CAT ACT CAC ACC AGA GTT GCT TTC AAT GGC ATC TTC AAT GGC    1507
   Ile Ser His Thr His Thr Arg Val Ala Phe Asn Gly Ile Phe Asn Gly
   430                 435                 440                 445
   TTG TCC AGT CTC GAA GTC TTG AAA ATG GCT GGC AAT TCT TTC CAG GAA    1555
   Leu Ser Ser Leu Glu Val Leu Lys Met Ala Gly Asn Ser Phe Gln Glu
                   450                 455                 460
   AAC TTC CTT CCA GAT ATC TTC ACA GAG CTG AGA AAC TTG ACC TTC CTG    1603
   Asn Phe Leu Pro Asp Ile Phe Thr Glu Leu Arg Asn Leu Thr Phe Leu
               465                 470                 475
   GAC CTC TCT CAG TGT CAA CTG GAG CAG TTG TCT CCA ACA GCA TTT AAC    1651
   Asp Leu Ser Gln Cys Gln Leu Glu Gln Leu Ser Pro Thr Ala Phe Asn
           480                 485                 490
   TCA CTC TCC AGT CTT CAG GTA CTA AAT ATG AGC CAC AAC AAC TTC TTT    1699
   Ser Leu Ser Ser Leu Gln Val Leu Asn Met Ser His Asn Asn Phe Phe
       495                 500                 505
   TCA TTG GAT ACG TTT CCT TAT AAG TGT CTG AAC TCC CTC CAG GTT CTT    1747
   Ser Leu Asp Thr Phe Pro Tyr Lys Cys Leu Asn Ser Leu Gln Val Leu
   510                 515                 520                 525
   GAT TAC AGT CTC AAT CAC ATA ATG ACT TCC AAA AAA CAG GAA CTA CAG    1795
   Asp Tyr Ser Leu Asn His Ile Met Thr Ser Lys Lys Gln Glu Leu Gln
                   530                 535                 540
   CAT TTT CCA AGT AGT CTA GCT TTC TTA AAT CTT ACT CAG AAT GAC TTT    1843
   His Phe Pro Ser Ser Leu Ala Phe Leu Asn Leu Thr Gln Asn Asp Phe
               545                 550                 555
   GCT TGT ACT TGT GAA CAC CAG AGT TTC CTG CAA TGG ATC AAG GAC CAG    1891
   Ala Cys Thr Cys Glu His Gln Ser Phe Leu Gln Trp Ile Lys Asp Gln
           560                 565                 570
   AGG CAG CTC TTG GTG GAA GTT GAA CGA ATG GAA TGT GCA ACA CCT TCA    1939
   Arg Gln Leu Leu Val Glu Val Glu Arg Met Glu Cys Ala Thr Pro Ser
       575                 580                 585
   GAT AAG CAG GGC ATG CCT GTG CTG AGT TTG AAT ATC ACC TGT CAG ATG    1987
   Asp Lys Gln Gly Met Pro Val Leu Ser Leu Asn Ile Thr Cys Gln Met
   590                 595                 600                 605
   AAT AAG ACC ATC ATT GGT GTG TCG GTC CTC AGT GTG CTT GTA GTA TCT    2035
   Asn Lys Thr Ile Ile Gly Val Ser Val Leu Ser Val Leu Val Val Ser
                   610                 615                 620
   GTT GTA GCA GTT CTG GTC TAT AAG TTC TAT TTT CAC CTG ATG CTT CTT    2083
   Val Val Ala Val Leu Val Tyr Lys Phe Tyr Phe His Leu Met Leu Leu
                   625                 630                 635
   GCT GGC TGC ATA AAG TAT GGT AGA GGT GAA AAC ATC TAT GAT GCC TTT     2131
   Ala Gly Cys Ile Lys Tyr Gly Arg Gly Glu Asn Ile Tyr Asp Ala Phe
           640                 645                 650
   GTT ATC TAC TCA AGC CAG GAT GAG GAC TGG GTA AGG AAT GAG CTA GTA     2179
   Val Ile Tyr Ser Ser Gln Asp Glu Asp Trp Val Arg Asn Glu Leu Val
       655                 660                 665
   AAG AAT TTA GAA GAA GGG GTG CCT CCA TTT CAG CTC TGC CTT CAC TAC     2227
   Lys Asn Leu Glu Glu Gly Val Pro Pro Phe Gln Leu Cys Leu His Tyr
   670                 675                 680                 685
   AGA GAC TTT ATT CCC GGT GTG GCC ATT GCT GCC AAC ATC ATC CAT GAA     2275
   Arg Asp Phe Ile Pro Gly Val Ala Ile Ala Ala Asn Ile Ile His Glu
                   690                 695                 700
   GGT TTC CAT AAA AGC CGA AAG GTG ATT GTT GTG GTG TCC CAG CAC TTC     2323
   Gly Phe His Lys Ser Arg Lys Val Ile Val Val Val Ser Gln His Phe
               705                 710                 715
   ATC CAG AGC CGC TGG TGT ATC TTT GAA TAT GAG ATT GCT CAG ACC TGG     2371
   Ile Gln Ser Arg Trp Cys Ile Phe Glu Tyr Glu Ile Ala Gln Thr Trp
           720                 725                 730
   CAG TTT CTG AGC AGT CGT GCT GGT ATC ATC TTC ATT GTC CTG CAG AAG     2419
   Gln Phe Leu Ser Ser Arg Ala Gly Ile Ile Phe Ile Val Leu Gln Lys
       735                 740                 745
   GTG GAG AAG ACC CTG CTC AGG CAG CAG GTG GAG CTG TAC CGC CTT CTC     2467
   Val Glu Lys Thr Leu Leu Arg Gln Gln Val Glu Leu Tyr Arg Leu Leu
   750                 755                 760                 765
   AGC AGG AAC ACT TAC CTG GAG TGG GAG GAC AGT GTC CTG GGG CGG CAC     2515
   Ser Arg Asn Thr Tyr Leu Glu Trp Glu Asp Ser Val Leu Gly Arg His
                   770                 775                 780
   ATC TTC TGG AGA CGA CTC AGA AAA GCC CTG CTG GAT GGT AAA TCA TGG     2563
   Ile Phe Trp Arg Arg Leu Arg Lys Ala Leu Leu Asp Gly Lys Ser Trp
               785                 790                 795
   AAT CCA GAA GGA ACA GTG GGT ACA GGA TGC AAT TGG CAG GAA GCA ACA     2611
   Asn Pro Glu Gly Thr Val Gly Thr Gly Cys Asn Trp Gln Glu Ala Thr
           800                 805                 810
   TCT ATC TGAAGAGGAA AAATAAAAAC CTCCTGAGGC ATTTCTTGCC CAGCTGGGTC      2667
   Ser Ile
       815
   CAACACTTGT TCAGTTAATA AGTATTAAAT GCTGCCACAT GTCAGGCCTT ATGCTAAGGG   2727
   TGAGTAATTC CATGGTGCAC TAGATATGCA GGGCTGCTAA TCTCAAGGAG CTTCCAGTGC   2787
   AGAGGGAATA AATGCTAGAC TAAAATACAG AGTCTTCCAG GTGGGCATTT CAACCAACTC   2847
   AGTCAAGGAA CCCATGACAA AGAAAGTCAT TTCAACTCTT ACCTCATCAA GTTGAATAAA   2907
   GACAGAGAAA ACAGAAAGAG ACATTGTTCT TTTCCTGAGT CTTTTGAATG GAAATTGTAT   2967
   TATGTTATAG CCATCATAAA ACCATTTTGG TAGTTTTGAC TGAACTGGGT GTTCACTTTT 3027
   TCCTTTTTGA TTGAATACAA TTTAAATTCT ACTTGATGAC TGCAGTCGTC AAGGGGCTCC 3087
   TGATGCAAGA TGCCCCTTCC ATTTTAAGTC TGTCTCCTTA CAGAGGTTAA AGTCTAATGG 3147
   CTAATTCCTA AGGAAACCTG ATTAACACAT GCTCACAACC ATCCTGGTCA TTCTCGAACA 3207
   TGTTCTATTT TTTAACTAAT CACCCCTGAT ATATTTTTAT TTTTATATAT CCAGTTTTCA 3267
   TTTTTTTACG TCTTGCCTAT AAGCTAATAT CATAAATAAG GTTGTTTAAG ACGTGCTTCA 3327
   AATATCCATA TTAACCACTA TTTTTCAAGG AAGTATGGAA AAGTACACTC TGTCACTTTG 3387
   TCACTCGATG TCATTCCAAA GTTATTGCCT ACTAAGTAAT GACTGTCATG AAAGCAGCAT 3447
   TGAAATAATT TGTTTAAAGG GGGCACTCTT TTAAACGGGA AGAAAATTTC CGCTTCCTGG 3507
   TCTTATCATG GACAATTTGG GCTAGAGGCA GGAAGGAAGT GGGATGACCT CAGGAAGTCA 3567
   CCTTTTCTTG ATTCCAGAAA CATATGGGCT GATAAACCCG GGGTGACCTC ATGAAATGAG 3627
   TTGCAGCAGA AGTTTATTTT TTTCAGAACA AGTGATGTTT GATGGACCTC TGAATCTCTT 3687
   TAGGGAGACA CAGATGGCTG GGATCCCTCC CCTGTACCCT TCTCACTGCC AGGAGAACTA 3747
   CGTGTGAAGG TATTCAAGGC AGGGAGTATA CATTGCTGTT TCCTGTTGGG CAATGCTCCT 3807
   TGACCACATT TTGGGAAGAG TGGATGTTAT CATTGAGAAA ACAATGTGTC TGGAATTAAT 3867
   GGGGTTCTTA TAAAGAAGGT TCCCAGAAAA GAATGTTCAT TCCAGCTTCT TCAGGAAACA 3927
   GGAACATTCA AGGAAAAGGA CAATCAGGAT GTCATCAGGG AAATGAAAAT AAAAACCACA 3987
   ATGAGATATC ACCTTATACC AGGTAGATGG CTACTATAAA AAAATGAAGT GTCATCAAGG 4047
   ATATAGAGAA ATTGGAACCC TTCTTCACTG CTGGAGGGAA TGGAAAATGG TGTAGCCGTT 4107
   ATGAAAAACA GTACGGAGGT TTCTCAAAAA TTAAAAATAG AACTGCTATA TGATCCAGCA 4167
   ATCTCACTTC TGTATATATA CCCAAAATAA TTGAAATCAG AATTTCAAGA AAATATTTAC 4227
   ACTCCCATGT TCATTGTGGC ACTCTTCACA ATCACTGTTT CCAAAGTTAT GGAAACAACC 4287
   CAAATTTCCA TTGGAAAATA AATGGACAAA GGAAATGTGC ATATAACGTA CAATGGGGAT 4347
   ATTATTCAGC CTAAAAAAAG GGGGGATCCT GTTATTTATG ACAACATGAA TAAACCCGGA 4407
   GGCCATTATG CTATGTAAAA TGAGCAAGTA ACAGAAAGAC AAATACTGCC TGATTTCATT 4467
   TATATGAGGT TCTAAAATAG TCAAACTCAT AGAAGCAGAG AATAGAACAG TGGTTCCTAG 4527
   GGAAAAGGAG GAAGGGAGAA ATGAGGAAAT AGGGAGTTGT CTAATTGGTA TAAAATTATA 4587
   GTATGCAAGA TGAATTAGCT CTAAAGATCA GCTGTATAGC AGAGTTCGTA TAATGAACAA 4647
   TACTGTATTA TGCACTTAAC ATTTTGTTAA GAGGGTACCT CTCATGTTAA GTGTTCTTAC 4707
   CATATACATA TACACAAGGA AGCTTTTGGA GGTGATGGAT ATATTTATTA CCTTGATTGT 4767
   GGTGATGGTT TGACAGGTAT GTGACTATGT CTAAACTCAT CAAATTGTAT ACATTAAATA 4827
   TATGCAGTTT TATAATATCA AAAAAAAAAA AAAAAAAA 4865
(2) INFORMATION FOR SEQ ID NO:26:
   (i) SEQUENCE CHARACTERISTICS:
      (A) LENGTH: 837 amino acids
      (B) TYPE: amino acid
      (D) TOPOLOGY: linear
   (ii) MOLECULE TYPE: protein
   (xi) SEQUENCE DESCRIPTION: SEQ ID NO:26:
   Met Ser Ala Ser Arg Leu Ala Gly Thr Leu Ile Pro Ala Met Ala Phe
   -22     -20                 -15                 -10
   Leu Ser Cys Val Arg Pro Glu Ser Trp Glu Pro Cys Val Glu Val Pro
       -5                  1               5                   10
   Asn Ile Thr Tyr Gln Cys Met Glu Leu Asn Phe Tyr Lys Ile Pro Asp
                   15                  20                  25
   Asn Leu Pro Phe Ser Thr Lys Asn Leu Asp Leu Ser Phe Asn Pro Leu
               30                  35                  40
   Arg His Leu Gly Ser Tyr Ser Phe Phe Ser Phe Pro Glu Leu Gln Val
           45                  50                  55
   Leu Asp Leu Ser Arg Cys Glu Ile Gln Thr Ile Glu Asp Gly Ala Tyr
       60                  65                  70
   Gln Ser Leu Ser His Leu Ser Thr Leu Ile Leu Thr Gly Asn Pro Ile
   75                  80                  85                  90
   Gln Ser Leu Ala Leu Gly Ala Phe Ser Gly Leu Ser Ser Leu Gln Lys
                   95                  100                 105
   Leu Val Ala Val Glu Thr Asn Leu Ala Ser Leu Glu Asn Phe Pro Ile
               110                 115                 120
   Gly His Leu Lys Thr Leu Lys Glu Leu Asn Val Ala His Asn Leu Ile
           125                 130                 135
   Gln Ser Phe Lys Leu Pro Glu Tyr Phe Ser Asn Leu Thr Asn Leu Glu
       140                 145                 150
   His Leu Asp Leu Ser Ser Asn Lys Ile Gln Ser Ile Tyr Cys Thr Asp
   155                 160                 165                 170
   Leu Arg Val Leu His Gln Met Pro Leu Leu Asn Leu Ser Leu Asp Leu
                   175                 180                 185
   Ser Leu Asn Pro Met Asn Phe Ile Gln Pro Gly Ala Phe Lys Glu Ile
               190                 195                 200
   Arg Leu His Lys Leu Thr Leu Arg Asn Asn Phe Asp Ser Leu Asn Val
           205                 210                 215
   Met Lys Thr Cys Ile Gln Gly Leu Ala Gly Leu Glu Val His Arg Leu
           220                     225                     230
   Val Leu Gly Glu Phe Arg Asn Glu Gly Asn Leu Glu Lys Phe Asp Lys
   235                 240                 245                 250
   Ser Ala Leu Glu Gly Leu Cys Asn Leu Thr Ile Glu Glu Phe Arg Leu
                   255                 260                 265
   Ala Tyr Leu Asp Tyr Tyr Leu Asp Asp Ile Ile Asp Leu Phe Asn Cys
               270                 275                 280
   Leu Thr Asn Val Ser Ser Phe Ser Leu Val Ser Val Thr Ile Glu Arg
           285                 290                 295
   Val Lys Asp Phe Ser Tyr Asn Phe Gly Trp Gln His Leu Glu Leu Val
       300                 305                 310
   Asn Cys Lys Phe Gly Gln Phe Pro Thr Leu Lys Leu Lys Ser Leu Lys
   315                 320                 325                 330
   Arg Leu Thr Phe Thr Ser Asn Lys Gly Gly Asn Ala Phe Ser Glu Val
                   335                 340                 345
   Asp Leu Pro Ser Leu Glu Phe Leu Asp Leu Ser Arg Asn Gly Leu Ser
               350                 355                 360
   Phe Lys Gly Cys Cys Ser Gln Ser Asp Phe Gly Thr Thr Ser Leu Lys
           365                 370                 375
   Tyr Leu Asp Leu Ser Phe Asn Gly Val Ile Thr Met Ser Ser Asn Phe
       380                 385                 390
   Leu Gly Leu Glu Gln Leu Glu His Leu Asp Phe Gln His Ser Asn Leu
   395                 400                 405                 410
   Lys Gln Met Ser Glu Phe Ser Val Phe Leu Ser Leu Arg Asn Leu Ile
                   415                 420                 425
   Tyr Leu Asp Ile Ser His Thr His Thr Arg Val Ala Phe Asn Gly Ile
               430                 435                 440
   Phe Asn Gly Leu Ser Ser Leu Glu Val Leu Lys Met Ala Gly Asn Ser
           445                 450                 455
   Phe Gln Glu Asn Phe Leu Pro Asp Ile Phe Thr Glu Leu Arg Asn Leu
       460                 465                 470
   Thr Phe Leu Asp Leu Ser Gln Cys Gln Leu Glu Gln Leu Ser Pro Thr
   475                 480                 485                 490
   Ala Phe Asn Ser Leu Ser Ser Leu Gln Val Leu Asn Met Ser His Asn
                   495                 500                 505
   Asn Phe Phe Ser Leu Asp Thr Phe Pro Tyr Lys Cys Leu Asn Ser Leu
               510                 515                 520
   Gln Val Leu Asp Tyr Ser Leu Asn His Ile Met Thr Ser Lys Lys Gln
           525                 530                 535
   Glu Leu Gln His Phe Pro Ser Ser Leu Ala Phe Leu Asn Leu Thr Gln
       540                 545                 550
   Asn Asp Phe Ala Cys Thr Cys Glu His Gln Ser Phe Leu Gln Trp Ile
   555 560 565 570
   Lys Asp Gln Arg Gln Leu Leu Val Glu Val Glu Arg Met Glu Cys Ala
   575 580 585
   Thr Pro Ser Asp Lys Gln Gly Met Pro Val Leu Ser Leu Asn Ile Thr
   590 595 600
   Cys Gln Met Asn Lys Thr Ile Ile Gly Val Ser Val Leu Ser Val Leu
   605 610 615
   Val Val Ser Val Val Ala Val Leu Val Tyr Lys Phe Tyr Phe His Leu
   620 625 630
   Met Leu Leu Ala Gly Cys Ile Lys Tyr Gly Arg Gly Glu Asn Ile Tyr
   635 640 645 650
   Asp Ala Phe Val Ile Tyr Ser Ser Gln Asp Glu Asp Trp Val Arg Asn
   655 660 665
   Glu Leu Val Lys Asn Leu Glu Glu Gly Val Pro Pro Phe Gln Leu Cys
   670 675 680
   Leu His Tyr Arg Asp Phe Ile Pro Gly Val Ala Ile Ala Ala Asn Ile
   685 690 695
   Ile His Glu Gly Phe His Lys Ser Arg Lys Val Ile Val Val Val Ser
   700 705 710
   Gln His Phe Ile Gln Ser Arg Trp Cys Ile Phe Glu Tyr Glu Ile Ala
   715 720 725 730
   Gln Thr Trp Gln Phe Leu Ser Ser Arg Ala Gly Ile Ile Phe Ile Val
   735 740 745
   Leu Gln Lys Val Glu Lys Thr Leu Leu Arg Gln Gln Val Glu Leu Tyr
   750 755 760
   Arg Leu Leu Ser Arg Asn Thr Tyr Leu Glu Trp Glu Asp Ser Val Leu
   765 770 775
   Gly Arg His Ile Phe Trp Arg Arg Leu Arg Lys Ala Leu Leu Asp Gly
   780 785 790
   Lys Ser Trp Asn Pro Glu Gly Thr Val Gly Thr Gly Cys Asn Trp Gln
   795 800 805 810
   Glu Ala Thr Ser Ile
   815
(2) INFORMATION FOR SEQ ID NO:27:
   (i) SEQUENCE CHARACTERISTICS:
      (A) LENGTH: 300 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..300
   (ix) FEATURE:
      (A) NAME/KEY: misc_feature
      (B) LOCATION: 186
      (D) OTHER INFORMATION: /note= "nucleotides 186, 196, 217, 276, and 300 designated C, each may be A, C, G, or T"
   (xi) SEQUENCE DESCRIPTION: SEQ ID NO:27:
   TCC TAT TCT ATG GAA AAA GAT GCT TTC CTA TTT ATG AGA AAT TTG AAG        48
   Ser Tyr Ser Met Glu Lys Asp Ala Phe Leu Phe Met Arg Asn Leu Lys
     1               5                  10                  15
   GTT CTC TCA CTA AAA GAT AAC AAT GTC ACA GCT GTC CCC ACC ACT TTG        96
   Val Leu Ser Leu Lys Asp Asn Asn Val Thr Ala Val Pro Thr Thr Leu
                20                  25                  30
   CCA CCT AAT TTA CTA GAG CTC TAT CTT TAT AAC AAT ATC ATT AAG AAA       144
   Pro Pro Asn Leu Leu Glu Leu Tyr Leu Tyr Asn Asn Ile Ile Lys Lys
            35                  40                  45
   ATC CAA GAA AAT GAT TTC AAT AAC CTC AAT GAG TTG CAA GTC CTT GAC       192
   Ile Gln Glu Asn Asp Phe Asn Asn Leu Asn Glu Leu Gln Val Leu Asp
        50                  55                  60
   CTA CGT GGA AAT TGC CCT CGA TGT CAT AAT GTC CCA TAT CCG TGT ACA       240
   Leu Arg Gly Asn Cys Pro Arg Cys His Asn Val Pro Tyr Pro Cys Thr
    65                  70                  75                  80
   CCG TGT GAA AAT AAT TCC CCC TTA CAG ATC CAT GAC AAT GCT TTC AAT       288
   Pro Cys Glu Asn Asn Ser Pro Leu Gln Ile His Asp Asn Ala Phe Asn
                    85                  90                  95
   TCA TCG ACA GAC                                                       300
   Ser Ser Thr Asp
               100
(2) INFORMATION FOR SEQ ID NO:28:
   (i) SEQUENCE CHARACTERISTICS:
      (A) LENGTH: 100 amino acids
      (B) TYPE: amino acid
      (D) TOPOLOGY: linear
   (ii) MOLECULE TYPE: protein
   (xi) SEQUENCE DESCRIPTION: SEQ ID NO:28:
   Ser Tyr Ser Met Glu Lys Asp Ala Phe Leu Phe Met Arg Asn Leu Lys
    1               5                  10                  15
   Val Leu Ser Leu Lys Asp Asn Asn Val Thr Ala Val Pro Thr Thr Leu
               20                  25                  30
   Pro Pro Asn Leu Leu Glu Leu Tyr Leu Tyr Asn Asn Ile Ile Lys Lys
           35                  40                  45
   Ile Gln Glu Asn Asp Phe Asn Asn Leu Asn Glu Leu Gln Val Leu Asp
       50                  55                  60
   Leu Arg Gly Asn Cys Pro Arg Cys His Asn Val Pro Tyr Pro Cys Thr
   65                  70                  75                  80
   Pro Cys Glu Asn Asn Ser Pro Leu Gln Ile His Asp Asn Ala Phe Asn
                   85                  90                  95
   Ser Ser Thr Asp
               100
(2) INFORMATION FOR SEQ ID NO:29:
   (i) SEQUENCE CHARACTERISTICS:
      (A) LENGTH: 1756 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1182
   (ix) FEATURE:
      (A) NAME/KEY: misc_feature
      (B) LOCATION: 1643
      (D) OTHER INFORMATION: /note= "nucleotide 1643 designated A, may be A or G"
   (ix) FEATURE:
      (A) NAME/KEY: misc_feature
      (B) LOCATION: 1664
      (D) OTHER INFORMATION: /note= "nucleotide 1664 designated C, may be A, C, G, or T"
   (ix) FEATURE:
      (A) NAME/KEY: misc_feature
      (B) LOCATION: 1680
      (D) OTHER INFORMATION: /note= "nucleotides 1680 and 1735 designated G, may be G or T"
   (ix) FEATURE:
      (A) NAME/KEY: misc_feature
      (B) LOCATION: 1719
      (D) OTHER INFORMATION: /note= "nucleotide 1719 designated C, may be C or T"
   (ix) FEATURE:
      (A) NAME/KEY: misc_feature
      (B) LOCATION: 1727
      (D) OTHER INFORMATION: /note= "nucleotide 1727 designated A, may be A, G, or T"
   (xi) SEQUENCE DESCRIPTION: SEQ ID NO:29:
   TCT CCA GAA ATT CCC TGG AAT TCC TTG CCT CCT GAG GTT TTT GAG GGT     48
   Ser Pro Glu Ile Pro Trp Asn Ser Leu Pro Pro Glu Val Phe Glu Gly
    1               5                  10                  15
   ATG CCG CCA AAT CTA AAG AAT CTC TCC TTG GCC AAA AAT GGG CTC AAA     96
   Met Pro Pro Asn Leu Lys Asn Leu Ser Leu Ala Lys Asn Gly Leu Lys
               20                  25                  30
   TCT TTC TTT TGG GAC AGA CTC CAG TTA CTG AAG CAT TTG GAA ATT TTG    144
   Ser Phe Phe Trp Asp Arg Leu Gln Leu Leu Lys His Leu Glu Ile Leu
           35                  40                  45
   GAC CTC AGC CAT AAC CAG CTG ACA AAA GTA CCT GAG AGA TTG GCC AAC    192
   Asp Leu Ser His Asn Gln Leu Thr Lys Val Pro Glu Arg Leu Ala Asn
       50                  55                  60
   TGT TCC AAA AGT CTC ACA ACA CTG ATT CTT AAG CAT AAT CAA ATC AGG    240
   Cys Ser Lys Ser Leu Thr Thr Leu Ile Leu Lys His Asn Gln Ile Arg
   65                  70                  75                  80
   CAA TTG ACA AAA TAT TTT CTA GAA GAT GCT TTG CAA TTG CGC TAT CTA    288
   Gln Leu Thr Lys Tyr Phe Leu Glu Asp Ala Leu Gln Leu Arg Tyr Leu
                   85                  90                  95
   GAC ATC AGT TCA AAT AAA ATC CAG GTC ATT CAG AAG ACT AGC TTC CCA    336
   Asp Ile Ser Ser Asn Lys Ile Gln Val Ile Gln Lys Thr Ser Phe Pro
               100                 105                 110
   GAA AAT GTC CTC AAC AAT CTG GAG ATG TTG GTT TTA CAT CAC AAT CGC    384
   Glu Asn Val Leu Asn Asn Leu Glu Met Leu Val Leu His His Asn Arg
           115                 120                 125
   TTT CTT TGC AAC TGT GAT GCT GTG TGG TTT GTC TGG TGG GTT AAC CAT    432
   Phe Leu Cys Asn Cys Asp Ala Val Trp Phe Val Trp Trp Val Asn His
       130                 135                 140
   ACA GAT GTT ACT ATT CCA TAC CTG GCC ACT GAT GTG ACT TGT GTA GGT    480
   Thr Asp Val Thr Ile Pro Tyr Leu Ala Thr Asp Val Thr Cys Val Gly
   145                 150                 155                 160
   CCA GGA GCA CAC AAA GGT CAA AGT GTC ATA TCC CTT GAT CTG TAT ACG    528
   Pro Gly Ala His Lys Gly Gln Ser Val Ile Ser Leu Asp Leu Tyr Thr
                   165                 170                 175
   TGT GAG TTA GAT CTC ACA AAC CTG ATT CTG TTC TCA GTT TCC ATA TCA    576
   Cys Glu Leu Asp Leu Thr Asn Leu Ile Leu Phe Ser Val Ser Ile Ser
               180                 185                 190
   TCA GTC CTC TTT CTT ATG GTA GTT ATG ACA ACA AGT CAC CTC TTT TTC    624
   Ser Val Leu Phe Leu Met Val Val Met Thr Thr Ser His Leu Phe Phe
           195                 200                 205
   TGG GAT ATG TGG TAC ATT TAT TAT TTT TGG AAA GCA AAG ATA AAG GGG    672
   Trp Asp Met Trp Tyr Ile Tyr Tyr Phe Trp Lys Ala Lys Ile Lys Gly
       210                 215                 220
   TAT CCA GCA TCT GCA ATC CCA TGG AGT CCT TGT TAT GAT GCT TTT ATT    720
   Tyr Pro Ala Ser Ala Ile Pro Trp Ser Pro Cys Tyr Asp Ala Phe Ile
   225                 230                 235                 240
   GTG TAT GAC ACT AAA AAC TCA GCT GTG ACA GAA TGG GTT TTG CAG GAG      768
   Val Tyr Asp Thr Lys Asn Ser Ala Val Thr Glu Trp Val Leu Gln Glu
                   245                 250                 255
   CTG GTG GCA AAA TTG GAA GAT CCA AGA GAA AAA CAC TTC AAT TTG TGT      816
   Leu Val Ala Lys Leu Glu Asp Pro Arg Glu Lys His Phe Asn Leu Cys
               260                 265                 270
   CTA GAA GAA AGA GAC TGG CTA CCA GGA CAG CCA GTT CTA GAA AAC CTT      864
   Leu Glu Glu Arg Asp Trp Leu Pro Gly Gln Pro Val Leu Glu Asn Leu
           275                 280                 285
   TCC CAG AGC ATA CAG CTC AGC AAA AAG ACA GTG TTT GTG ATG ACA CAG      912
   Ser Gln Ser Ile Gln Leu Ser Lys Lys Thr Val Phe Val Met Thr Gln
       290                 295                 300
   AAA TAT GCT AAG ACT GAG AGT TTT AAG ATG GCA TTT TAT TTG TCT CAT      960
   Lys Tyr Ala Lys Thr Glu Ser Phe Lys Met Ala Phe Tyr Leu Ser His
   305                 310                 315                 320
   CAG AGG CTC CTG GAT GAA AAA GTG GAT GTG ATT ATC TTG ATA TTC TTG     1008
   Gln Arg Leu Leu Asp Glu Lys Val Asp Val Ile Ile Leu Ile Phe Leu
                   325                 330                 335
   GAA AGA CCT CTT CAG AAG TCT AAG TTT CTT CAG CTC AGG AAG AGA CTC     1056
   Glu Arg Pro Leu Gln Lys Ser Lys Phe Leu Gln Leu Arg Lys Arg Leu
               340                 345                 350
   TGC AGG AGC TCT GTC CTT GAG TGG CCT GCA AAT CCA CAG GCT CAC CCA     1104
   Cys Arg Ser Ser Val Leu Glu Trp Pro Ala Asn Pro Gln Ala His Pro
           355                 360                 365
   TAC TTC TGG CAG TGC CTG AAA AAT GCC CTG ACC ACA GAC AAT CAT GTG     1152
   Tyr Phe Trp Gln Cys Leu Lys Asn Ala Leu Thr Thr Asp Asn His Val
       370                 375                 380
   GCT TAT AGT CAA ATG TTC AAG GAA ACA GTC TAGCTCTCTG AAGAATGTCA       1202
   Ala Tyr Ser Gln Met Phe Lys Glu Thr Val
   385                 390
   CCACCTAGGA CATGCCTTGG TACCTGAAGT TTTCATAAAG GTTTCCATAA ATGAAGGTCT   1262
   GAATTTTTCC TAACAGTTGT CATGGCTCAG ATTGGTGGGA AATCATCAAT ATATGGCTAA   1322
   GAAATTAAGA AGGGGAGACT GATAGAAGAT AATTTCTTTC TTCATGTGCC ATGCTCAGTT   1382
   AAATATTTCC CCTAGCTCAA ATCTGAAAAA CTGTGCCTAG GAGACAACAC AAGGCTTTGA   1442
   TTTATCTGCA TACAATTGAT AAGAGCCACA CATCTGCCCT GAAGAAGTAC TAGTAGTTTT   1502
   AGTAGTAGGG TAAAAATTAC ACAAGCTTTC TCTCTCTCTG ATACTGAACT GTACCAGAGT   1562
   TCAATGAAAT AAAAGCCCAG AGAACTTCTC AGTAAATGGT TTCATTATCA TGTAGTATCC   1622
   ACCATGCAAT ATGCCACAAA ACCGCTACTG GTACAGGACA GCTGGTAGCT GCTTCAAGGC   1682
   CTCTTATCAT TTTCTTGGGG CCCATGGAGG GGTTCTCTGG GAAAAAGGGA AGGTTTTTTT   1742
   TGGCCATCCA TGAA                                                     1756
(2) INFORMATION FOR SEQ ID NO:30:
   (i) SEQUENCE CHARACTERISTICS:
      (A) LENGTH: 394 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Ser Pro Glu Ile Pro Trp Asn Ser Leu Pro Pro Glu Val Phe Glu Gly
   1 5 10 15
   Met Pro Pro Asn Leu Lys Asn Leu Ser Leu Ala Lys Asn Gly Leu Lys
   20 25 30
   Ser Phe Phe Trp Asp Arg Leu Gln Leu Leu Lys His Leu Glu Ile Leu
   35 40 45
   Asp Leu Ser His Asn Gln Leu Thr Lys Val Pro Glu Arg Leu Ala Asn
   50 55 60
   Cys Ser Lys Ser Leu Thr Thr Leu Ile Leu Lys His Asn Gln Ile Arg
   65 70 75 80
   Gln Leu Thr Lys Tyr Phe Leu Glu Asp Ala Leu Gln Leu Arg Tyr Leu
   85 90 95
   Asp Ile Ser Ser Asn Lys Ile Gln Val Ile Gln Lys Thr Ser Phe Pro
   100 105 110
   Glu Asn Val Leu Asn Asn Leu Glu Met Leu Val Leu His His Asn Arg
   115 120 125
   Phe Leu Cys Asn Cys Asp Ala Val Trp Phe Val Trp Trp Val Asn His
   130 135 140
   Thr Asp Val Thr Ile Pro Tyr Leu Ala Thr Asp Val Thr Cys Val Gly
   145 150 155 160
   Pro Gly Ala His Lys Gly Gln Ser Val Ile Ser Leu Asp Leu Tyr Thr
   165 170 175
   Cys Glu Leu Asp Leu Thr Asn Leu Ile Leu Phe Ser Val Ser Ile Ser
   180 185 190
   Ser Val Leu Phe Leu Met Val Val Met Thr Thr Ser His Leu Phe Phe
   195 200 205
   Trp Asp Met Trp Tyr Ile Tyr Tyr Phe Trp Lys Ala Lys Ile Lys Gly
   210 215 220
   Tyr Pro Ala Ser Ala Ile Pro Trp Ser Pro Cys Tyr Asp Ala Phe Ile
   225 230 235 240
   Val Tyr Asp Thr Lys Asn Ser Ala Val Thr Glu Trp Val Leu Gln Glu
   245 250 255
   Leu Val Ala Lys Leu Glu Asp Pro Arg Glu Lys His Phe Asn Leu Cys
                   260                 265                 270
   Leu Glu Glu Arg Asp Trp Leu Pro Gly Gln Pro Val Leu Glu Asn Leu
           275                 280                 285
   Ser Gln Ser Ile Gln Leu Ser Lys Lys Thr Val Phe Val Met Thr Gln
       290                 295                 300
   Lys Tyr Ala Lys Thr Glu Ser Phe Lys Met Ala Phe Tyr Leu Ser His
   305                 310                 315                 320
   Gln Arg Leu Leu Asp Glu Lys Val Asp Val Ile Ile Leu Ile Phe Leu
                   325                 330                 335
   Glu Arg Pro Leu Gln Lys Ser Lys Phe Leu Gln Leu Arg Lys Arg Leu
               340                 345                 350
   Cys Arg Ser Ser Val Leu Glu Trp Pro Ala Asn Pro Gln Ala His Pro
           355                 360                 365
   Tyr Phe Trp Gln Cys Leu Lys Asn Ala Leu Thr Thr Asp Asn His Val
       370                 375                 380
   Ala Tyr Ser Gln Met Phe Lys Glu Thr Val
   385                 390
(2) INFORMATION FOR SEQ ID NO:31:
   (i) SEQUENCE CHARACTERISTICS:
      (A) LENGTH: 999 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 2..847
   (ix) FEATURE:
      (A) NAME/KEY: misc_feature
      (B) LOCATION: 4
      (D) OTHER INFORMATION: /note= "nucleotides 4 and 23 designated C, each may be A, C, G, or T"
   (ix) FEATURE:
      (A) NAME/KEY: misc_feature
      (B) LOCATION: 650
      (D) OTHER INFORMATION: /note= "nucleotide 650 designated G, may be A or G"
   (ix) FEATURE:
      (A) NAME/KEY: misc_feature
      (B) LOCATION: 715
      (D) OTHER INFORMATION: /note= "nucleotides 715, 825, and 845 designated C, each may be C or T"
   (xi) SEQUENCE DESCRIPTION: SEQ ID NO:31:
   C TCC GAT GCC AAG ATT CGG CAC CAG GCA TAT TCA GAG GTC ATG ATG          46
     Ser Asp Ala Lys Ile Arg His Gln Ala Tyr Ser Glu Val Met Met
       1               5                  10                  15
   GTT GGA TGG TCA GAT TCA TAC ACC TGT GAA TAC CCT TTA AAC CTA AGG        94
   Val Gly Trp Ser Asp Ser Tyr Thr Cys Glu Tyr Pro Leu Asn Leu Arg
                   20                  25                  30
   GGA ACT AGG TTA AAA GAC GTT CAT CTC CAC GAA TTA TCT TGC AAC ACA       142
   Gly Thr Arg Leu Lys Asp Val His Leu His Glu Leu Ser Cys Asn Thr
               35                  40                  45
   GCT CTG TTG ATT GTC ACC ATT GTG GTT ATT ATG CTA GTT CTG GGG TTG       190
   Ala Leu Leu Ile Val Thr Ile Val Val Ile Met Leu Val Leu Gly Leu
           50                  55                  60
   GCT GTG GCC TTC TGC TGT CTC CAC TTT GAT CTG CCC TGG TAT CTC AGG       238
   Ala Val Ala Phe Cys Cys Leu His Phe Asp Leu Pro Trp Tyr Leu Arg
       65                  70                  75
   ATG CTA GGT CAA TGC ACA CAA ACA TGG CAC AGG GTT AGG AAA ACA ACC       286
   Met Leu Gly Gln Cys Thr Gln Thr Trp His Arg Val Arg Lys Thr Thr
   80                  85                  90                  95
   CAA GAA CAA CTC AAG AGA AAT GTC CGA TTC CAC GCA TTT ATT TCA TAC       334
   Gln Glu Gln Leu Lys Arg Asn Val Arg Phe His Ala Phe Ile Ser Tyr
                   100                 105                 110
   AGT GAA CAT GAT TCT CTG TGG GTG AAG AAT GAA TTG ATC CCC AAT CTA       382
   Ser Glu His Asp Ser Leu Trp Val Lys Asn Glu Leu Ile Pro Asn Leu
               115                 120                 125
   GAG AAG GAA GAT GGT TCT ATC TTG ATT TGC CTT TAT GAA AGC TAC TTT       430
   Glu Lys Glu Asp Gly Ser Ile Leu Ile Cys Leu Tyr Glu Ser Tyr Phe
           130                 135                 140
   GAC CCT GGC AAA AGC ATT AGT GAA AAT ATT GTA AGC TTC ATT GAG AAA       478
   Asp Pro Gly Lys Ser Ile Ser Glu Asn Ile Val Ser Phe Ile Glu Lys
       145                 150                 155
   AGC TAT AAG TCC ATC TTT GTT TTG TCT CCC AAC TTT GTC CAG AAT GAG       526
   Ser Tyr Lys Ser Ile Phe Val Leu Ser Pro Asn Phe Val Gln Asn Glu
   160                 165                 170                 175
   TGG TGC CAT TAT GAA TTC TAC TTT GCC CAC CAC AAT CTC TTC CAT GAA       574
   Trp Cys His Tyr Glu Phe Tyr Phe Ala His His Asn Leu Phe His Glu
                   180                 185                 190
   AAT TCT GAT CAC ATA ATT CTT ATC TTA CTG GAA CCC ATT CCA TTC TAT       622
   Asn Ser Asp His Ile Ile Leu Ile Leu Leu Glu Pro Ile Pro Phe Tyr
               195                 200                 205
   TGC ATT CCC ACC AGG TAT CAT AAA CTG GAA GCT CTC CTG GAA AAA AAA       670
   Cys Ile Pro Thr Arg Tyr His Lys Leu Glu Ala Leu Leu Glu Lys Lys
           210                 215                 220
   GCA TAC TTG GAA TGG CCC AAG GAT AGG CGT AAA TGT GGG CTT TTC TGG       718
   Ala Tyr Leu Glu Trp Pro Lys Asp Arg Arg Lys Cys Gly Leu Phe Trp
       225                 230                 235
   GCA AAC CTT CGA GCT GCT GTT AAT GTT AAT GTA TTA GCC ACC AGA GAA        766
   Ala Asn Leu Arg Ala Ala Val Asn Val Asn Val Leu Ala Thr Arg Glu
   240                 245                 250                 255
   ATG TAT GAA CTG CAG ACA TTC ACA GAG TTA AAT GAA GAG TCT CGA GGT        814
   Met Tyr Glu Leu Gln Thr Phe Thr Glu Leu Asn Glu Glu Ser Arg Gly
                   260                 265                 270
   TCT ACA ATC TCT CTG ATG AGA ACA GAC TGT CTA TAAAATCCCA CAGTCCTTGG      867
   Ser Thr Ile Ser Leu Met Arg Thr Asp Cys Leu
               275                 280
   GAAGTTGGGG ACCACATACA CTGTTGGGAT GTACATTGAT ACAACCTTTA TGATGGCAAT      927
   TTGACAATAT TTATTAAAAT AAAAAATGGT TATTCCCTTC AAAAAAAAAA AAAAAAAAAA      987
   AAAAAAAAAA AA                                                          999
(2) INFORMATION FOR SEQ ID NO:32:
   (i) SEQUENCE CHARACTERISTICS:
      (A) LENGTH: 282 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Ser Asp Ala Lys Ile Arg His Gln Ala Tyr Ser Glu Val Met Met Val
   1 5 10 15
   Gly Trp Ser Asp Ser Tyr Thr Cys Glu Tyr Pro Leu Asn Leu Arg Gly
   20 25 30
   Thr Arg Leu Lys Asp Val His Leu His Glu Leu Ser Cys Asn Thr Ala
   35 40 45
   Leu Leu Ile Val Thr Ile Val Val Ile Met Leu Val Leu Gly Leu Ala
   50 55 60
   Val Ala Phe Cys Cys Leu His Phe Asp Leu Pro Trp Tyr Leu Arg Met
   65 70 75 80
   Leu Gly Gln Cys Thr Gln Thr Trp His Arg Val Arg Lys Thr Thr Gln
   85 90 95
   Glu Gln Leu Lys Arg Asn Val Arg Phe His Ala Phe Ile Ser Tyr Ser
   100 105 110
   Glu His Asp Ser Leu Trp Val Lys Asn Glu Leu Ile Pro Asn Leu Glu
   115 120 125
   Lys Glu Asp Gly Ser Ile Leu Ile Cys Leu Tyr Glu Ser Tyr Phe Asp
   130 135 140
   Pro Gly Lys Ser Ile Ser Glu Asn Ile Val Ser Phe Ile Glu Lys Ser
   145 150 155 160
   Tyr Lys Ser Ile Phe Val Leu Ser Pro Asn Phe Val Gln Asn Glu Trp
   165 170 175
   Cys His Tyr Glu Phe Tyr Phe Ala His His Asn Leu Phe His Glu Asn
   180 185 190
   Ser Asp His Ile Ile Leu Ile Leu Leu Glu Pro Ile Pro Phe Tyr Cys
   195 200 205
   Ile Pro Thr Arg Tyr His Lys Leu Glu Ala Leu Leu Glu Lys Lys Ala
   210 215 220
   Tyr Leu Glu Trp Pro Lys Asp Arg Arg Lys Cys Gly Leu Phe Trp Ala
   225 230 235 240
   Asn Leu Arg Ala Ala Val Asn Val Asn Val Leu Ala Thr Arg Glu Met
   245 250 255
   Tyr Glu Leu Gln Thr Phe Thr Glu Leu Asn Glu Glu Ser Arg Gly Ser
   260 265 270
   Thr Ile Ser Leu Met Arg Thr Asp Cys Leu
   275 280
(2) INFORMATION FOR SEQ ID NO:33:
   (i) SEQUENCE CHARACTERISTICS:
      (A) LENGTH: 1173 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1008
   (ix) FEATURE:
      (A) NAME/KEY: misc_feature
      (B) LOCATION: 854
      (D) OTHER INFORMATION: /note= "nucleotide 854 designated A, may be A or T"
   (ix) FEATURE:
      (A) NAME/KEY: misc_feature
      (B) LOCATION: 1171
      (D) OTHER INFORMATION: /note= "nucleotides 1171 and 1172 designated C, each may be A, C, G, or T"
   (xi) SEQUENCE DESCRIPTION: SEQ ID NO:33:
   CTG CCT GCT GGC ACC CGG CTC CGG AGG CTG GAT GTC AGC TGC AAC AGC        48
   Leu Pro Ala Gly Thr Arg Leu Arg Arg Leu Asp Val Ser Cys Asn Ser
     1               5                  10                  15
   ATC AGC TTC GTG GCC CCC GGC TTC TTT TCC AAG GCC AAG GAG CTG CGA        96
   Ile Ser Phe Val Ala Pro Gly Phe Phe Ser Lys Ala Lys Glu Leu Arg
                20                  25                  30
   GAG CTC AAC CTT AGC GCC AAC GCC CTC AAG ACA GTG GAC CAC TCC TGG      144
   Glu Leu Asn Leu Ser Ala Asn Ala Leu Lys Thr Val Asp His Ser Trp
            35                  40                  45
   TTT GGG CCC CTG GCG AGT GCC CTG CAA ATA CTA GAT GTA AGC GCC AAC      192
   Phe Gly Pro Leu Ala Ser Ala Leu Gln Ile Leu Asp Val Ser Ala Asn
        50                  55                  60
   CCT CTG CAC TGC GCC TGT GGG GCG GCC TTT ATG GAC TTC CTG CTG GAG      240
   Pro Leu His Cys Ala Cys Gly Ala Ala Phe Met Asp Phe Leu Leu Glu
    65                  70                  75                  80
   GTG CAG GCT GCC GTG CCC GGT CTG CCC AGC CGG GTG AAG TGT GGC AGT      288
   Val Gln Ala Ala Val Pro Gly Leu Pro Ser Arg Val Lys Cys Gly Ser
                    85                  90                  95
   CCG GGC CAG CTC CAG GGC CTC AGC ATC TTT GCA CAG GAC CTG CGC CTC      336
   Pro Gly Gln Leu Gln Gly Leu Ser Ile Phe Ala Gln Asp Leu Arg Leu
               100                 105                 110
   TGC CTG GAT GAG GCC CTC TCC TGG GAC TGT TTC GCC CTC TCG CTG CTG      384
   Cys Leu Asp Glu Ala Leu Ser Trp Asp Cys Phe Ala Leu Ser Leu Leu
           115                 120                 125
   GCT GTG GCT CTG GGC CTG GGT GTG CCC ATG CTG CAT CAC CTC TGT GGC      432
   Ala Val Ala Leu Gly Leu Gly Val Pro Met Leu His His Leu Cys Gly
       130                 135                 140
   TGG GAC CTC TGG TAC TGC TTC CAC CTG TGC CTG GCC TGG CTT CCC TGG      480
   Trp Asp Leu Trp Tyr Cys Phe His Leu Cys Leu Ala Trp Leu Pro Trp
   145                 150                 155                 160
   CGG GGG CGG CAA AGT GGG CGA GAT GAG GAT GCC CTG CCC TAC GAT GCC      528
   Arg Gly Arg Gln Ser Gly Arg Asp Glu Asp Ala Leu Pro Tyr Asp Ala
                   165                 170                 175
   TTC GTG GTC TTC GAC AAA ACG CAG AGC GCA GTG GCA GAC TGG GTG TAC      576
   Phe Val Val Phe Asp Lys Thr Gln Ser Ala Val Ala Asp Trp Val Tyr
               180                 185                 190
   AAC GAG CTT CGG GGG CAG CTG GAG GAG TGC CGT GGG CGC TGG GCA CTC      624
   Asn Glu Leu Arg Gly Gln Leu Glu Glu Cys Arg Gly Arg Trp Ala Leu
           195                 200                 205
   CGC CTG TGC CTG GAG GAA CGC GAC TGG CTG CCT GGC AAA ACC CTC TTT      672
   Arg Leu Cys Leu Glu Glu Arg Asp Trp Leu Pro Gly Lys Thr Leu Phe
       210                 215                 220
   GAG AAC CTG TGG GCC TCG GTC TAT GGC AGC CGC AAG ACG CTG TTT GTG      720
   Glu Asn Leu Trp Ala Ser Val Tyr Gly Ser Arg Lys Thr Leu Phe Val
   225                 230                 235                 240
   CTG GCC CAC ACG GAC CGG GTC AGT GGT CTC TTG CGC GCC AGC TTC CTG      768
   Leu Ala His Thr Asp Arg Val Ser Gly Leu Leu Arg Ala Ser Phe Leu
                   245                 250                 255
   CTG GCC CAG CAG CGC CTG CTG GAG GAC CGC AAG GAC GTC GTG GTG CTG      816
   Leu Ala Gln Gln Arg Leu Leu Glu Asp Arg Lys Asp Val Val Val Leu
               260                 265                 270
   GTG ATC CTG AGC CCT GAC GGC CGC CGC TCC CGC TAC GAG CGG CTG CGC      864
   Val Ile Leu Ser Pro Asp Gly Arg Arg Ser Arg Tyr Glu Arg Leu Arg
           275                 280                 285
   CAG CGC CTC TGC CGC CAG AGT GTC CTC CTC TGG CCC CAC CAG CCC AGT      912
   Gln Arg Leu Cys Arg Gln Ser Val Leu Leu Trp Pro His Gln Pro Ser
       290                 295                 300
   GGT CAG CGC AGC TTC TGG GCC CAG CTG GGC ATG GCC CTG ACC AGG GAC      960
   Gly Gln Arg Ser Phe Trp Ala Gln Leu Gly Met Ala Leu Thr Arg Asp
   305                 310                 315                 320
   AAC CAC CAC TTC TAT AAC CGG AAC TTC TGC CAG GGA CCC ACG GCC GAA     1008
   Asn His His Phe Tyr Asn Arg Asn Phe Cys Gln Gly Pro Thr Ala Glu
                   325                 330                 335
   TAGCCGTGAG CCGGAATCCT GCACGGTGCC ACCTCCACAC TCACCTCACC TCTGCCTGCC   1068
   TGGTCTGACC CTCCCCTGCT CGCCTCCCTC ACCCCACACC TGACACAGAG CAGGCACTCA   1128
   ATAAATGCTA CCGAAGGCTA AAAAAAAAAA AAAAAAAAAA AACCA                   1173
(2) INFORMATION FOR SEQ ID NO:34:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:34:
   Leu Pro Ala Gly Thr Arg Leu Arg Arg Leu Asp Val Ser Cys Asn Ser
    1               5                  10                  15
   Ile Ser Phe Val Ala Pro Gly Phe Phe Ser Lys Ala Lys Glu Leu Arg
               20                  25                  30
   Glu Leu Asn Leu Ser Ala Asn Ala Leu Lys Thr Val Asp His Ser Trp
           35                  40                  45
   Phe Gly Pro Leu Ala Ser Ala Leu Gln Ile Leu Asp Val Ser Ala Asn
       50                  55                  60
   Pro Leu His Cys Ala Cys Gly Ala Ala Phe Met Asp Phe Leu Leu Glu
   65                  70                  75                  80
   Val Gln Ala Ala Val Pro Gly Leu Pro Ser Arg Val Lys Cys Gly Ser
                   85                  90                  95
   Pro Gly Gln Leu Gln Gly Leu Ser Ile Phe Ala Gln Asp Leu Arg Leu
               100                 105                 110
   Cys Leu Asp Glu Ala Leu Ser Trp Asp Cys Phe Ala Leu Ser Leu Leu
           115                 120                 125
   Ala Val Ala Leu Gly Leu Gly Val Pro Met Leu His His Leu Cys Gly
       130                 135                 140
   Trp Asp Leu Trp Tyr Cys Phe His Leu Cys Leu Ala Trp Leu Pro Trp
   145                 150                 155                 160
   Arg Gly Arg Gln Ser Gly Arg Asp Glu Asp Ala Leu Pro Tyr Asp Ala
                   165                 170                 175
   Phe Val Val Phe Asp Lys Thr Gln Ser Ala Val Ala Asp Trp Val Tyr
               180                 185                 190
   Asn Glu Leu Arg Gly Gln Leu Glu Glu Cys Arg Gly Arg Trp Ala Leu
           195                 200                 205
   Arg Leu Cys Leu Glu Glu Arg Asp Trp Leu Pro Gly Lys Thr Leu Phe
       210                 215                 220
   Glu Asn Leu Trp Ala Ser Val Tyr Gly Ser Arg Lys Thr Leu Phe Val
   225                 230                 235                 240
   Leu Ala His Thr Asp Arg Val Ser Gly Leu Leu Arg Ala Ser Phe Leu
                   245                 250                 255
   Leu Ala Gln Gln Arg Leu Leu Glu Asp Arg Lys Asp Val Val Val Leu
               260                 265                 270
   Val Ile Leu Ser Pro Asp Gly Arg Arg Ser Arg Tyr Glu Arg Leu Arg
           275                 280                 285
   Gln Arg Leu Cys Arg Gln Ser Val Leu Leu Trp Pro His Gln Pro Ser
       290                 295                 300
   Gly Gln Arg Ser Phe Trp Ala Gln Leu Gly Met Ala Leu Thr Arg Asp
   305                 310                 315                 320
   Asn His His Phe Tyr Asn Arg Asn Phe Cys Gln Gly Pro Thr Ala Glu
                   325                 330                 335
(2) INFORMATION FOR SEQ ID NO:35:
   (i) SEQUENCE CHARACTERISTICS:
      (A) LENGTH: 497 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:35:
   TGGCCCACAC GGACCGCGTC AGTGGCCTCC TGCGCACCAG CTTCCTGCTG GCTCAGCAGC     60
   GCCTGTTGGA AGACCGCAAG GACGTGGTGG TGTTGGTGAT CCTGCGTCCG GATGCCCCAC    120
   CGTCCCGCTA TGTGCGACTG CGCCAGCGTC TCTGCCGCCA GAGTGTGCTC TTCTGGCCCC    180
   AGCGACCCAA CGGGCAGGGG GGCTTCTGGG CCCAGCTGAG TACAGCCCTG ACTAGGGACA    240
   ACCGCCACTT CTATAACCAG AACTTCTGCC GGGGACCTAC AGCAGAATAG CTCAGAGCAA    300
   CAGCTGGAAA CAGCTGCATC TTCATGTCTG GTTCCCGAGT TGCTCTGCCT GCCTTGCTCT    360
   GTCTTACTAC ACCGCTATTT GGCAAGTGCG CAATATATGC TACCAAGCCA CCAGGCCCAC    420
   GGAGCAAAGG TTGGCTGTAA AGGGTAGTTT TCTTCCCATG CATCTTTCAG GAGAGTGAAG    480
   ATAGACACCA AACCCAC                                                   497

## Description

This filing claims priority from U.S. Patent Applications USSN 60/044,293, filed May 7, 1997; USSN 60/072,212, filed January 22, 1998; and USSN 60/076,947, filed March 5, 1998, each of which is incorporated herein by reference.

### FIELD OF THE INVENTION

The present invention relates to compositions and methods for affecting mammalian physiology, including morphogenesis or immune system function. In particular, it provides nucleic acids, proteins, and antibodies which regulate development and/or the immune system. Diagnostic and therapeutic uses of these materials are also disclosed.

### BACKGROUND OF THE INVENTION

Recombinant DNA technology refers generally to techniques of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host.

For some time, it has been known that the mammalian immune response is based on a series of complex cellular interactions, called the "immune network". Recent research has provided new insights into the inner workings of this network. While it remains clear that much of the immune response does, in fact, revolve around the network-like interactions of lymphocytes, macrophages, granulocytes, and other cells, immunologists now generally hold the opinion that soluble proteins, known as lymphokines, cytokines, or monokines, play critical roles in controlling these cellular interactions. Thus, there is considerable interest in the isolation, characterization, and mechanisms of action of cell modulatory factors, an understanding of which will lead to significant advancements in the diagnosis and therapy of numerous medical abnormalities, e.g., immune system disorders.

Lymphokines apparently mediate cellular activities in a variety of ways. They have been shown to support the proliferation, growth, and/or differentiation of pluripotential hematopoietic stem cells into vast numbers of progenitors comprising diverse cellular lineages which make up a complex immune system. Proper and balanced interactions between the cellular components are necessary for a healthy immune response. The different cellular lineages often respond in a different manner when lymphokines are administered in conjunction with other agents.

Cell lineages especially important to the immune response include two classes of lymphocytes: B-cells, which can produce and secrete immunoglobulins (proteins with the capability of recognizing and binding to foreign matter to effect its removal), and T-cells of various subsets that secrete lymphokines and induce or suppress the B-cells and various other cells (including other T-cells) making up the immune network. These lymphocytes interact with many other cell types.

Another important cell lineage is the mast cell (which has not been positively identified in all mammalian species), which is a granule-containing connective tissue cell located proximal to capillaries throughout the body. These cells are found in especially high concentrations in the lungs, skin, and gastrointestinal and genitourinary tracts. Mast cells play a central role in allergy-related disorders, particularly anaphylaxis as follows: when selected antigens crosslink one class of immunoglobulins bound to receptors on the mast cell surface, the mast cell degranulates and releases mediators,e.g., histamine, serotonin, heparin, and prostaglandins, which cause allergic reactions, e.g., anaphylaxis.

Research to better understand and treat various immune disorders has been hampered by the general inability to maintain cells of the immune system in vitro. Immunologists have discovered that culturing many of these cells can be accomplished through the use of T-cell and other cell supernatants, which contain various growth factors, including many of the lymphokines.

The interleukin-1 family of proteins includes the IL-1α, the IL-1β, the IL-1RA, and recently the IL-1γ (also designated Interferon-Gamma Inducing Factor, IGIF). This related family of genes have been implicated in a broad range of biological functions. See Dinarello (1994) FASEB J. 8:1314-1325; Dinarello (1991) Blood 77:1627-1652; and Okamura, et al. (1995) Nature 378:88-91.

In addition, various growth and regulatory factors exist which modulate morphogenetic development. This includes, e.g., the Toll ligands, which signal through binding to receptors which share structural, and mechanistic, features characteristic of the IL-1 receptors. See, e.g., Lemaitre, et al. (1996) Cell 86:973-983; and Belvin and Anderson (1996) Ann. Rev. Cell & Devel. Biol. 12:393-416.

From the foregoing, it is evident that the discovery and development of new soluble proteins and their receptors, including ones similar to lymphokines, should contribute to new therapies for a wide range of degenerative or abnormal conditions which directly or indirectly involve development, differentiation, or function, e.g., of the immune system and/or hematopoietic cells. In particular, the discovery and understanding of novel receptors for lymphokine-like molecules which enhance or potentiate the beneficial activities of other lymphokines would be highly advantageous. The present invention provides new receptors for ligands exhibiting similarity to interleukin-1 like compositions and related compounds, and methods for their use.

### BRIEF DESCRIPTION OF THE DRAWINGS

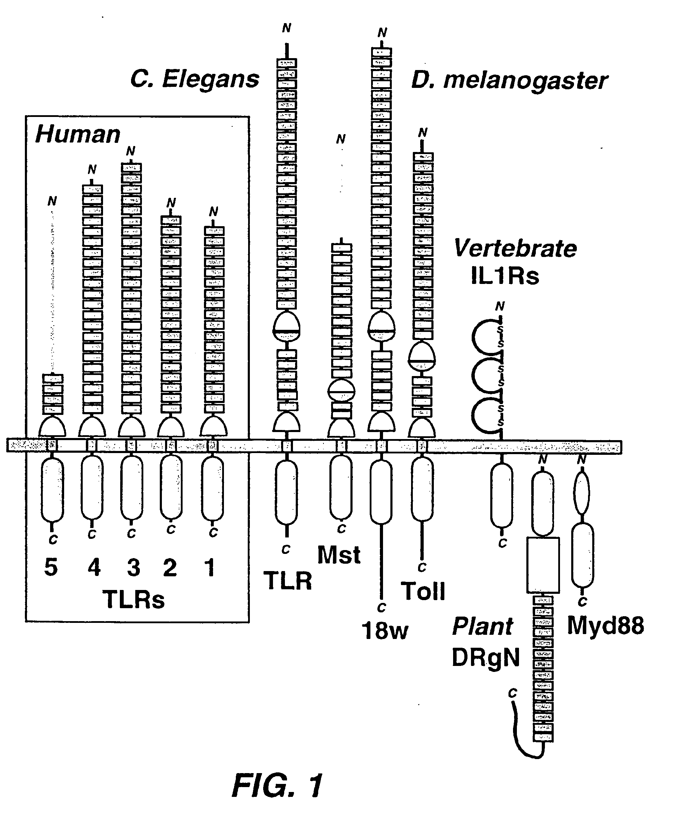

Figure 1 shows a schematic comparison of the protein architectures of Drosophila and human DTLRs, and their relationship to vertebrate IL-1 receptors and plant disease resistance proteins. Three Drosophila (Dm) DTLRs (Toll, 18w, and the Mst ORF fragment) (Morisato and Anderson (1995) Ann. Rev. Genet. 29:371-399; Chiang and Beachy (1994) Mech. Develop. 47:225-239; Mitcham, et al. (1996) J. Biol. Chem. 271:5777-5783; and Eldon, et al. (1994) Develop. 120:885-899) are arrayed beside four complete (DTLRs 1-4) and one partial (DTLR5) human (Hu) receptors. Individual LRRs in the receptor ectodomains that are flagged by PRINTS (Attwood, et al. (1997) Nucleic Acids Res. 25:212-217) are explicitely noted by boxes; 'top' and 'bottom' Cys-rich clusters that flank the C- or N-terminal ends of LRR arrays are respectively drawn by apposed half-circles. The loss of the internal Cys-rich region in DTLRs 1-5 largely accounts for their smaller ectodomains (558, 570, 690, and 652 aa, respectively) when compared to the 784 and 977 aa extensions of Toll and 18w. The incomplete chains of DmMst and HuDTLR5 (519 and 153 aa ectodomains, respectively) are represented by dashed lines. The intracellular signaling module common to DTLRs, IL-1-type receptors (IL-1Rs), the intracellular protein Myd88, and the tobacco disease resistance gene N product (DRgN) is indicated below the membrane. See, e.g., Hardiman, et al. (1996) Oncogene 13:2467-2475; and Rock, et al. (1998) Proc. Nat'l Acad. Sci. USA 95:588-. Additional domains include the trio of Ig-like modules in IL-1Rs (disulfide-linked loops); the DRgN protein features an NTPase domain (box) and Myd88 has a death domain (black oval).

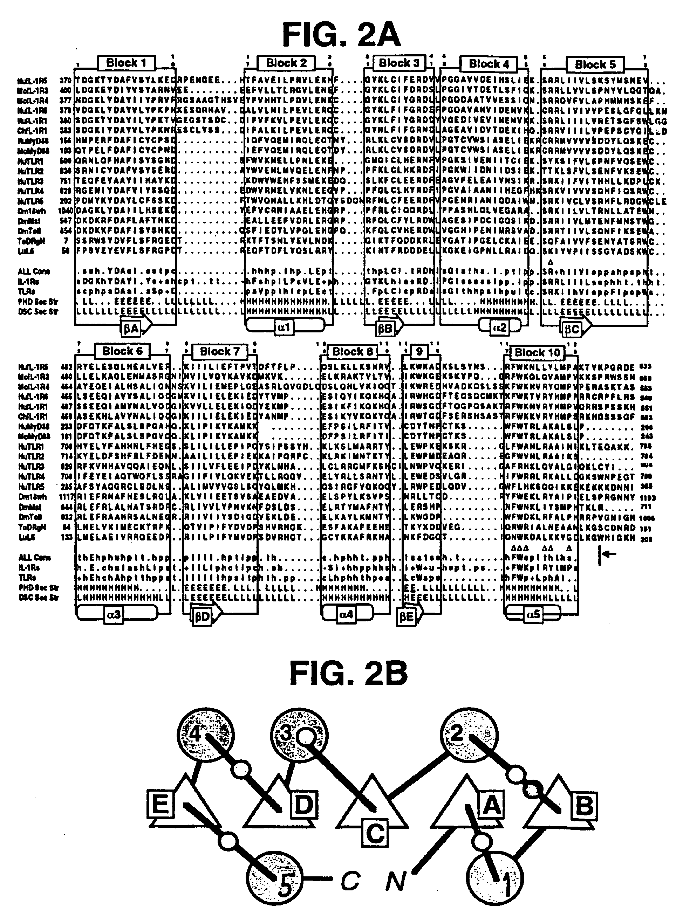

Figures 2A-2B show conserved structural patterns in the signaling domains of Toll- and IL-1-like cytokine receptors, and two divergent modular proteins. Figure 2A shows a sequence alignment of the common TH domain. DTLRs are labeled as in Figure 1; the human (Hu) or mouse (Mo) IL-1 family receptors (IL-1R1-6) are sequentially numbered as earlier proposed (Hardiman, et al. (1996) Oncogene 13:2467-2475); Myd88 and the sequences from tobacco (To) and flax, L. usitatissimum (Lu), represent C- and N-terminal domains, respectively, of larger, multidomain molecules. Ungapped blocks of sequence (numbered 1-10) are boxed. Triangles indicate deleterious mutations, while truncations N-terminal of the arrow eliminate bioactivity in human IL-1R1 (Heguy, et al. (1992) J. Biol. Chem. 267:2605-2609). PHD (Rost and Sander (1994) Proteins 19:55-72) and DSC (King and Sternberg (1996) Protein Sci. 5:2298-2310) secondary structure predictions of α-helix (H), β-strand (E), or coil (L) are marked. The amino acid shading scheme depicts chemically similar residues: hydrophobic, acidic, basic, Cys, aromatic, structure-breaking, and tiny. Diagnostic sequence patterns for IL-1Rs, DTLRs, and full alignment (ALL) were derived by Consensus at a stringency of 75%. Symbols for amino acid subsets are (see internet site for detail): o, alcohol; l, aliphatic; •, any amino acid; a, aromatic; c, charged; h, hydrophobic; -, negative; p, polar; +, positive; s, small; u, tiny; t, turnlike. Figure 2B shows a topology diagram of the proposed TH β/α domain fold. The parallel β-sheet (with β-strands A-E as yellow triangles) is seen at its C-terminal end; α-helices (circles labeled 1-5) link the β-strands; chain connections are to the front (visible) or back (hidden). Conserved, charged residues at the C-end of the β-sheet are noted in gray (Asp) or as a lone black (Arg) residue (see text).

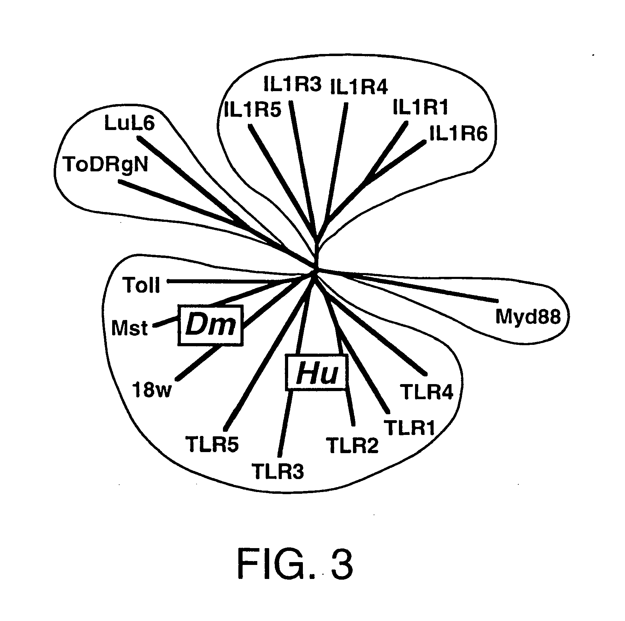

Figure 3 shows evolution of a signaling domain superfamily. The multiple TH module alignment of Figure 2A was used to derive a phylogenetic tree by the Neighbor-Joining method (Thompson, et al. (1994) Nucleic Acids Res. 22:4673-4680). Proteins labeled as in the alignment; the tree was rendered with TreeView.

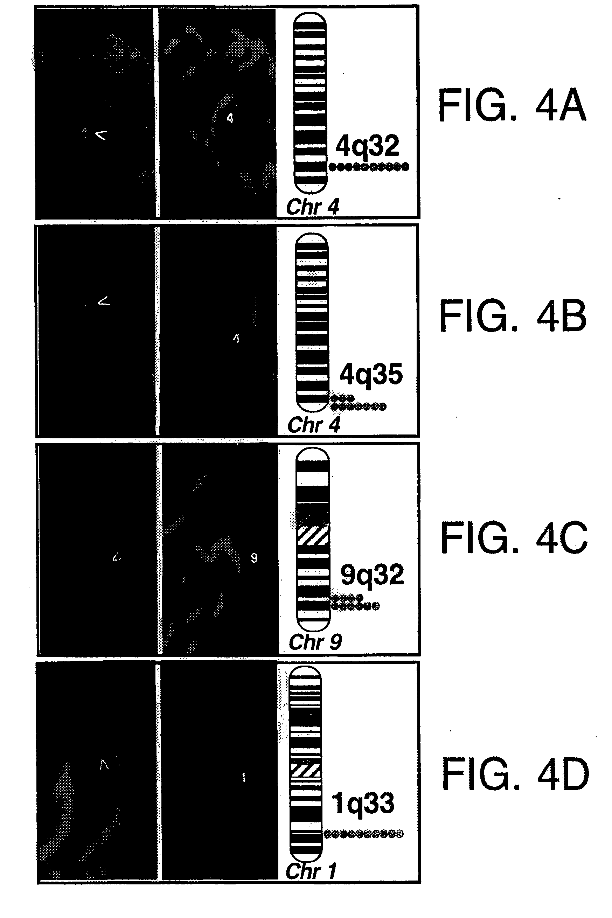

Figures 4A-4D show FISH chromosomal mapping of human DTLR genes. Denatured chromosomes from synchronous cultures of human lymphocytes were hybridized to biotinylated DTLR cDNA probes for localization. The assignment of the FISH mapping data (left, Figures 4A, DTLR2; 4B, DTLR3; 4C, DTLR4; 4D, DTLR5) with chromosomal bands was achieved by superimposing FISH signals with DAPI banded chromosomes (center panels). Heng and Tsui (1994) Meth. Molec. Biol. 33:109-122. Analyses are summarized in the form of human chromosome ideograms (right panels).

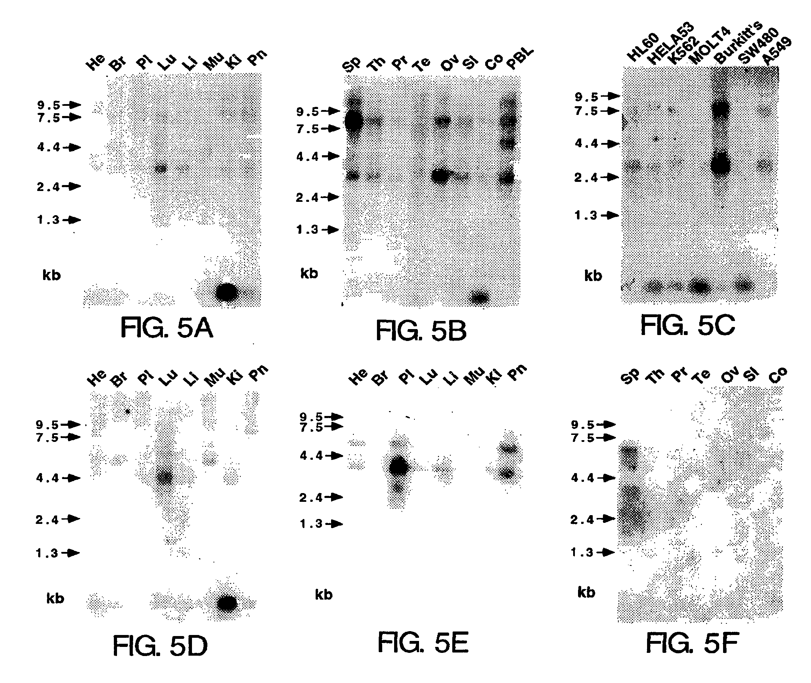

Figures 5A-5F show mRNA blot analyses of Human DTLRs. Human multiple tissue blots (He, heart; Br, brain; P1, placenta; Lu, lung; Li, liver; Mu, muscle; Ki, kidney; Pn, Pancreas; Sp, spleen; Th, thymus; Pr, prostate; Te, testis; Ov, ovary, SI, small intestine; Co, colon; PBL, peripheral blood lymphocytes) and cancer cell line (promyelocytic leukemia, HL60; cervical cancer, HELAS3; chronic myelogenous leukemia, K562; lymphoblastic leukemia, Molt4; colorectal adenocarcinoma, SW480; melanoma, G361; Burkitt's Lymphoma Raji, Burkitt's; colorectal adenocarcinoma, SW480; lung carcinoma, A549) containing approximately 2 µg of poly(A)⁺ RNA per lane were probed with radiolabeled cDNAs encoding DTLR1 (Figures 5A-5C), DTLR2 (Figure 5D), DTLR3 (Figure 5E), and DTLR4 (Figure 5F) as described. Blots were exposed to X-ray film for 2 days (Figures 5A-5C) or one week (Figure 5D-5F) at -70° C with intensifying screens. An anomalous 0.3 kB species appears in some lanes; hybridization experiments exclude a message encoding a DTLR cytoplasmic fragment.

### SUMMARY OF THE INVENTION

The present invention is directed to novel related mammalian receptors, e.g., human, Toll receptor like molecular structures, designated DTLR6, and their biological activities. It includes nucleic acids coding for the polypeptides themselves and methods for their production and use. The nucleic acids of the invention are characterized, in part, by their homology to cloned complementary DNA (cDNA) sequences enclosed herein.

The invention provides a composition of matter selected from the group of: a substantially pure or recombinant DTLR6 protein or peptide exhibiting at least about 85% sequence identity over a length of at least about 12 amino acids to SEQ ID NO: 12; a natural sequence DTLR6 of SEQ ID NO: 12; a fusion protein comprising DTLR6 sequence.

Preferably, the substantially pure or isolated protein comprises a segment exhibiting sequence identity to a corresponding portion of a DTLR6, wherein: the homology is at least about 90% identity and the portion is at least about 9 amino acids; the homology is at least about 80% identity and the portion is at least about 17 amino acids; or the homology is at least about 70% identity and the portion is at least about 25 amino acids. In specific embodiments, the composition of matter: is DTLR6, which comprises a mature sequence of SEQ ID NO: 12; or exhibits a post-translational modification pattern distinct from natural DTLR6; or the composition of matter may be a protein or peptide which: is from a warm blooded animal selected from a mammal, including a primate, such as a human; comprises at least one polypeptide segment of SEQ ID NO: 12; exhibits a plurality of portions exhibiting said identity; is a natural allelic variant of DTLR6; has a length at least about 30 amino acids; exhibits at least two non-overlapping epitopes which are specific for a primate DTLR6; exhibits a sequence identity at least about 90% over a length of at least about 20 amino acids to a primate DTLR6; exhibits at least two non-overlapping epitopes which are specific for a primate DTLR6; exhibits a sequence identity at least about 90% over a length of at least about 20 amino acids to a primate DTLR6; is glycosylated; has a molecular weight of at least 100 kD with natural glycosylation; is a synthetic polypeptide; is attached to a solid substrate; is conjugated to another chemical moiety; is a 5-fold or less substitution from natural sequence; or is a deletion or insertion variant from a natural sequence.

Other embodiments include a composition comprising: a sterile a sterile DTLR6 protein or peptide; or the DTLR6 protein or peptide and a carrier, wherein the carrier is: an aqueous compound, including water, saline, and/or buffer; and/or formulated for oral, rectal, nasal, topical, or parenteral administration.

In certain fusion protein embodiments, the invention provides a fusion protein comprising: mature protein sequence of SEQ ID NO: 12; a detection or purification tag, including a FLAG, His6, or Ig sequence; or sequence of another receptor protein.

Various kit embodiments include a kit comprising a DTLR protein or polypeptide, and: a compartment comprising the protein or polypeptide; and/or instructions for use or disposal of reagents in the kit.

Binding compound embodiments include those comprising an antigen binding site from an antibody, which specifically binds to a natural DTLR6 protein, wherein: the protein is a primate protein; the binding compound is an Fv, Fab, or Fab2 fragment; the binding compound is conjugated to another chemical moiety; or the antibody: is raised against a peptide sequence of a mature polypeptide of SEQ ID NO: 12 is raised against a mature DTLR6; is raised to a purified human DTLR6; is immunoselected; is a polyclonal antibody; binds to a denatured DTLR6; exhibits a Kd to antigen of at least 30 µM; is attached to a solid substrate, including a bead or plastic membrane; is in a sterile composition; or is detectably labeled, including a radioactive or fluorescent label. A binding composition kit often comprises the binding compound, and: a compartment comprising said binding compound; and/or instructions for use or disposal of reagents in the kit. Often the kit is capable of making a qualitative or quantitative analysis.

Other compositions include a composition comprising: a sterile binding compound, or the binding compound and a carrier, wherein the carrier is: an aqueous compound, including water, saline, and/or buffer; and/or formulated for oral, rectal, nasal, topical, or parenteral administration.

Nucleic acid embodiments include an isolated or recombinant nucleic acid encoding a DTLR2-10 protein or peptide or fusion protein, wherein: the DTLR is from a mammal; or the nucleic acid: encodes an antigenic peptide sequence of of SEQ ID NO: 12; encodes a plurality of antigenic peptide sequences of of SEQ ID NO: 12, exhibits at least about 80% identity to a natural cDNA encoding said segment; is an expression vector; further comprises an origin of replication; is from a natural source; comprises a detectable label; comprises synthetic nucleotide sequence; is less than 6 kb, preferably less than 3 kb; is from a mammal, including a primate; comprises a natural full length coding sequence; is a hybridization probe for a gene encoding said DTLR; or is a PCR primer, PCR product, or mutagenesis primer. A cell, tissue, or organ comprising such a recombinant nucleic acid is also provided. Preferably, the cell is: a prokaryotic cell; a eukaryotic cell; a bacterial cell; a yeast cell; an insect cell; a mammalian cell; a mouse cell; a primate cell; or a human cell. Kits are provided comprising such nucleic acids, and: a compartment comprising said nucleic acid; a compartment further comprising a primate DTLR6 protein or polypeptide; and/or instructions for use or disposal of reagents in the kit. Often, the kit is capable of making a qualitative or quantitative analysis.

Other embodiments include a nucleic acid which hybridizes under wash conditions of 30° C and less than 2M salt to SEQ ID NO: 11; exhibits at least about 85% identity over a stretch of at least about 30 nucleotides to a primate DTLR6.

Preferably, such nucleic acid will have such properties, wherein: wash conditions are at 45° C and/or 500 mM salt; or the identity is at least 90% and/or the stretch is at least 55 nucleotides. More preferably, the wash conditions are at 55° C and/or 150 mM salt; or the identity is at least 95% and/or the stretch is at least 75 nucleotides.

The invention also provides a method of modulating physiology or development of a cell or tissue culture cells comprising contacting the cell with an agonist or antagonist of a mammalian DTLR6.

### DETAILED DESCRIPTION OF THE PREFERRED EMBODIMENTS

### I. General

The present invention provides the amino acid sequence and DNA sequence of mammalian, herein primate DNAX Toll like receptor molecules (DTLR) having particular defined properties, both structural and biological. These have been designated herein as DTLR6. Various cDNAs encoding these molecules were obtained from primate, e.g., human, cDNA sequence libraries. Other primate or other mammalian counterparts would also be desired.

Some of the standard methods applicable are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols 1-3, CSH Press, NY; Ausubel, et al., Biology, Greene Publishing Associates, Brooklyn, NY; or Ausubel, et al. (1987 and periodic supplements) Current Protocols in Molecular Biology, Greene/Wiley, New York; each of which is incorporated herein by reference.

A complete nucleotide and corresponding amino acid sequence of a human DTLR1 coding segment is shown in SEQ ID NO: 1 and 2. See also Nomura, et al. (1994) DNA Res 1:27-35. A complete nucleotide and corresponding amino acid sequence of a human DTLR2 coding segment is shown in SEQ ID NO: 3 and 4. A complete nucleotide and corresponding amino acid sequence of a human DTLR3 coding segment is shown in SEQ ID NO: 5 and 6. A complete nucleotide and corresponding amino acid sequence of a human DTLR4 coding segment is shown in SEQ ID NO: 7 and 8. An alternate nucleic acid and corresponding amino acid sequence of a human DTLR4 coding segment is provided in SEQ ID NO: 25 and 26. A partial nucleotide and corresponding amino acid sequence of a human DTLR5 coding segment is shown in SEQ ID NO: 9 and 10. A complete nucleotide and corresponding amino acid sequence of a human DTLR6 coding segment is shown in SEQ ID NO: 11 and 12 and a partial sequence of a mouse DTLR6 is provided in SEQ ID NO: 13 and 14. Additional mouse DTLR6 sequence is provided in SEQ ID NO: 27 and 29 (nucleotide sequence) and SEQ ID NO: 28 and 30 (amino acid sequence). Partial nucleotide (SEQ ID NO: 15 and 17) and corresponding amino acid sequence (SEQ ID NO: 16 and 18) of a human DTLR7 coding segment is also provided. Partial nucleotide and corresponding amino acid sequence of a human DTLR8 coding segment is shown in SEQ ID NO: 19 and 20. A more complete nucleotide and corresponding amino acid sequence of a human DTLR coding segment is shown in SEQ ID NO: 31 and 32. Partial nucleotide and corresponding amino acid sequence of a human DTLR9 coding segment is shown in SEQ ID NO: 21 and 22. Partial nucleotide and corresponding amino acid sequence of a human DTLR10 coding segment is shown in SEQ ID NO: 23 and 24. More complete nucleotide and corresponding amino acid sequence of a human DTLR10 coding segment is shown in SEQ ID NO: 33 and 34. A partial nucleotide sequence for a mouse DTLR10 coding segment is provided in SEQ ID NO: 35.

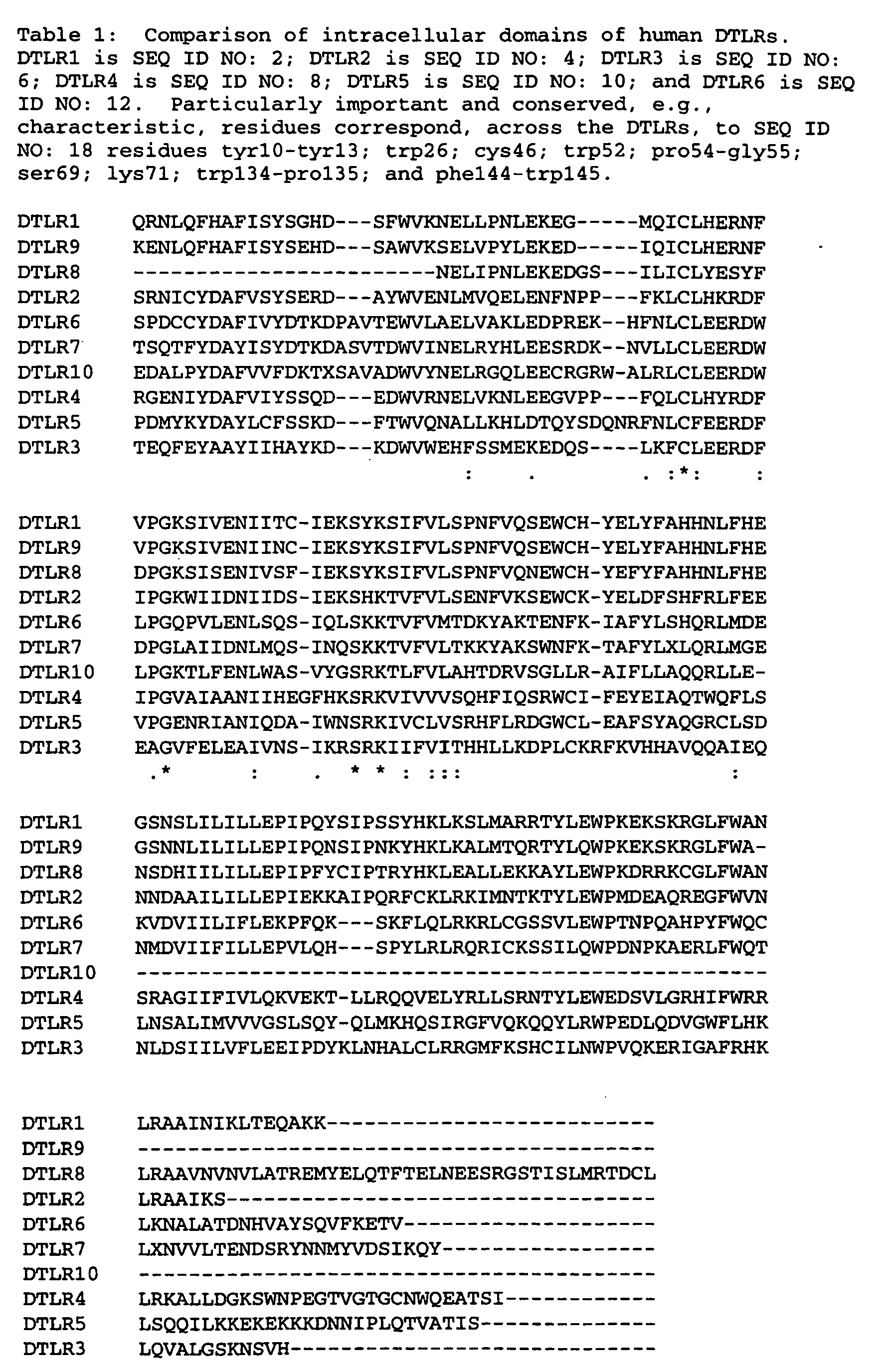

As used herein, the term DNAX Toll like receptor 2 (DTLR2) shall be used to describe a protein comprising a protein or peptide segment having or sharing the amino acid sequence shown in SEQ ID NO: 4, or a substantial fragment thereof. Similarly, with a DTLR3 and SEQ ID NO: 6; DTLR4 and SEQ ID NO: 26; DTLR5 and SEQ ID NO: 10; DTLR6 and SEQ ID NO: 12; DTLR7 and SEQ ID NO: 16 and 18; DTLR8 and SEQ ID NO: 32; DTLR9 and SEQ ID NO: 22; and DTLR10 and SEQ ID NO: 34.

The invention also includes a protein variations of the respective DTLR allele whose sequence is provided, e.g., a mutein agonist or antagonist. Typically, such agonists or antagonists will exhibit less than about 10% sequence differences, and thus will often have between 1- and 11-fold substitutions, e.g., 2-, 3-, 5-, 7-fold, and others. It also encompasses allelic and other variants, e.g., natural polymorphic, of the protein described. Typically, it will bind to its corresponding biological receptor with high affinity, e.g., at least about 100 nM, usually better than about 30 nM, preferably better than about 10 nM, and more preferably at better than about 3 nM. The term shall also be used herein to refer to related naturally occurring forms, e.g., alleles, polymorphic variants, and metabolic variants of the mammalian protein.

This invention also encompasses proteins or peptides having substantial amino acid sequence identity with the amino acid sequence in SEQ ID NO: 12. It will include sequence variants with relatively few substitutions, e.g., preferably less than about 3-5.

A substantial polypeptide "fragment", or "segment", is a stretch of amino acid residues of at least about 8 amino acids, generally at least 10 amino acids, more generally at least 12 amino acids, often at least 14 amino acids, more often at least 16 amino acids, typically at least 18 amino acids, more typically at least 20 amino acids, usually at least 22 amino acids, more usually at least 24 amino acids, preferably at least 26 amino acids, more preferably at least 28 amino acids, and, in particularly preferred embodiments, at least about 30 or more amino acids. Sequences of segments of different proteins can be compared to one another over appropriate length stretches.

Amino acid sequence homology, or sequence identity, is determined by optimizing residue matches, if necessary, by introducing gaps as required. See, e.g., Needleham, et al., (1970) J. Mol. Biol. 48:443-453; Sankoff, et al., (1983) chapter one in Time Warps, String Edits, and Macromolecules: The Theory and Practice of Sequence Comparsion, Addison-Wesley, Reading, MA; and software packages from IntelliGenetics, Mountain View, CA; and the University of Wisconsin Genetics Computer Group (GCG), Madison, WI; each of which is incorporated herein by reference. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Homologous amino acid sequences are intended to include natural allelic and interspecies variations in the cytokine sequence. Typical homologous proteins or peptides will have from 50-100% homology (if gaps can be introduced), to 60-100% homology (if conservative substitutions are included) with an amino acid sequence segment of SEQ ID NO: 12. Homology measures will be at least about 70%, generally at least 76%, more generally at least 81%, often at least 85%, more often at least 88%, typically at least 90%, more typically at least 92%, usually at least 94%, more usually at least 95%, preferably at least 96%, and more preferably at least 97%, and in particularly preferred embodiments, at least 98% or more. The degree of homology will vary with the length of the compared segments. Homologous proteins or peptides, such as the allelic variants, will share most biological activities with the embodiments described in SEQ ID NO: 12. Particularly interesting regions of comparison, at the amino acid or nucleotide levels, correspond to those within each of the blocks 1-10, or intrablock regions, corresponding to those indicated in Figure 2A.

As used herein, the term "biological activity" is used to describe, without limitation, effects on inflammatory responses, innate immunity, and/or morphogenic development by respective ligands. For example, these receptors should, like IL-1 receptors, mediate phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738. The receptors exhibit biological activities much like regulatable enzymes, regulated by ligand binding. However, the enzyme turnover number is more close to an enzyme than a receptor complex. Moreover, the numbers of occupied receptors necessary to induce such enzymatic activity is less than most receptor systems, and may number closer to dozens per cell, in contrast to most receptors which will trigger at numbers in the thousands per cell. The receptors, or portions thereof, may be useful as phosphate labeling enzymes to label general or specific substrates.

The terms ligand, agonist, antagonist, and analog of, e.g., a DTLR, include molecules that modulate the characteristic cellular responses to Toll ligand like proteins, as well as molecules possessing the more standard structural binding competition features of ligand-receptor interactions, e.g., where the receptor is a natural receptor or an antibody. The cellular responses likely are mediated through binding of various Toll ligands to cellular receptors related to, but possibly distinct from, the type I or type II IL-1 receptors. See, e.g., Belvin and Anderson (1996) Ann. Rev. Cell Dev. Biol. 12:393-416; Morisato and Anderson (1995) Ann. Rev. Genetics 29:371-3991 and Hultmark (1994) Nature 367:116-117.

Also, a ligand is a molecule which serves either as a natural ligand to which said receptor, or an analog thereof, binds, or a molecule which is a functional analog of the natural ligand. The functional analog may be a ligand with structural modifications, or may be a wholly unrelated molecule which has a molecular shape which interacts with the appropriate ligand binding determinants. The ligands may serve as agonists or antagonists, see, e.g., Goodman, et al. (eds) (1990) Goodman & Gilman's: The Pharmacological Bases of Therapeutics, Pergamon Press, New York.

Rational drug design may also be based upon structural studies of the molecular shapes of a receptor or antibody and other effectors or ligands. Effectors may be other proteins which mediate other functions in response to ligand binding, or other proteins which normally interact with the receptor. One means for determining which sites interact with specific other proteins is a physical structure determination, e.g., x-ray crystallography or 2 dimensional NMR techniques. These will provide guidance as to which amino acid residues form molecular contact regions. For a detailed description of protein structural determination, see, e.g., Blundell and Johnson (1976) Protein Crystallography, Academic Press, New York, which is hereby incorporated herein by reference.

### II. Activities

The Toll like receptor proteins will have a number of different biological activities, e.g., in phosphate metabolism, being added to or removed from specific substrates, typically proteins. Such will generally result in modulation of an inflammatory function, other innate immunity response, or a morphological effect. The DTLR 6, proteins are homologous to other Toll like receptor proteins, but each have structural differences. At the amino acid level, there is also likely to be reasonable identity.

The biological activities of the DTLRs will be related to addition or removal of phosphate moieties to substrates, typically in a specific manner, but occasionally in a non specific manner. Substrates may be identified, or conditions for enzymatic activity may be assayed by standard methods, e.g., as described in Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738.

### III. Nucleic Acids

This invention contemplates use of isolated nucleic acid or fragments, e.g., which encode these or closely related proteins, or fragments thereof, e.g., to encode a corresponding polypeptide, preferably one which is biologically active. In addition, this invention covers isolated or recombinant DNA which encodes such proteins or polypeptides having characteristic sequences of the respective DTLRs, individually or as a group. Typically, the nucleic acid is capable of hybridizing, under appropriate conditions, with a nucleic acid sequence segment shown in SEQ ID NOs: 11 but preferably not with a corresponding segment of SEQ ID NO: 1. Said biologically active protein or polypeptide can be a full length protein, or fragment, and will typically have a segment of amino acid sequence highly homologous to one shown in SEQ ID NO: 6. Further, this invention covers the use of isolated or recombinant nucleic acid, or fragments thereof, which encode proteins having fragments which are equivalent to the DTLR2-10 proteins. The isolated nucleic acids can have the respective regulatory sequences in the 5' and 3' flanks, e.g., promoters, enhancers, poly-A addition signals, and others from the natural gene.

An "isolated" nucleic acid is a nucleic acid, e.g., an RNA, DNA, or a mixed polymer, which is substantially pure, e.g., separated from other components which naturally accompany a native sequence, such as ribosomes, polymerases, and flanking genomic sequences from the originating species. The term embraces a nucleic acid sequence which has been removed from its naturally occurring environment, and includes recombinant or cloned DNA isolates, which are thereby distinguishable from naturally occurring compositions, and chemically synthesized analogs or analogs biologically synthesized by heterologous systems. A substantially pure molecule includes isolated forms of the molecule, either completely or substantially pure.

An isolated nucleic acid will generally be a homogeneous composition of molecules, but will, in some embodiments, contain heterogeneity, preferably minor. This heterogeneity is typically found at the polymer ends or portions not critical to a desired biological function or activity.

A "recombinant" nucleic acid is typically defined either by its method of production or its structure. In reference to its method of production, e.g., a product made by a process, the process is use of recombinant nucleic acid techniques, e.g., involving human intervention in the nucleotide sequence. Typically this intervention involves in vitro manipulation, although under certain circumstances it may involve more classical animal breeding techniques. Alternatively, it can be a nucleic acid made by generating a sequence comprising fusion of two fragments which are not naturally contiguous to each other, but is meant to exclude products of nature, e.g., naturally occurring mutants as found in their natural state. Thus, for example, products made by transforming cells with any unnaturally occurring vector is encompassed, as are nucleic acids comprising sequence derived using any synthetic oligonucleotide process. Such a process is often done to replace a codon with a redundant codon encoding the same or a conservative amino acid, while typically introducing or removing a restriction enzyme sequence recognition site. Alternatively, the process is performed to join together nucleic acid segments of desired functions to generate a single genetic entity comprising a desired combination of functions not found in the commonly available natural forms, e.g., encoding a fusion protein. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. This will include a dimeric repeat. Specifically included are synthetic nucleic acids which, by genetic code redundancy, encode equivalent polypeptides to fragments of DTLR6 and fusions of sequences from various different related molecules, e.g., other IL-1 receptor family members.

A "fragment" in a nucleic acid context is a contiguous segment of at least about 17 nucleotides, generally at least 21 nucleotides, more generally at least 25 nucleotides, ordinarily at least 30 nucleotides, more ordinarily at least 35 nucleotides, often at least 39 nucleotides, more often at least 45 nucleotides, typically at least 50 nucleotides, more typically at least 55 nucleotides, usually at least 60 nucleotides, more usually at least 66 nucleotides, preferably at least 72 nucleotides, more preferably at least 79 nucleotides, and in particularly preferred embodiments will be at least 85 or more nucleotides. Typically, fragments of different genetic sequences can be compared to one another over appropriate length stretches, particularly defined segments such as the domains described below.

A nucleic acid which codes for a DTLR2-10 will be particularly useful to identify genes, mRNA, and cDNA species which code for itself or closely related proteins, as well as DNAs which code for polymorphic, allelic, or other genetic variants, e.g., from different individuals or related species. Preferred probes for such screens are those regions of the interleukin which are conserved between different polymorphic variants or which contain nucleotides which lack specificity, and will preferably be full length or nearly so. In other situations, polymorphic variant specific sequences will be more useful.

This invention further covers recombinant nucleic acid molecules and fragments having a nucleic acid sequence identical to or highly homologous to the isolated DNA set forth herein. In particular, the sequences will often be operably linked to DNA segments which control transcription, translation, and DNA replication. These additional segments typically assist in expression of the desired nucleic acid segment.

Homologous, or highly identical, nucleic acid sequences, when compared to one another or the sequences shown in SEQ ID NO: 11 exhibit significant similarity. The standards for homology in nucleic acids are either measures for homology generally used in the art by sequence comparison or based upon hybridization conditions. Comparative hybridization conditions are described in greater detail below.

Substantial identity in the nucleic acid sequence comparison context means either that the segments, or their complementary strands, when compared, are identical when optimally aligned, with appropriate nucleotide insertions or deletions, in at least about 60% of the nucleotides, generally at least 66%, ordinarily at least 71%, often at least 76%, more often at least 80%, usually at least 84%, more usually at least 88%, typically at least 91%, more typically at least about 93%, preferably at least about 95%, more preferably at least about 96 to 98% or more, and in particular embodiments, as high at about 99% or more of the nucleotides, including, e.g., segments encoding structural domains such as the segments described below. Alternatively, substantial identity will exist when the segments will hybridize under selective hybridization conditions, to a strand or its complement, typically using a sequence derived from SEQ ID NO: 11.
Typically, selective hybridization will occur when there is at least about 55% homology over a stretch of at least about 14 nucleotides, more typically at least about 65%, preferably at least about 75%, and more preferably at least about 90%. See, Kanehisa (1984) Nuc. Acids Res. 12:203-213, which is incorporated herein by reference. The length of homology comparison, as described, may be over longer stretches, and in certain embodiments will be over a stretch of at least about 17 nucleotides, generally at least about 20 nucleotides, ordinarily at least about 24 nucleotides, usually at least about 28 nucleotides, typically at least about 32 nucleotides, more typically at least about 40 nucleotides, preferably at least about 50 nucleotides, and more preferably at least about 75 to 100 or more nucleotides.

Stringent conditions, in referring to homology in the hybridization context, will be stringent combined conditions of salt, temperature, organic solvents, and other parameters typically controlled in hybridization reactions. Stringent temperature conditions will usually include temperatures in excess of about 30° C, more usually in excess of about 37° C, typically in excess of about 45° C, more typically in excess of about 55° C, preferably in excess of about 65° C, and more preferably in excess of about 70° C. Stringent salt conditions will ordinarily be less than about 500 mM, usually less than about 400 mM, more usually less than about 300 mM, typically less than about 200 mM, preferably less than about 100 mM, and more preferably less than about 80 mM, even down to less than about 20 mM. However, the combination of parameters is much more important than the measure of any single parameter. See, e.g., Wetmur and Davidson (1968) J. Mol. Biol. 31:349-370, which is hereby incorporated herein by reference.

Alternatively, for sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optical alignment of sequences for comparison can be conducted, e.g., by the local homology algorithm of Smith and Waterman (1981) Adv. Appl. Math. 2:482, by the homology alignment algorithm of Needlman and Wunsch (1970) J. Mol. Biol. 48:443, by the search for similarity method of Pearson and Lipman (1988) Proc. Nat'l Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra).

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng and Doolittle (1987) J. Mol. Evol. 35:351-360. The method used is similar to the method described by Higgins and Sharp (1989) CABIOS 5:151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. For example, a reference sequence can be compared to other test sequences to determine the percent sequence identity relationship using the following parameters: default gap weight (3.00), default gap length weight (0.10), and weighted end gaps.

Another example of algorithm that is suitable for determining percent sequence identity and sequence similarity is the BLAST algorithm, which is described Altschul, et al. (1990) J. Mol. Biol. 215:403-410. Software for performing BLAST analyses is publicly available through the National Center for Biotechnology Information (http:www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul, et al., supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff and Henikoff (1989) Proc. Nat'l Acad. Sci. USA 89:10915) alignments (B) of 50, expectation (E) of 10, M=5, N=4, and a comparison of both strands.

In addition to calculating percent sequence identity, the BLAST algorithm also performs a statistical analysis of the similarity between two sequences (see, e.g., Karlin and Altschul (1993) Proc. Nat'l Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

A further indication that two nucleic acid sequences of polypeptides are substantially identical is that the polypeptide encoded by the first nucleic acid is immunologically cross reactive with the polypeptide encoded by the second nucleic acid, as described below. Thus, a polypeptide is typically substantially identical to a second polypeptide, e.g., where the two peptides differ only by conservative substitutions. Another indication that two nucleic acid sequences are substantially identical is that the two molecules hybridize to each other under stringent conditions, as described below.

The isolated DNA can be readily modified by nucleotide substitutions, nucleotide deletions, nucleotide insertions, and inversions of nucleotide stretches. These modifications result in novel DNA sequences which encode this protein or its derivatives. These modified sequences can be used to produce mutant proteins (muteins) or to enhance the expression of variant species. Enhanced expression may involve gene amplification, increased transcription, increased translation, and other mechanisms. Such mutant DTLR-like derivatives include predetermined or site-specific mutations of the protein or its fragments, including silent mutations using genetic code degeneracy. "Mutant DTLR" as used herein encompasses a polypeptide otherwise falling within the homology definition of the DTLR as set forth above, but having an amino acid sequence which differs from that of other DTLR-like proteins as found in nature, whether by way of deletion, substitution, or insertion. In particular, "site specific mutant DTLR" encompasses a protein having substantial homology with a protein of SEQ ID NO:12 and typically shares most of the biological activities or effects of the forms disclosed herein.

Although site specific mutation sites are predetermined, mutants need not be site specific. Mammalian DTLR mutagenesis can be achieved by making amino acid insertions or deletions in the gene, coupled with expression. Substitutions, deletions, insertions, or any combinations may be generated to arrive at a final construct. Insertions include amino- or carboxy-terminal fusions. Random mutagenesis can be conducted at a target codon and the expressed mammalian DTLR mutants can then be screened for the desired activity. Methods for making substitution mutations at predetermined sites in DNA having a known sequence are well known in the art, e.g., by M13 primer mutagenesis. See also Sambrook, et al. (1989) and Ausubel, et al. (1987 and periodic Supplements).

The mutations in the DNA normally should not place coding sequences out of reading frames and preferably will not create complementary regions that could hybridize to produce secondary mRNA structure such as loops or hairpins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Polymerase chain reaction (PCR) techniques can often be applied in mutagenesis. Alternatively, mutagenisis primers are commonly used methods for generating defined mutations at predetermined sites. See, e.g, Innis, et al. (eds. 1990) PCR Protocols: A Guide to Methods and Applications Academic Press, San Diego, CA; and Dieffenbach and Dveksler (1995; eds.) PCR Primer: A Laboratory Manual Cold Spring Harbor Press, CSH, NY.

### IV. Proteins, Peptides

As described above, the present invention encompasses primate DTLR6 whose sequences are disclosed in SEQ ID NOS: 12 and described above. Allelic and other variants are also contemplated, including, e.g., fusion proteins combining portions of such sequences with others, including epitope tags and functional domains.

The present invention also provides recombinant proteins, e.g., heterologous fusion proteins using segments from these rodent proteins. A heterologous fusion protein is a fusion of proteins or segments which are naturally not normally fused in the same manner. Thus, the fusion product of a DTLR with an IL-1 receptor is a continuous protein molecule having sequences fused in a typical peptide linkage, typically made as a single translation product and exhibiting properties, e.g., sequence or antigenicity, derived from each source peptide. A similar concept applies to heterologous nucleic acid sequences.

In addition, new constructs may be made from combining similar functional or structural domains from other related proteins, e.g., IL-1 receptors or other DTLRs, including species variants. For example, ligand-binding or other segments may be "swapped" between different new fusion polypeptides or fragments. See, e.g., Cunningham, et al. (1989) Science 243:1330-1336; and O'Dowd, et al. (1988) J. Biol. Chem. 263:15985-15992, each of which is incorporated herein by reference. Thus, new chimeric polypeptides exhibiting new combinations of specificities will result from the functional linkage of receptor-binding specificities. For example, the ligand binding domains from other related receptor molecules may be added or substituted for other domains of this or related proteins. The resulting protein will often have hybrid function and properties. For example, a fusion protein may include a targetting domain which may serve to provide sequestering of the fusion protein to a particular subcellular organelle.

Candidate fusion partners and sequences can be selected from various sequence data bases, e.g., GenBank, c/o IntelliGenetics, Mountain View, CA; and BCG, University of Wisconsin Biotechnology Computing Group, Madison, WI, which are each incorporated herein by reference.

The present invention particularly provides muteins which bind Toll ligands, and/or which are affected in signal transduction. Structural alignment of human DTLR1-10 with other members of the IL-1 family show conserved features/residues. See, e.g., Figure 3A. Alignment of the human DTLR sequences with other members of the IL-1 family indicates various structural and functionally shared features. See also, Bazan, et al. (1996) Nature 379:591; Lodi, et al. (1994) Science 263:1762-1766; Sayle and Milner-White (1995) TIBS 20:374-376; and Gronenberg, et al. (1991) Protein Engineering 4:263-269.

The IL-1α and IL-1β ligands bind an IL-1 receptor type I as the primary receptor and this complex then forms a high affinity receptor complex with the IL-1 receptor type III. Such receptor subunits are probably shared with the new IL-1 family members.

Similar variations in other species counterparts of DTLR6 sequences, e.g., in the corresponding regions, should provide similar interactions with ligand or substrate. Substitutions with either mouse sequences or human sequences are particularly preferred. Conversely, conservative substitutions away from the ligand binding interaction regions will probably preserve most signaling activities.

"Derivatives" of the primate DTLR6 include amino acid sequence mutants, glycosylation variants, metabolic derivatives and covalent or aggregative conjugates with other chemical moieties. Covalent derivatives can be prepared by linkage of functionalities to groups which are found in the DTLR amino acid side chains or at the N- or C- termini, e.g., by means which are well known in the art. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties including C3 to C18 normal alkyl, thereby forming alkanoyl aroyl species.

In particular, glycosylation alterations are included, e.g., made by modifying the glycosylation patterns of a polypeptide during its synthesis and processing, or in further processing steps. Particularly preferred means for accomplishing this are by exposing the polypeptide to glycosylating enzymes derived from cells which normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine.

A major group of derivatives are covalent conjugates of the receptors or fragments thereof with other proteins of polypeptides. These derivatives can be synthesized in recombinant culture such as N- or C-terminal fusions or by the use of agents known in the art for their usefulness in cross-linking proteins through reactive side groups. Preferred derivatization sites with cross-linking agents are at free amino groups, carbohydrate moieties, and cysteine residues.

Fusion polypeptides between the receptors and other homologous or heterologous proteins are also provided. Homologous polypeptides may be fusions between different receptors, resulting in, for instance, a hybrid protein exhibiting binding specificity for multiple different Toll ligands, or a receptor which may have broadened or weakened specificity of substrate effect. Likewise, heterologous fusions may be constructed which would exhibit a combination of properties or activities of the derivative proteins. Typical examples are fusions of a reporter polypeptide, e.g., luciferase, with a segment or domain of a receptor, e.g., a ligand-binding segment, so that the presence or location of a desired ligand may be easily determined. See, e.g., Dull, et al., U.S. Patent No. 4,859,609, which is hereby incorporated herein by reference. Other gene fusion partners include glutathione-S-transferase (GST), bacterial β-galactosidase, trpE, Protein A, β-lactamase, alpha amylase, alcohol dehydrogenase, and yeast alpha mating factor. See, e.g., Godowski, et al. (1988) Science 241:812-816.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Such polypeptides may also have amino acid residues which have been chemically modified by phosphorylation, sulfonation, biotinylation, or the addition or removal of other moieties, particularly those which have molecular shapes similar to phosphate groups. In some embodiments, the modifications will be useful labeling reagents, or serve as purification targets, e.g., affinity ligands.

Fusion proteins will typically be made by either recombinant nucleic acid methods or by synthetic polypeptide methods. Techniques for nucleic acid manipulation and expression are described generally, for example, in Sambrook, et al. (1989) Molecular Cloning: A Laboratory Manual (2d ed.), Vols. 1-3, Cold Spring Harbor Laboratory, and Ausubel, et al. (eds. 1987 and periodic supplements) Current Protocols in Molecular Biology, Greene/Wiley, New York, which are each incorporated herein by reference. Techniques for synthesis of polypeptides are described, for example, in Merrifield (1963) J. Amer. Chem. Soc. 85:2149-2156; Merrifield (1986) Science 232: 341-347; and Atherton, et al. (1989) Solid Phase Peptide Synthesis: A Practical Approach, IRL Press, Oxford; each of which is incorporated herein by reference. See also Dawson, et al. (1994) Science 266:776-779 for methods to make larger polypeptides.

This invention also contemplates the use of derivatives of a DTLR6 other than variations in amino acid sequence or glycosylation. Such derivatives may involve covalent or aggregative association with chemical moieties. These derivatives generally fall into three classes: (1) salts, (2) side chain and terminal residue covalent modifications, and (3) adsorption complexes, for example with cell membranes. Such covalent or aggregative derivatives are useful as immunogens, as reagents in immunoassays, or in purification methods such as for affinity purification of a receptor or other binding molecule, e.g., an antibody. For example, a Toll ligand can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of a DTLR receptor, antibodies, or other similar molecules. The ligand can also be labeled with a detectable group, for example radioiodinated by the chloramine T procedure, covalently bound to rare earth chelates, or conjugated to another fluorescent moiety for use in diagnostic assays.

A DTLR of this invention can be used as an immunogen for the production of antisera or antibodies specific, e.g., capable of distinguishing between other IL-1 receptor family members, for the DTLR or various fragments thereof. The purified DTLR can be used to screen monoclonal antibodies or antigen-binding fragments prepared by immunization with various forms of impure preparations containing the protein. In particular, the term "antibodies" also encompasses antigen binding fragments of natural antibodies, e.g., Fab, Fab2, Fv, etc. The purified DTLR can also be used as a reagent to detect antibodies generated in response to the presence of elevated levels of expression, or immunological disorders which lead to antibody production to the endogenous receptor. Additionally, DTLR fragments may also serve as immunogens to produce the antibodies of the present invention, as described immediately below. For example, this invention contemplates antibodies having binding affinity to or being raised against the amino acid sequences shown in SEQ ID NOS: 12 fragments thereof. In particular, this invention contemplates antibodies having binding affinity to, or having been raised against, specific fragments which are predicted to be, or actually are, exposed at the exterior protein surface of the native DTLR.

The blocking of physiological response to the receptor ligands may result from the inhibition of binding of the ligand to the receptor, likely through competitive inhibition. Thus, in vitro assays of the present invention will often use antibodies or antigen binding segments of these antibodies, or fragments attached to solid phase substrates. These assays will also allow for the diagnostic determination of the effects of either ligand binding region mutations and modifications, or other mutations and modifications, e.g., whcih affect signaling or enzymatic function.

This invention also contemplates the use of competitive drug screening assays, e.g., where neutralizing antibodies to the receptor or fragments compete with a test compound for binding to a ligand or other antibody. In this manner, the neutralizing antibodies or fragments can be used to detect the presence of a polypeptide which shares one or more binding sites to a receptor and can also be used to occupy binding sites on a receptor that might otherwise bind a ligand.

### V. Making Nucleic Acids and Protein

DNA which encodes the protein or fragments thereof can be obtained by chemical synthesis, screening cDNA libraries, or by screening genomic libraries prepared from a wide variety of cell lines or tissue samples. Natural sequences can be isolated using standard methods and the sequences provided herein. Other species counterparts can be identified by hybridization techniques, or by various PCR techniques, combined with or by searching in sequence databases, e.g., GenBank.

This DNA can be expressed in a wide variety of host cells for the synthesis of a full-length receptor or fragments which can in turn, for example, be used to generate polyclonal or monoclonal antibodies; for binding studies; for construction and expression of modified ligand binding or kinase/phosphatase domains; and for structure/function studies. Variants or fragments can be expressed in host cells that are transformed or transfected with appropriate expression vectors. These molecules can be substantially free of protein or cellular contaminants, other than those derived from the recombinant host, and therefore are particularly useful in pharmaceutical compositions when combined with a pharmaceutically acceptable carrier and/or diluent. The protein, or portions thereof, may be expressed as fusions with other proteins.

Expression vectors are typically self-replicating DNA or RNA constructs containing the desired receptor gene or its fragments, usually operably linked to suitable genetic control elements that are recognized in a suitable host cell. These control elements are capable of effecting expression within a suitable host. The specific type of control elements necessary to effect expression will depend upon the eventual host cell used. Generally, the genetic control elements can include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention include those which contain DNA which encodes a protein, as described, or a fragment thereof encoding a biologically active equivalent polypeptide. The DNA can be under the control of a viral promoter and can encode a selection marker. This invention further contemplates use of such expression vectors which are capable of expressing eukaryotic cDNA coding for such a protein in a prokaryotic or eukaryotic host, where the vector is compatible with the host and where the eukaryotic cDNA coding for the receptor is inserted into the vector such that growth of the host containing the vector expresses the cDNA in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the protein or its fragments in various hosts using vectors that do not contain a replication origin that is recognized by the host cell. It is also possible to use vectors that cause integration of the protein encoding portion or its fragments into the host DNA by recombination.

Vectors, as used herein, comprise plasmids, viruses, bacteriophage, integratable DNA fragments, and other vehicles which enable the integration of DNA fragments into the genome of the host. Expression vectors are specialized vectors which contain genetic control elements that effect expression of operably linked genes. Plasmids are the most commonly used form of vector but all other forms of vectors which serve an equivalent function and which are, or become, known in the art are suitable for use herein. See, e.g., Pouwels, et al. (1985 and Supplements) Cloning Vectors: A Laboratory Manual, Elsevier, N.Y., and Rodriquez, et al. (eds) Vectors: A Survey of Molecular Cloning Vectors and Their Uses, Buttersworth, Boston, 1988, which are incorporated herein by reference.

Transformed cells are cells, preferably mammalian, that have been transformed or transfected with receptor vectors constructed using recombinant DNA techniques. Transformed host cells usually express the desired protein or its fragments, but for purposes of cloning, amplifying, and manipulating its DNA, do not need to express the subject protein. This invention further contemplates culturing transformed cells in a nutrient medium, thus permitting the receptor to accumulate in the cell membrane. The protein can be recovered, either from the culture or, in certain instances, from the culture medium.

For purposes of this invention, nucleic sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include prokaryotes, lower eukaryotes, and higher eukaryotes. Prokaryotes include both gram negative and gram positive organisms, e.g., E. coli and B. subtilis. Lower eukaryotes include yeasts, e.g., S. cerevisiae and Pichia, and species of the genus Dictyostelium. Higher eukaryotes include established tissue culture cell lines from animal cells, both of non-mammalian origin, e.g., insect cells, and birds, and of mammalian origin, e.g., human, primates, and rodents.

Prokaryotic host-vector systems include a wide variety of vectors for many different species. As used herein, E. coli and its vectors will be used generically to include equivalent vectors used in other prokaryotes. A representative vector for amplifying DNA is pBR322 or many of its derivatives. Vectors that can be used to express the receptor or its fragments include, but are not limited to, such vectors as those containing the lac promoter (pUC-series); trp promoter (pBR322-trp); Ipp promoter (the pIN-series); lambda-pP or pR promoters (pOTS); or hybrid promoters such as ptac (pDR540). See Brosius, et al. (1988) "Expression Vectors Employing Lambda-, trp-, lac-, and Ipp-derived Promoters", in Vectors: A Survey of Molecular Cloning Vectors and Their Uses, (eds. Rodriguez and Denhardt), Buttersworth, Boston, Chapter 10, pp. 205-236, which is incorporated herein by reference.

Lower eukaryotes, e.g., yeasts and Dictyostelium, may be transformed with DTLR sequence containing vectors. For purposes of this invention, the most common lower eukaryotic host is the baker's yeast, Saccharomyces cerevisiae. It will be used to generically represent lower eukaryotes although a number of other strains and species are also available. Yeast vectors typically consist of a replication origin (unless of the integrating type), a selection gene, a promoter, DNA encoding the receptor or its fragments, and sequences for translation termination, polyadenylation, and transcription termination. Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-replicating high copy number (such as the YEp-series); integrating types (such as the YIp-series), or mini-chromosomes (such as the YCp-series).

Higher eukaryotic tissue culture cells are normally the preferred host cells for expression of the functionally active interleukin protein. In principle, any higher eukaryotic tissue culture cell line is workable, e.g., insect baculovirus expression systems, whether from an invertebrate or vertebrate source. However, mammalian cells are preferred. Transformation or transfection and propagation of such cells has become a routine procedure. Examples of useful cell lines include HeLa cells, Chinese hamster ovary (CHO) cell lines, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also usually contain a selection gene or amplification gene. Suitable expression vectors may be plasmids, viruses, or retroviruses carrying promoters derived, e.g., from such sources as from adenovirus, SV40, parvoviruses, vaccinia virus, or cytomegalovirus. Representative examples of suitable expression vectors include pCDNA1; pCD, see Okayama, et al. (1985) Mol. Cell Biol. 5:1136-1142; pMClneo PolyA, see Thomas, et al. (1987) Cell 51:503-512; and a baculovirus vector such as pAC 373 or pAC 610.

For secreted proteins, an open reading frame usually encodes a polypeptide that consists of a mature or secreted product covalently linked at its N-terminus to a signal peptide. The signal peptide is cleaved prior to secretion of the mature, or active, polypeptide. The cleavage site can be predicted with a high degree of accuracy from empirical rules, e.g., von-Heijne (1986) Nucleic Acids Research 14:4683-4690, and the precise amino acid composition of the signal peptide does not appear to be critical to its function, e.g., Randall, et al. (1989) Science 243:1156-1159; Kaiser st al. (1987) Science 235:312-317.

It will often be desired to express these polypeptides in a system which provides a specific or defined glycosylation pattern. In this case, the usual pattern will be that provided naturally by the expression system. However, the pattern will be modifiable by exposing the polypeptide, e.g., an unglycosylated form, to appropriate glycosylating proteins introduced into a heterologous expression system. For example, the receptor gene may be co-transformed with one or more genes encoding mammalian or other glycosylating enzymes. Using this approach, certain mammalian glycosylation patterns will be achievable in prokaryote or other cells.

The source of DTLR can be a eukaryotic or prokaryotic host expressing recombinant DTLR, such as is described above. The source can also be a cell line such as mouse Swiss 3T3 fibroblasts, but other mammalian cell lines are also contemplated by this invention, with the preferred cell line being from the human species.

Now that the sequences are known, the primate DTLRs, fragments, or derivatives thereof can be prepared by conventional processes for synthesizing peptides. These include processes such as are described in Stewart and Young (1984) Solid Phase Peptide Synthesis, Pierce Chemical Co., Rockford, IL; Bodanszky and Bodanszky (1984) The Practice of Peptide Synthesis, Springer-Verlag, New York; and Bodanszky (1984) The Principles of Peptide Synthesis, Springer-Verlag, New York; all of each which are incorporated herein by reference. For example, an azide process, an acid chloride process, an acid anhydride process, a mixed anhydride process, an active ester process (e.g., p-nitrophenyl ester, N-hydroxysuccinimide ester, or cyanomethyl ester), a carbodiimidazole process, an oxidative-reductive process, or a dicyclohexylcarbodiimide (DCCD)/additive process can be used. Solid phase and solution phase syntheses are both applicable to the foregoing processes. Similar techniques can be used with partial DTLR sequences.

The DTLR proteins, fragments, or derivatives are suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino acid. Amino groups that are not being used in the coupling reaction typically must be protected to prevent coupling at an incorrect location.

If a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins, such as chloromethyl resin or bromomethyl resin, hydroxymethyl resins, phenol resins, tert-alkyloxycarbonylhydrazidated resins, and the like.

An amino group-protected amino acid is bound in sequence through condensation of its activated carboxyl group and the reactive amino group of the previously formed peptide or chain, to synthesize the peptide step by step. After synthesizing the complete sequence, the peptide is split off from the insoluble carrier to produce the peptide. This solid-phase approach is generally described by Merrifield, et al. (1963) in J. Am. Chem. Soc. 85:2149-2156, which is incorporated herein by reference.

The prepared protein and fragments thereof can be isolated and purified from the reaction mixture by means of peptide separation, for example, by extraction, precipitation, electrophoresis, various forms of chromatography, and the like. The receptors of this invention can be obtained in varying degrees of purity depending upon desired uses. Purification can be accomplished by use of the protein purification techniques disclosed herein, see below, or by the use of the antibodies herein described in methods of immunoabsorbant affinity chromatography. This immunoabsorbant affinity chromatography is carried out by first linking the antibodies to a solid support and then contacting the linked antibodies with solubilized lysates of appropriate cells, lysates of other cells expressing the receptor, or lysates or supernatants of cells producing the protein as a result of DNA techniques, see below.

Generally, the purified protein will be at least about 40% pure, ordinarily at least about 50% pure, usually at least about 60% pure, typically at least about 70% pure, more typically at least about 80% pure, preferable at least about 90% pure and more preferably at least about 95% pure, and in particular embodiments, 97%-99% or more. Purity will usually be on a weight basis, but can also be on a molar basis. Different assays will be applied as appropriate.

### VI. Antibodies

Antibodies can be raised to the various mammalian, e.g., primate DTLR proteins and fragments thereof, both in naturally occurring native forms and in their recombinant forms, the difference being that antibodies to the active receptor are more likely to recognize epitopes which are only present in the native conformations. Denatured antigen detection can also be useful in, e.g., Western analysis. Anti-idiotypic antibodies are also contemplated, which would be useful as agonists or antagonists of a natural receptor or an antibody.

Antibodies, including binding fragments and single chain versions, against predetermined fragments of the protein can be raised by immunization of animals with conjugates of the fragments with immunogenic proteins. Monoclonal antibodies are prepared from cells secreting the desired antibody. These antibodies can be screened for binding to normal or defective protein, or screened for agonistic or antagonistic activity. These monoclonal antibodies will usually bind with at least a K_{D} of about 1 mM, more usually at least about 300 µM, typically at least about 100µM, more typically at least about 30 µM, preferably at least about 10 µM, and more preferably at least about 3 µM or better.

The antibodies, including antigen binding fragments, of this invention can have significant diagnostic or therapeutic value. They can be potent antagonists that bind to the receptor and inhibit binding to ligand or inhibit the ability of the receptor to elicit a biological response, e.g., act on its substrate. They also can be useful as non-neutralizing antibodies and can be coupled to toxins or radionuclides to bind producing cells, or cells localized to the source of the interleukin. Further, these antibodies can be conjugated to drugs or other therapeutic agents, either directly or indirectly by means of a linker.

The antibodies of this invention can also be useful in diagnostic applications. As capture or non-neutralizing antibodies, they might bind to the receptor without inhibiting ligand or substrate binding. As neutralizing antibodies, they can be useful in competitive binding assays. They will also be useful in detecting or quantifying ligand. They may be used as reagents for Western blot analysis, or for immunoprecipitation or immunopurification of the respective protein.

Protein fragments may be joined to other materials, particularly polypeptides, as fused or covalently joined polypeptides to be used as immunogens. Mammalian DTLR and its fragments may be fused or covalently linked to a variety of immunogens, such as keyhole limpet hemocyanin, bovine serum albumin, tetanus toxoid, etc. See Microbiology, Hoeber Medical Division, Harper and Row, 1969; Landsteiner (1962) Specificity of Serological Reactions, Dover Publications, New York; and Williams, et al. (1967) Methods in Immunology and Immunochemistry, Vol. 1, Academic Press, New York; each of which are incorporated herein by reference, for descriptions of methods of preparing polyclonal antisera. A typical method involves hyperimmunization of an animal with an antigen. The blood of the animal is then collected shortly after the repeated immunizations and the gamma globulin is isolated.

In some instances, it is desirable to prepare monoclonal antibodies from various mammalian hosts, such as mice, rodents, primates, humans, etc. Description of techniques for preparing such monoclonal antibodies may be found in, e.g., Stites, et al. (eds) Basic and Clinical Immunology (4th ed.), Lange Medical Publications, Los Altos, CA, and references cited therein; Harlow and Lane (1988) Antibodies: A Laboratory Manual, CSH Press; Goding (1986) Monoclonal Antibodies: Principles and Practice (2d ed) Academic Press, New York; and particularly in Kohler and Milstein (1975) in Nature 256: 495-497, which discusses one method of generating monoclonal antibodies. Each of these references is incorporated herein by reference. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 246:1275-1281; and Ward, et al. (1989) Nature 341:544-546, each of which is hereby incorporated herein by reference. The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents, teaching the use of such labels include U.S. Patent Nos. 3,817,837; 3,850,752; 3,939,350; 3,996,345; 4,277,437; 4,275,149; and 4,366,241. Also, recombinant or chimeric immunoglobulins may be produced, see Cabilly, U.S. Patent No. 4,816,567; or made in transgenic mice, see Mendez, et al. (1997) Nature Genetics 15:146-156. These references are incorporated herein by reference.

The antibodies of this invention can also be used for affinity chromatography in isolating the DTLRs. Columns can be prepared where the antibodies are linked to a solid support, e.g., particles, such as agarose, Sephadex, or the like, where a cell lysate may be passed through the column, the column washed, followed by increasing concentrations of a mild denaturant, whereby the purified protein will be released. The protein may be used to purify antibody.

The antibodies may also be used to screen expression libraries for particular expression products. Usually the antibodies used in such a procedure will be labeled with a moiety allowing easy detection of presence of antigen by antibody binding.

Antibodies raised against a DTLR will also be used to raise anti-idiotypic antibodies. These will be useful in detecting or diagnosing various immunological conditions related to expression of the protein or cells which express the protein. They also will be useful as agonists or antagonists of the ligand, which may be competitive inhibitors or substitutes for naturally occurring ligands.

A DTLR protein that specifically binds to or that is specifically immunoreactive with an antibody generated against a defined immunogen, such as an immunogen consisting of the amino acid sequence of SEQ ID NO: 12 is typically determined in an immunoassay. The immunoassay typically uses a polyclonal antiserum which was raised, e.g., to a protein of SEQ ID NO: 12. This antiserum is selected to have low crossreactivity against other IL-1R family members, e.g., DTLR1, preferably from the same species, and any such crossreactivity is removed by immunoabsorption prior to use in the immunoassay.

In order to produce antisera for use in an immunoassay, the protein of SEQ ID NO: 12, is isolated as described herein. For example, recombinant protein may be produced in a mammalian cell line. An appropriate host, e.g., an inbred strain of mice such as balb/c, is immunized with the selected protein, typically using a standard adjuvant, such as Freund's adjuvant, and a standard mouse immunization protocol (see Harlow and Lane, supra). Alternatively, a synthetic peptide derived from the sequences disclosed herein and conjugated to a carrier protein can be used an immunogen. Polyclonal sera are collected and titered against the immunogen protein in an immunoassay, e.g., a solid phase immunoassay with the immunogen immobilized on a solid support. Polyclonal antisera with a titer of 10⁴ or greater are selected and tested for their cross reactivity against other IL-1R family members, e.g., mouse DTLRs or human DTLR1, using a competitive binding immunoassay such as the one described in Harlow and Lane, supra, at pages 570-573. Preferably at least two DTLR family members are used in this determination in conjunction with the human DTLR6. These IL-1R family members can be produced as recombinant proteins and isolated using standard molecular biology and protein chemistry techniques as described herein.

Immunoassays in the competitive binding format can be used for the crossreactivity determinations. For example, the proteins of SEQ ID NO: 12 or various fragments thereof, can be immobilized to a solid support. Proteins added to the assay compete with the binding of the antisera to the immobilized antigen. The ability of the above proteins to compete with the binding of the antisera to the immobilized protein is compared to the protein of SEQ ID NO: 12. The percent crossreactivity for the above proteins is calculated, using standard calculations. Those antisera with less than 10% crossreactivity with each of the proteins listed above are selected and pooled. The cross-reacting antibodies are then removed from the pooled antisera by immunoabsorbtion with the above-listed proteins.

The immunoabsorbed and pooled antisera are then used in a competitive binding immunoassay as described above to compare a second protein to the immunogen protein (e.g., the IL-1R like protein of SEQ ID NO: 12). In order to make this comparison, the two proteins are each assayed at a wide range of concentrations and the amount of each protein required to inhibit 50% of the binding of the antisera to the immobilized protein is determined. If the amount of the second protein required is less than twice the amount of the protein of the selected protein or proteins that is required, then the second protein is said to specifically bind to an antibody generated to the immunogen.

It is understood that these DTLR proteins are members of a family of homologous proteins that comprise at least 10 so far identified genes. For a particular gene product, such as the DTLR2-10, the term refers not only to the amino acid sequences disclosed herein, but also to other proteins that are allelic, non-allelic or species variants. It also understood that the terms include nonnatural mutations introduced by deliberate mutation using conventional recombinant technology such as single site mutation, or by excising short sections of DNA encoding the respective proteins, or by substituting new amino acids, or adding new amino acids. Such minor alterations must substantially maintain the immunoidentity of the original molecule and/or its biological activity. Thus, these alterations include proteins that are specifically immunoreactive with a designated naturally occurring IL-1R related protein, for example, the DTLR proteins shown in SEQ ID NO: 12. The biological properties of the altered proteins can be determined by expressing the protein in an appropriate cell line and measuring the appropriate effect upon lymphocytes. Particular protein modifications considered minor would include conservative substitution of amino acids with similar chemical properties, as described above for the IL-1R family as a whole. By aligning a protein optimally with the protein of DTLR2-10 and by using the conventional immunoassays described herein to determine immunoidentity, one can determine the protein compositions of the invention.

### VII. Kits and quantitation

Both naturally occurring and recombinant forms of the IL-1R like molecules of this invention are particularly useful in kits and assay methods. For example, these methods would also be applied to screening for binding activity, e.g., ligands for these proteins. Several methods of automating assays have been developed in recent years so as to permit screening of tens of thousands of compounds per year. See, e.g, a BIOMEK automated workstation, Beckman Instruments, Palo Alto, California, and Fodor, et al. (1991) Science 251:767-773, which is incorporated herein by reference. The latter describes means for testing binding by a plurality of defined polymers synthesized on a solid substrate. The development of suitable assays to screen for a ligand or agonist/antagonist homologous proteins can be greatly facilitated by the availability of large amounts of purified, soluble DTLRs in an active state such as is provided by this invention.

Purified DTLR can be coated directly onto plates for use in the aforementioned ligand screening techniques. However, non-neutralizing antibodies to these proteins can be used as capture antibodies to immobilize the respective receptor on the solid phase, useful, e.g., in diagnostic uses.

This invention also contemplates use of DTLR6, fragments thereof, peptides, and their fusion products in a variety of diagnostic kits and methods for detecting the presence of the protein or its ligand.
Alternatively, or additionally, antibodies against the molecules may be incorporated into the kits and methods. Typically the kit will have a compartment containing either a defined DTLR peptide or gene segment or a reagent which recognizes one or the other. Typically, recognition reagents, in the case of peptide, would be a receptor or antibody, or in the case of a gene segment, would usually be a hybridization probe.

A preferred kit for determining the concentration of DTLR6, a sample would typically comprise a labeled compound, e.g., ligand or antibody, having known binding affinity for DTLR6, source of DTLR6 (naturally occurring or recombinant) as a positive control, and a means for separating the bound from free labeled compound, for example a solid phase for immobilizing the DTLR4 in the test sample. Compartments containing reagents, and instructions, will normally be provided.

Antibodies, including antigen binding fragments, specific for mammalian DTLR or a peptide fragment, or receptor fragments are useful in diagnostic applications to detect the presence of elevated levels of ligand and/or its fragments. Diagnostic assays may be homogeneous (without a separation step between free reagent and antibody-antigen complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA) and the like. For example, unlabeled antibodies can be employed by using a second antibody which is labeled and which recognizes the antibody to DTLR6 or to a particular fragment thereof. These assays have also been extensively discussed in the literature. See, e.g., Harlow and Lane (1988) Antibodies: A Laboratory Manual, CSH., and Coligan (Ed.) (1991) and periodic supplements, Current Protocols In Immunology Greene/Wiley, New York.

Anti-idiotypic antibodies may have similar use to serve as agonists or antagonists of DTLR4. These should be useful as therapeutic reagents under appropriate circumstances.

Frequently, the reagents for diagnostic assays are supplied in kits, so as to optimize the sensitivity of the assay. For the subject invention, depending upon the nature of the assay, the protocol, and the label, either labeled or unlabeled antibody, or labeled ligand is provided. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent, and will contain instructions for proper use and disposal of reagents. Desirably, the reagents are provided as a dry lyophilized powder, where the reagents may be reconstituted in an aqueous medium having appropriate concentrations for performing the assay.

The aforementioned constituents of the diagnostic assays may be used without modification or may be modified in a variety of ways. For example, labeling may be achieved by covalently or non-covalently joining a moiety which directly or indirectly provides a detectable signal. In any of these assays, a test compound, DTLR, or antibodies thereto can be labeled either directly or indirectly. Possibilities for direct labeling include label groups: radiolabels such as ¹²⁵I, enzymes (U.S. Pat. No. 3,645,090) such as peroxidase and alkaline phosphatase, and fluorescent labels (U.S. Pat. No. 3,940,475) capable of monitoring the change in fluorescence intensity, wavelength shift, or fluorescence polarization. Both of the patents are incorporated herein by reference. Possibilities for indirect labeling include biotinylation of one constituent followed by binding to avidin coupled to one of the above label groups.

There are also numerous methods of separating the bound from the free ligand, or alternatively the bound from the free test compound. The DTLR can be immobilized on various matrixes followed by washing. Suitable matrices include plastic such as an ELISA plate, filters, and beads. Methods of immobilizing the receptor to a matrix include, without limitation, direct adhesion to plastic, use of a capture antibody, chemical coupling, and biotin-avidin. The last step in this approach involves the precipitation of antibody/antigen complex by any of several methods including those utilizing, e.g., an organic solvent such as polyethylene glycol or a salt such as ammonium sulfate. Other suitable separation techniques include, without limitation, the fluorescein antibody magnetizable particle method described in Rattle, et al. (1984) Clin. Chem. 30(9):1457-1461, and the double antibody magnetic particle separation as described in U.S. Pat. No. 4,659,678, each of which is incorporated herein by reference.

The methods for linking protein or fragments to various labels have been extensively reported in the literature and do not require detailed discussion here. Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like. Fusion proteins will also find use in these applications.

Another diagnostic aspect of this invention involves use of oligonucleotide or polynucleotide sequences taken from the sequence of a DTLR. These sequences can be used as probes for detecting levels of the respective DTLR in patients suspected of having an immulogoical disorder. The preparation of both RNA and DNA nucleotide sequences, the labeling of the sequences, and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly ³²P. However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes, DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel anti-sense RNA may be carried out in any conventional techniques such as nucleic acid hybridization, plus and minus screening, recombinational probing, hybrid released translation (HRT), and hybrid arrested translation (HART). This also includes amplification techniques such as polymerase chain reaction (PCR).

Diagnostic kits which also test for the qualitative or quantitative presence of other markers are also contemplated. Diagnosis or prognosis may depend on the combination of multiple indications used as markers. Thus, kits may test for combinations of markers. See, e.g., Viallet, et al. (1989) Progress in Growth Factor Res. 1:89-97.

### VIII. Therapeutic Utility

This invention provides reagents with significant therapeutic value. The DTLRs (naturally occurring or recombinant), fragments thereof, mutein receptors, and antibodies, along with compounds identified as having binding affinity to the receptors or antibodies, should be useful in the treatment of conditions exhibiting abnormal expression of the receptors of their ligands. Such abnormality will typically be manifested by immunological disorders. Additionally, this invention should provide therapeutic value in various diseases or disorders associated with abnormal expression or abnormal triggering of response to the ligand. The Toll ligands have been suggested to be involved in morphologic development, e.g., dorso-ventral polarity determination, and immune responses, particularly the primitive innate responses. See, e.g., Sun, et al. (1991) Eur. J. Biochem. 196:247-254; Hultmark (1994) Nature 367:116-117.

Recombinant DTLRs, muteins, agonist or antagonist antibodies thereto, or antibodies can be purified and then administered to a patient. These reagents can be combined for therapeutic use with additional active ingredients, e.g., in conventional pharmaceutically acceptable carriers or diluents, along with physiologically innocuous stabilizers and excipients. These combinations can be sterile, e.g., filtered, and placed into dosage forms as by lyophilization in dosage vials or storage in stabilized aqueous preparations. This invention also contemplates use of antibodies or binding fragments thereof which are not complement binding.

Ligand screening using DTLR or fragments thereof can be performed to identify molecules having binding affinity to the receptors. Subsequent biological assays can then be utilized to determine if a putative ligand can provide competitive binding, which can block intrinsic stimulating activity. Receptor fragments can be used as a blocker or antagonist in that it blocks the activity of ligand. Likewise, a compound having intrinsic stimulating activity can activate the receptor and is thus an agonist in that it simulates the activity of ligand, e.g., inducing signaling. This invention further contemplates the therapeutic use of antibodies to DTLRs as antagonists.

The quantities of reagents necessary for effective therapy will depend upon many different factors, including means of administration, target site, physiological state of the patient, and other medicants administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds) (1990) Goodman and Gilman's: The Pharmacological Bases of Therapeutics, 8th Ed., Pergamon Press; and Remington's Pharmaceutical Sciences, (current edition), Mack Publishing Co., Easton, Penn.; each of which is hereby incorporated herein by reference. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Because of the likely high affinity binding, or turnover numbers, between a putative ligand and its receptors, low dosages of these reagents would be initially expected to be effective. And the signaling pathway suggests extremely low amounts of ligand may have effect. Thus, dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or slow release apparatus will often be utilized for continuous administration.

DTLRs, fragments thereof, and antibodies or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in any conventional dosage formulation. While it is possible for the active ingredient to be administered alone, it is preferable to present it as a pharmaceutical formulation. Formulations comprise at least one active ingredient, as defined above, together with one or more acceptable carriers thereof. Each carrier must be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds) (1990) Goodman and Gilman's: The Pharmacological Bases of Therapeutics, 8th Ed., Pergamon Press; and Remington's Pharmaceutical Sciences (current edition), Mack Publishing Co., Easton, Penn.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. The therapy of this invention may be combined with or used in association with other therapeutic agents, particularly agonists or antagonists of other IL-1 family members.

### IX. Ligands

The description of the Toll receptors herein provide means to identify ligands, as described above. Such ligand should bind specifically to the respective receptor with reasonably high affinity. Various constructs are made available which allow either labeling of the receptor to detect its ligand. For example, directly labeling DTLR, fusing onto it markers for secondary labeling, e.g., FLAG or other epitope tags, etc., will allow detection of receptor. This can be histological, as an affinity method for biochemical purification, or labeling or selection in an expression cloning approach. A two-hybrid selection system may also be applied making appropriate constructs with the available DTLR sequences. See, e.g., Fields and Song (1989) Nature 340:245-246.

Generally, descriptions of DTLRs will be analogously applicable to individual specific embodiments directed to DTLR6, reagents and compositions.

The broad scope of this invention is best understood with reference to the following examples, which are not intended to limit the inventions to the specific embodiments.

### EXAMPLES

### I. General Methods

Some of the standard methods are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols 1-3, CSH Press, NY; Ausubel, et al., Biology, Greene Publishing Associates, Brooklyn, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. See, e.g., Ausubel, et al. (1987 and periodic supplements); Coligan, et al. (ed. 1996) and periodic supplements, Current Protocols In Protein Science Greene/Wiley, New York; Deutscher (1990) "Guide to Protein Purification" in Methods in Enzymology, vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1989) Chemische Industrie 12:69-70; Hochuli (1990) "Purification of Recombinant Proteins with Metal Chelate Absorbent" in Setlow (ed.) Genetic Engineering, Principle and Methods 12:87-98, Plenum Press, N.Y.; and Crowe, et al. (1992) OIAexpress: The High Level Expression & Protein Purification System QUIAGEN, Inc., Chatsworth, CA.

Standard immunological techniques and assays are described, e.g., in Hertzenberg, et al. (eds. 1996) Weir's Handbook of Experimental Immunology vols. 1-4, Blackwell Science; Coligan (1991) Current Protocols in Immunology Wiley/Greene, NY; and Methods in Enzymology volumes. 70, 73, 74, 84, 92, 93, 108, 116, 121, 132, 150, 162, and 163.

Assays for vascular biological activities are well known in the art. They will cover angiogenic and angiostatic activities in tumor, or other tissues, e.g., arterial smooth muscle proliferation (see, e.g., Koyoma, et al. (1996) Cell 87:1069-1078), monocyte adhesion to vascular epithelium (see McEvoy, et al. (1997) J. Exp. Med. 185:2069-2077), etc. See also Ross (1993) Nature 362:801-809; Rekhter and Gordon (1995) Am. J. Pathol. 147:668-677; Thyberg, et al. (1990) Atherosclerosis 10:966-990; and Gumbiner (1996) Cell 84:345-357.

Assays for neural cell biological activities are described, e.g., in Wouterlood (ed. 1995) Neuroscience Protocols modules 10, Elsevier; Methods in Neurosciences Academic Press; and Neuromethods Humana Press, Totowa, NJ. Methodology of developmental systems is described, e.g., in Meisami (ed.) Handbook of Human Growth and Developmental Biology CRC Press; and Chrispeels (ed.) Molecular Techniques and Approaches in Developmental Biology Interscience.

Computer sequence analysis is performed, e.g., using available software programs, including those from the GCG (U. Wisconsin) and GenBank sources. Public sequence databases were also used, e.g., from GenBank, NCBI, EMBO, and others.

Many techniques applicable to IL-10 receptors may be applied to DTLRs, as described, e.g., in USSN 08/110,683 (IL-10 receptor), which is incorporated herein by reference for all purposes.

### II. Novel Family of Human Receptors

Abbreviations: DTLR, Toll-like receptor; IL-1R, interleukin-1 receptor; TH, Toll homology; LRR, leucine-rich repeat; EST, expressed sequence tag; STS, sequence tagged site; FISH, fluoresence in situ hybridization.

The discovery of sequence homology between the cytoplasmic domains of Drosophila Toll and human interleukin-1 (IL-1) receptors has sown the conviction that both molecules trigger related signaling pathways tied to the nuclear translocation of Rel-type transcription factors. This conserved signaling scheme governs an evolutionarily ancient immune response in both insects and vertebrates. We report the molecular cloning of a novel class of putative human receptors with a protein architecture that is closely similar to Drosophila Toll in both intra- and extra-cellular segments. Five human Toll-like receptors, designated DTLRs 1-5, are likely the direct homologs of the fly molecule, and as such could constitute an important and unrecognized component of innate immunity in humans; intriguingly, the evolutionary retention of DTLRs in vertebrates may indicate another role, akin to Toll in the dorso-ventralization of the Drosophila embryo, as regulators of early morphogenetic patterning. Multiple tissue mRNA blots indicate markedly different patterns of expression for the human DTLRs. Using fluorescence in situ hybridization and Sequence-Tagged Site database analyses, we also show that the cognate DTLR genes reside on chromosomes 4 (DTLRs 1, 2, and 3), 9 (DTLR4), and 1 (DTLR5). Structure prediction of the aligned Toll-homology (TH) domains from varied insect and human DTLRs, vertebrate IL-1 receptors, and MyD88 factors, and plant disease resistance proteins, recognizes a parallel β/α fold with an acidic active site; a similar structure notably recurs in a class of response regulators broadly involved in transducing sensory information in bacteria.

The seeds of the morphogenetic gulf that so dramatically separates flies from humans are planted in familiar embryonic shapes and patterns, but give rise to very different cell complexities. DeRobertis and Sasai (1996) Nature 380:37-40; and Arendt and Nübler-Jung (1997) Mech. Develop. 61:7-21. This divergence of developmental plans between insects and vertebrates is choreographed by remarkably similar signaling pathways, underscoring a greater conservation of protein networks and biochemical mechanisms from unequal gene repertoires. Miklos and Rubin (1996) Cell 86:521-529; and Chothia (1994) Develop. 1994 Suppl., 27-33. A powerful way to chart the evolutionary design of these regulatory pathways is by inferring their likely molecular components (and biological functions) through interspecies comparisons of protein sequences and structures. Miklos and Rubin (1996) Cell 86:521-529; Chothia (1994) Develop. 1994 Suppl., 27-33 (3-5); and Banfi, et al. (1996) Nature Genet. 13:167-174.

A universally critical step in embryonic development is the specification of body axes, either born from innate asymmetries or triggered by external cues. DeRobertis and Sasai (1996) Nature 380:37-40; and Arendt and Nubler-Jung (1997) Mech. Develop. 61:7-21. As a model system, particular attention has been focused on the phylogenetic basis and cellular mechanisms of dorsoventral polarization. DeRobertis and Sasai (1996) Nature 380:37-40; and Arendt and Nübler-Jung (1997) Mech. Develop. 61:7-21. A prototype molecular strategy for this transformation has emerged from the Drosophila embryo, where the sequential action of a small number of genes results in a ventralizing gradient of the transcription factor Dorsal. St. Johnston and Nüsslein-Volhard (1992) Cell 68:201-219; and Morisato and Anderson (1995) Ann. Rev. Genet. 29:371-399.

This signaling pathway centers on Toll, a transmembrane receptor that transduces the binding of a maternally-secreted ventral factor, Spätzle, into the cytoplasmic engagement of Tube, an accessory molecule, and the activation of Pelle, a Ser/Thr kinase that catalyzes the dissociation of Dorsal from the inhibitor Cactus and allows migration of Dorsal to ventral nuclei (Morisato and Anderson (1995) Ann. Rev. Genet. 29:371-399; and Belvin and Anderson (1996) Ann. Rev. Cell Develop. Biol. 12:393-416. The Toll pathway also controls the induction of potent antimicrobial factors in the adult fly (Lemaitre, et al. (1996) Cell 86:973-983); this role in Drosophila immune defense strengthens mechanistic parallels to IL-1 pathways that govern a host of immune and inflammatory responses in vertebrates. Belvin and Anderson (1996) Ann. Rev. Cell Develop. Biol. 12:393-416; and Wasserman (1993) Molec. Biol, Cell 4:767-771. A Toll-related cytoplasmic domain in IL-1 receptors directs the binding of a Pelle-like kinase, IRAK, and the activation of a latent NF-κB/I-κB complex that mirrors the embrace of Dorsal and Cactus. Belvin and Anderson (1996) Ann. Rev. Cell Develop. Biol. 12:393-416; and Wasserman (1993) Molec. Biol. Cell 4:767-771.

We describe the cloning and molecular characterization of four new Toll-like molecules in humans, designated DTLRs 2-5 (following Chiang & Beachy (1994) Mech. Develop. 47:225-239), that reveal a receptor family more closely tied to Drosophila Toll homologs than to vertebrate IL-1 receptors. The DTLR sequences are derived from human ESTs; these partial cDNAs were used to draw complete expression profiles in human tissues for the five DTLRs, map the chromosomal locations of cognate genes, and narrow the choice of cDNA libraries for full-length cDNA retrievals. Spurred by other efforts (Banfi, et al. (1996) Nature Genet. 13:167-174; and Wang, et al. (1996) J. Biol. Chem. 271:4468-4476), we are assembling, by structural conservation and molecular parsimony, a biological system in humans that is the counterpart of a compelling regulatory scheme in Drosophila. In addition, a biochemical mechanism driving Toll signaling is suggested by the proposed tertiary fold of the Toll-homology (TH) domain, a core module shared by DTLRs, a broad family of IL-1 receptors, mammalian MyD88 factors and plant disease resistance proteins. Mitcham, et al. (1996) J. Biol. Chem. 271:5777-5783; and Hardiman, et al. (1996) Oncogene 13:2467-2475. We propose that a signaling route coupling morphogenesis and primitive immunity in insects, plants, and animals (Belvin and Anderson (1996) Ann. Rev. Cell Develop. Biol. 12:393-416; and Wilson, et al. (1997) Curr. Biol. 7:175-178) may have roots in bacterial two-component pathways.

### Computational Analysis.

Human sequences related to insect DTLRs were identified from the EST database (dbEST) at the National Center for Biotechnology Information (NCBI) using the BLAST server (Altschul, et al. (1994) Nature Genet. 6:119-129). More sensitive pattern- and profile-based methods (Bork and Gibson (1996) Meth. Enzymol. 266:162-184) were used to isolate the signaling domains of the DTLR family that are shared with vertebrate and plant proteins present in nonredundant databases. The progressive alignment of DTLR intra- or extracellular domain sequences was carried out by ClustalW (Thompson, et al. (1994) Nucleic Acids Res. 22:4673-4680); this program also calculated the branching order of aligned sequences by the Neighbor-Joining algorithm (5000 bootstrap replications provided confidence values for the tree groupings).

Conserved alignment patterns, discerned at several degrees of stringency, were drawn by the Consensus program (internet URL http://www.bork.emblheidelberg.de/Alignment/ consensus.html). The PRINTS library of protein fingerprints (http://www.biochem.ucl.ac.uk/bsm/dbbrowser/PRINTS/ PRINTS.html) (Attwood, et al. (1997) Nucleic Acids Res. 25:212-217) reliably identified the myriad leucine-rich repeats (LRRs) present in the extracellular segments of DTLRs with a compound motif (PRINTS code Leurichrpt) that flexibly matches N- and C-terminal features of divergent LRRs. Two prediction algorithms whose three-state accuracy is above 72% were used to derive a consensus secondary structure for the intracellular domain alignment, as a bridge to fold recognition efforts (Fischer, et al. (1996) FASEB J. 10:126-136). Both the neural network program PHD (Rost and Sander (1994) Proteins 19:55-72) and the statistical prediction method DSC (King and Sternberg (1996) Protein Sci. 5:2298-2310) have internet servers (URLs http://www.emblheidelberg.de/ predictprotein/phd_pred.html and http://bonsai.lif.icnet.uk/bmm/dsc/dsc_read_align.html, respectively). The intracellular region encodes the THD region discussed, e.g., in Hardiman, et al. (1996) Oncogene 13:2467-2475; and Rock, et al. (1998) Proc. Nat'l Acad. Sci. USA 95:588-593, each of which is incorporated herein by reference. This domain is very important in the mechanism of signaling by the receptors, which transfers a phosphate group to a substrate.

### Cloning of full-length human DTLR cDNAs.

PCR primers derived from the Toll-like Humrsc786 sequence (Genbank accession code D13637) (Nomura, et al. (1994) DNA Res 1:27-35) were used to probe a human erythroleukemic, TF-1 cell line-derived cDNA library (Kitamura, et al. (1989) Blood 73:375-380) to yield the DTLR1 cDNA sequence. The remaining DTLR sequences were flagged from dbEST, and the relevant EST clones obtained from the I.M.A.G.E. consortium (Lennon, et al. (1996) Genomics 33:151-152) via Research Genetics (Huntsville, AL): CloneID#'s 80633 and 117262 (DTLR2), 144675 (DTLR3), 202057 (DTLR4) and 277229 (DTLR5). Full length cDNAs for human DTLRs 2-4 were cloned by DNA hybridization screening of λgt10 phage, human adult lung, placenta, and fetal liver 5'-Stretch Plus cDNA libraries (Clontech), respectively; the DTLR5 sequence is derived from a human multiple-sclerosis plaque EST. All positive clones were sequenced and aligned to identify individual DTLR ORFs: DTLR1 (2366 bp clone, 786 aa ORF), DTLR2 (2600 bp, 784 aa), DTLR3 (3029 bp, 904 aa), DTLR4 (3811 bp, 879 aa) and DTLR5 (1275 bp, 370 aa). Probes for DTLR3 and DTLR4 hybridizations were generated by PCR using human placenta (Stratagene) and adult liver (Clontech) cDNA libraries as templates, respectively; primer pairs were derived from the respective EST sequences. PCR reactions were conducted using T. aquaticus Taqplus DNA polymerase (Stratagene) under the following conditions: 1 x (94° C, 2 min) 30 x (55° C, 20 sec; 72° C 30 sec; 94° C 20 sec), 1 x (72° C, 8 min). For DTLR2 full-length cDNA screening, a 900 bp fragment generated by EcoRI/XbaI digestion of the first EST clone (ID# 80633) was used as a probe.

### mRNA blots and chromosomal localization.

Human multiple tissue (Cat# 1, 2) and cancer cell line blots (Cat# 7757-1), containing approximately 2 µg of poly(A)⁺ RNA per lane, were purchased from Clontech (Palo Alto, CA). For DTLRs 1-4, the isolated full-length cDNAs served as probes, for DTLR5 the EST clone (ID #277229) plasmid insert was used. Briefly, the probes were radiolabeled with [α-³²P] dATP using the Amersham Rediprime random primer labeling kit (RPN1633). Prehybridization and hybridizations were performed at 65° C in 0.5 M Na₂HPO₄, 7% SDS, 0.5 M EDTA (pH 8.0). All stringency washes were conducted at 65° C with two initial washes in 2 x SSC, 0.1% SDS for 40 min followed by a subsequent wash in 0.1 x SSC, 0.1% SDS for 20 min. Membranes were then exposed at -70° C to X-Ray film (Kodak) in the presence of intensifying screens. More detailed studies by cDNA library Southerns (14) were performed with selected human DTLR clones to examine their expression in hemopoietic cell subsets.

Human chromosomal mapping was conducted by the method of fluorescence in situ hybridization (FISH) as described in Heng and Tsui (1994) Meth. Molec. Biol. 33:109-122, using the various full-length (DTLRs 2-4) or partial (DTLR5) cDNA clones as probes. These analyses were performed as a service by SeeDNA Biotech Inc. (Ontario, Canada). A search for human syndromes (or mouse defects in syntenic loci) associated with the mapped DTLR genes was conducted in the Dysmorphic Human-Mouse Homology Database by internet server (http://www.hgmp.mrc.ac.uk/DHMHD/ hum_chromel.html).

### Conserved architecture of insect and human DTLR ectodomains.

The Toll family in Drosophila comprises at least four distinct gene products: Toll, the prototype receptor involved in dorsoventral patterning of the fly embryo (Morisato and Anderson (1995) Ann. Rev. Genet. 29:371-399) and a second named '18 Wheeler' (18w) that may also be involved in early embryonic development (Chiang and Beachy (1994) Mech. Develop. 47:225-239; Eldon, et al. (1994) Develop. 120:885-899); two additional receptors are predicted by incomplete, Toll-like ORFs downstream of the male-specific-transcript (Mst) locus (Genbank code X67703) or encoded by the 'sequence-tagged-site' (STS) Dm2245 (Genbank code G01378) (Mitcham, et al. (1996) J. Biol. Chem. 271:5777-5783). The extracellular segments of Toll and 18w are distinctively composed of imperfect, ~24 amino acid LRR motifs (Chiang and Beachy (1994) Mech. Develop. 47:225-239; and Eldon, et al. (1994) Develop. 120:885-899). Similar tandem arrays of LRRs commonly form the adhesive antennae of varied cell surface molecules and their generic tertiary structure is presumed to mimic the horseshoe-shaped cradle of a ribonuclease inhibitor fold, where seventeen LRRs show a repeating β/α-hairpin, 28 residue motif (Buchanan and Gay (1996) Prog. Biophys. Molec. Biol. 65:1-44). The specific recognition of Spätzle by Toll may follow a model proposed for the binding of cystine-knot fold glycoprotein hormones by the multi-LRR ectodomains of serpentine receptors, using the concave side of the curved β-sheet (Kajava, et al. (1995) Structure 3:867-877); intriguingly, the pattern of cysteines in Spätzle, and an orphan Drosophila ligand, Trunk, predict a similar cystine-knot tertiary structure (Belvin and Anderson (1996) Ann. Rev. Cell Develop. Biol. 12:393-416; and Casanova, et al. (1995) Genes Develop. 9:2539-2544).

The 22 and 31 LRR ectodomains of Toll and 18w, respectively (the Mst ORF fragment displays 16 LRRs), are most closely related to the comparable 18, 19, 24, and 22 LRR arrays of DTLRs 1-4 (the incomplete DTLR5 chain presently includes four membrane-proximal LRRs) by sequence and pattern analysis (Altschul, et al. (1994) Nature Genet. 6:119-129; and Bork and Gibson (1996) Meth. Enzymol. 266:162-184) (Fig. 1). However, a striking difference in the human DTLR chains is the common loss of a ~90 residue cysteine-rich region that is variably embedded in the ectodomains of Toll, 18w and the Mst ORF (distanced four, six and two LRRs, respectively, from the membrane boundary). These cysteine clusters are bipartite, with distinct 'top' (ending an LRR) and 'bottom' (stacked atop an LRR) halves (Chiang and Beachy (1994) Mech. Develop. 47:225-239; Eldon, et al. (1994) Develop. 120:885-899; and ,Buchanan and Gay (1996) Prog. Biophys. Molec. Biol. 65:1-44); the 'top' module recurs in both Drosophila and human DTLRs as a conserved juxtamembrane spacer (Fig. 1). We suggest that the flexibly located cysteine clusters in Drosophila receptors (and other LRR proteins), when mated 'top' to 'bottom', form a compact module with paired termini that can be inserted between any pair of LRRs without altering the overall fold of DTLR ectodomains; analogous 'extruded' domains decorate the structures of other proteins (Russell (1994) Protein Engin. 7:1407-1410).

### Molecular design of the TH signaling domain.

Sequence comparison of Toll and IL-1 type-I (IL-1R1) receptors has disclosed a distant resemblance of a ~200 amino acid cytoplasmic domain that presumably mediates signaling by similar Rel-type transcription factors. Belvin and Anderson (1996) Ann. Rev. Cell Develop. Biol. 12:393-416; and (Belvin and Anderson (1996) Ann. Rev. Cell Develop. Biol. 12:393-416; and Wasserman (1993) Molec. Biol. Cell 4:767-771). More recent additions to this functional paradigm include a pair of plant disease resistance proteins from tobacco and flax that feature an N-terminal TH module followed by nucleotide-binding (NTPase) and LRR segments (Wilson, et al. (1997) Curr. Biol. 7:175-178); by contrast, a 'death domain' preceeds the TH chain of MyD88, an intracellular myeloid differentiation marker (Mitcham, et al. (1996) J. Biol. Chem. 271:5777-5783; and Hardiman, et al. (1996) Oncogene 13:2467-2475) (Fig. 1). New IL-1-type receptors include IL-1R3, an accessory signaling molecule, and orphan receptors IL-1R4 (also called ST2/Fit-1/T1), IL-1R5 (IL-1R-related protein), and IL-1R6 (IL-1R-related protein-2) (Mitcham, et al. (1996) J. Biol. Chem. 271:5777-5783;Hardiman, et al. (1996) Oncogene 13:2467-2475). With the new human DTLR sequences, we have sought a structural definition of this evolutionary thread by analyzing the conformation of the common TH module: ten blocks of conserved sequence comprising 128 amino acids form the minimal TH domain fold; gaps in the alignment mark the likely location of sequence and length-variable loops (Fig. 2a).

Two prediction algorithms that take advantage of the patterns of conservation and variation in multiply aligned sequences, PHD (Rost and Sander (1994) Proteins 19:55-72) and DSC (King and Sternberg (1996) Protein Sci. 5:2298-2310), produced strong, concordant results for the TH signaling module (Fig. 2a). Each block contains a discrete secondary structural element: the imprint of alternating β-strands (labeled A-E) and α-helices (numbered 1-5) is diagnostic of an β/α-class fold with α-helices on both faces of a parallel β-sheet. Hydrophobic β-strands A, C and D are predicted to form 'interior' staves in the β-sheet, while the shorter, amphipathic β-strands B and E resemble typical 'edge' units (Fig. 2a). This assignment is consistent with a strand order of B-A-C-D-E in the core β-sheet (Fig. 2b); fold comparison ('mapping') and recognition ('threading') programs (Fischer, et al. (1996) FASEB J. 10:126-136) strongly return this doubly wound β/α topology. A surprising, functional prediction of this outline structure for the TH domain is that many of the conserved, charged residues in the multiple alignment map to the C-terminal end of the β-sheet: residue Asp16 (block numbering scheme - Fig. 2a) at the end of βA, Arg39 and Asp40 following βB, Glu75 in the first turn of α3, and the more loosely conserved Glu/Asp residues in the βD-α4 loop, or after βE (Fig. 2a). The location of four other conserved residues (Asp7, Glu28, and the Arg57-Arg/Lys58 pair) is compatible with a salt bridge network at the opposite, N-terminal end of the β-sheet (Fig. 2a).

Signaling function depends on the structural integrity of the TH domain. Inactivating mutations or deletions within the module boundaries (Fig. 2a) have been catalogued for IL-1R1 and Toll. Heguy, et al. (1992) J. Biol. Chem. 267:2605-2609; Croston, et al. (1995) J. Biol. Chem. 270:16514-16517; Schneider, et al. (1991) Genes Develop. 5:797-807; Norris and Manley. (1992) Genes Develop. 6:1654-1667; Norris and Manley (1995) Genes Develop. 9:358-369; and Norris and Manley (1996) Genes Develop. 10:862-872. The human DTLR1-5 chains extending past the minimal TH domain (8, 0, 6, 22 and 18 residue lengths, respectively) are most closely similar to the stubby, 4 aa 'tail' of the Mst ORF. Toll and 18w display unrelated 102 and 207 residue tails (Fig. 2a) that may negatively regulate the signaling of the fused TH domains. Norris and Manley (1995) Genes Develop. 9:358-369; and Norris and Manley (1996) Genes Develop. 10:862-872.

The evolutionary relationship between the disparate proteins that carry the TH domain can best be discerned by a phylogenetic tree derived from the multiple alignment (Fig. 3). Four principal branches segregate the plant proteins, the MyD88 factors, IL-1 receptors and Toll-like molecules; the latter branch clusters the Drosophila and human DTLRs.

### Chromosomal dispersal of human DTLR genes.

In order to investigate the genetic linkage of the nascent human DTLR gene family, we mapped the chromosomal loci of four of the five genes by FISH (Fig. 4). The DTLR1 gene has previously been charted by the human genome project: an STS database locus (dbSTS accession number G06709, corresponding to STS WI-7804 or SHGC-12827) exists for the Humrsc786 cDNA (Nomura, et al. (1994) DNA Res 1:27-35) and fixes the gene to chromosome 4 marker interval D4S1587-D42405 (50-56 cM) circa 4p14. This assignment has recently been corroborated by FISH analysis. Taguchi, et al. (1996) Genomics 32:486-488. In the present work, we reliably assign the remaining DTLR genes to loci on chromosome 4q32 (DTLR2), 4q35 (DTLR3), 9q32-33 (DTLR4) and 1q33.3 (DTLR5). During the course of this work, an STS for the parent DTLR2 EST (cloneID # 80633) has been generated (dbSTS accession number T57791 for STS SHGC-33147) and maps to the chromosome 4 marker interval D4S424-D4S1548 (143-153 cM) at 4q32 -in accord with our findings. There is a ~50 cM gap between DTLR2 and DTLR3 genes on the long arm of chromosome 4.

### DTLR genes are differentially expressed.

Both Toll and 18w have complex spatial and temporal patterns of expression in Drosophila that may point to functions beyond embryonic patterning. St. Johnston and Nüsslein-Volhard (1992) Cell 68:201-219; Morisato and Anderson (1995) Ann. Rev. Genet. 29:371-399; Belvin and Anderson (1996) Ann. Rev. Cell Develop. Biol. 12:393-416; Lemaitre, et al. (1996) Cell 86:973-983; Chiang and Beachy (1994) Mech. Develop. 47:225-239; and Eldon, et al. (1994) Develop. 120:885-899. We have examined the spatial distribution of DTLR transcripts by mRNA blot analysis with varied human tissue and cancer cell lines using radioabeled DTLR cDNAs (Fig. 5). DTLR1 is found to be ubiquitously expressed, and at higher levels than the other receptors. Presumably reflecting alternative splicing, 'short' 3.0 kB and 'long' 8.0 kB DTLR1 transcript forms are present in ovary and spleen, respectively (Fig. 5, panels A & B). A cancer cell mRNA panel also shows the prominent overexpression of DTLR1 in a Burkitt's Lymphoma Raji cell line (Fig. 5, panel C). DTLR2 mRNA is less widely expressed than DTLR1, with a 4.0 kB species detected in lung and a 4.4 kB transcript evident in heart, brain and muscle. The tissue distribution pattern of DTLR3 echoes that of DTLR2 (Fig. 5, panel E). DTLR3 is also present as two major transcripts of approximately 4.0 and 6.0 kB in size, and the highest levels of expression are observed in placenta and pancreas. By contrast, DTLR4 and DTLR5 messages appear to be extremely tissue-specific. DTLR4 was detected only in placenta as a single transcript of ~7.0 kB in size. A faint 4.0 kB signal was observed for DTLR5 in ovary and peripheral blood monocytes.

### Components of an evolutionarily ancient regulatory system.

The original molecular blueprints and divergent fates of signaling pathways can be reconstructed by comparative genomic approaches. Miklos and Rubin (1996) Cell 86:521-529; Chothia (1994) Develop. 1994 Suppl., 27-33; Banfi, et al. (1996) Nature Genet. 13:167-174; and Wang, et al. (1996) J. Biol. Chem. 271:4468-4476. We have used this logic to identify an emergent gene family in humans, encoding five receptor paralogs at present, DTLRs 1-5, that are the direct evolutionary counterparts of a Drosophila gene family headed by Toll (Figs. 1-3). The conserved architecture of human and fly DTLRs, conserved LRR ectodomains and intracellular TH modules (Fig. 1), intimates that the robust pathway coupled to Toll in Drosophila (6, 7) survives in vertebrates. The best evidence borrows from a reiterated pathway: the manifold IL-1 system and its repertoire of receptor-fused TH domains, IRAK, NF-κB and I-κB homologs (Belvin and Anderson (1996) Ann. Rev. Cell Develop. Biol. 12:393-416; Wasserman (1993) Molec. Biol. Cell 4:767-771; Hardiman, et al. (1996) Oncogene 13:2467-2475; and Cao, et al. (1996) Science 271:1128-1131); a Tube-like factor has also been characterized. It is not known whether DTLRs can productively couple to the IL-1R signaling machinery, or instead, a parallel set of proteins is used. Differently from IL-1 receptors, the LRR cradle of human DTLRs is predicted to retain an affinity for Spätzle/Trunk-related cystine-knot factors; candidate DTLR ligands (called PENs) that fit this mold have been isolated.

Biochemical mechanisms of signal transduction can be gauged by the conservation of interacting protein folds in a pathway. Miklos and Rubin (1996) Cell 86:521-529; Chothia (1994) Develop. 1994 Suppl., 27-33. At present, the Toll signaling paradigm involves some molecules whose roles are narrowly defined by their structures, actions or fates: Pelle is a Ser/Thr kinase (phosphorylation), Dorsal is an NF-κB-like transcription factor (DNA-binding) and Cactus is an ankyrin-repeat inhibitor (Dorsal binding, degradation). Belvin and Anderson (1996) Ann. Rev. Cell Develop. Biol. 12:393-416. By contrast, the functions of the Toll TH domain and Tube remain enigmatic. Like other cytokine receptors (Heldin (1995) Cell 80:213-223), ligand-mediated dimerization of Toll appears to be the triggering event: free cysteines in the juxtamembrane region of Toll create constitutively active receptor pairs (Schneider, et al. (1991) Genes Develop. 5:797-807), and chimeric Torso-Toll receptors signal as dimers (Galindo, et al. (1995) Develop. 121:2209-2218); yet, severe truncations or wholesale loss of the Toll ectodomain results in promiscuous intracellular signaling (Norris and Manley (1995) Genes Develop. 9:358-369; and Winans and Hashimoto (1995) Molec. Biol. Cell 6:587-596), reminiscent of oncogenic receptors with catalytic domains (Heldin (1995) Cell 80:213-223). Tube is membrane-localized, engages the N-terminal (death) domain of Pelle and is phosphorylated, but neither Toll-Tube or Toll-Pelle interactions are registered by two-hybrid analysis (Galindo, et al. (1995) Develop. 121:2209-2218; and Großhans, et al. (1994) Nature 372:563-566); this latter result suggests that the conformational 'state' of the Toll TH domain somehow affects factor recruitment. Norris and Manley (1996) Genes Develop. 10:862-872; and Galindo, et al. (1995) Develop. 121:2209-2218.

At the heart of these vexing issues is the structural nature of the Toll TH module. To address this question, we have taken advantage of the evolutionary diversity of TH sequences from insects, plants and vertebrates, incorporating the human DTLR chains, and extracted the minimal, conserved protein core for structure prediction and fold recognition (Fig. 2). The strongly predicted (β/α)₅ TH domain fold with its asymmetric cluster of acidic residues is topologically identical to the structures of response regulators in bacterial two-component signaling pathways (Volz (1993) Biochemistry 32:11741-11753; and Parkinson (1993) Cell 73:857-871) (Fig. 2). The prototype chemotaxis regulator CheY transiently binds a divalent cation in an 'aspartate pocket' at the C-end of the core β-sheet; this cation provides electrostatic stability and facilitates the activating phosphorylation of an invariant Asp. Volz (1993) Biochemistry 32:11741-11753. Likewise, the TH domain may capture cations in its acidic nest, but activation, and downstream signaling, could depend on the specific binding of a negatively charged moiety: anionic ligands can overcome intensely negative binding-site potentials by locking into precise hydrogen-bond networks. Ledvina, et al. (1996) Proc. Natl. Acad. Sci. USA 93:6786-6791. Intriguingly, the TH domain may not simply act as a passive scaffold for the assembly of a Tube/Pelle complex for Toll, or homologous systems in plants and vertebrates, but instead actively participate as a true conformational trigger in the signal transducing machinery. Perhaps explaining the conditional binding of a Tube/Pelle complex, Toll dimerization could promote unmasking, by regulatory receptor tails (Norris and Manley (1995) Genes Develop. 9:358-369; Norris and Manley (1996) Genes Develop. 10:862-872), or binding by small molecule activators of the TH pocket. However, 'free' TH modules inside the cell (Norris and Manley (1995) Genes Develop. 9:358-369; Winans and Hashimoto (1995) Molec. Biol. Cell 6:587-596) could act as catalytic, CheY-like triggers by activating and docking with errant Tube/Pelle complexes.

### Morphogenetic receptors and immune defense.

The evolutionary link between insect and vertebrate immune systems is stamped in DNA: genes encoding antimicrobial factors in insects display upstream motifs similar to acute phase response elements known to bind NF-κB transcription factors in mammals. Hultmark (1993) Trends Genet. 9:178-183. Dorsal, and two Dorsal-related factors, Dif and Relish, help induce these defense proteins after bacterial challenge (Reichhart, et al. (1993) C. R. Acad. Sci. Paris 316:1218-1224; Ip, et al. (1993) Cell 75:753-763; and Dushay, et al. (1996) Proc. Natl. Acad. Sci. USA 93:10343-10347); Toll, or other DTLRs, likely modulate these rapid immune responses in adult Drosophila (Lemaitre, et al. (1996) Cell 86:973-983; and Rosetto, et al. (1995) Biochem. Biophys. Res. Commun. 209:111-116). These mechanistic parallels to the IL-1 inflammatory response in vertebrates are evidence of the functional versatility of the Toll signaling pathway, and suggest an ancient synergy between embryonic patterning and innate immunity (Belvin and Anderson (1996) Ann. Rev. Cell Develop. Biol. 12:393-416; Lemaitre, et al. (1996) Cell 86:973-983; Wasserman (1993) Molec. Biol. Cell 4:767-771; Wilson, et al. (1997) Curr. Biol. 7:175-178; Hultmark (1993) Trends Genet. 9:178-183; Reichhart, et al. (1993) C. R. Acad. Sci. Paris 316:1218-1224; Ip, et al. (1993) Cell 75:753-763; Dushay, et al. (1996) Proc. Natl. Acad. Sci. USA 93:10343-10347; Rosetto, et al. (1995) Biochem. Biophys. Res. Commun. 209:111-116; Medzhitov and Janeway (1997) Curr. Opin. Immunol. 9:4-9; and Medzhitov and Janeway (1997) Curr. Opin. Immunol. 9:4-9). The closer homology of insect and human DTLR proteins invites an even stronger overlap of biological functions that supersedes the purely immune parallels to IL-1 systems, and lends potential molecular regulators to dorso-ventral and other transformations of vertebrate embryos. DeRobertis and Sasai (1996) Nature 380:37-40; and Arendt and Nübler-Jung (1997) Mech. Develop. 61:7-21.

The present description of an emergent, robust receptor family in humans mirrors the recent discovery of the vertebrate Frizzled receptors for Wnt patterning factors. Wang, et al. (1996) J. Biol. Chem. 271:4468-4476. As numerous other cytokine-receptor systems have roles in early development (Lemaire and Kodjabachian (1996) Trends Genet. 12:525-531), perhaps the distinct cellular contexts of compact embryos and gangly adults simply result in familiar signaling pathways and their diffusible triggers having different biological outcomes at different times, e.g., morphogenesis versus immune defense for DTLRs. For insect, plant, and human Toll-related systems (Hardiman, et al. (1996) Oncogene 13:2467-2475; Wilson, et al. (1997) Curr. Biol. 7:175-178), these signals course through a regulatory TH domain that intriguingly resembles a bacterial transducing engine (Parkinson (1993) Cell 73:857-871).

In particular, the DTLR6 exhibits structural features which establish its membership in the family. Moreover, members of the family have been implicated in a number of significant developmental disease conditions and with function of the innate immune system. In particular, the DTLR6 has been mapped to the X chromosome to a location which is a hot spot for major developmental abnormalities. See, e.g., The Sanger Center: human X chromosome website http://www.sanger.ac.uk/HGP/Chrx/index.shtml; and the Baylor College of Medicine Human Genome Sequencing website http://gc.bcm.tmc.edu:8088/cgi-bin/seq/home.

The accession number for the deposited PAC is AC003046. This accession number contains sequence from two PACs: RPC-164K3 and RPC-263P4. These two PAC sequences mapped on human chromosome Xp22 at the Baylor web site between STS markers DXS704 and DXS7166. This region is a "hot spot" for severe developmental abnormalities.

### III. Amplification of DTLR fragment by PCR

Two appropriate primer seqwuences are selected (see Tables 1 through 10). RT-PCR is used on an appropriate mRNA sample selected for the presence of message to produce a partial or full length cDNA, e.g., a sample which expresses the gene. See, e.g., Innis, et al. (eds. 1990) PCR Protocols: A Guide to Methods and Applications Academic Press, San Diego, CA; and Dieffenbach and Dveksler (1995; eds.) PCR Primer: A Laboratory Manual Cold Spring Harbor Press, CSH, NY. Such will allow determination of a useful sequence to probe for a full length gene in a cDNA library. The TLR6 is a contiguous sequence in the genome, which may suggest that the other TLRs are also. Thus, PCR on genomic DNA may yield full length contiguous sequence, and chromosome walking methodology would then be applicable. Alternatively, sequence databases will contain sequence corresponding to portions of the described embodiments, or closely related forms, e.g., alternative splicing, etc. Expression cloning techniques also may be applied on cDNA libraries.

### IV. Tissue distribution of DTLRs

Message for each gene encoding these DTLRs has been detected. See Figures 5A-5F. Other cells and tissues will be assayed by appropriate technology, e.g., PCR, immunoassay, hybridization, or otherwise. Tissue and organ cDNA preparations are available, e.g., from Clontech, Mountain View, CA. Identification of sources of natural expression are useful, as described.

Southern Analysis: DNA (5 µg) from a primary amplified cDNA library is digested with appropriate restriction enzymes to release the inserts, run on a 1% agarose gel and transferred to a nylon membrane (Schleicher and Schuell, Keene, NH).

Samples for human mRNA isolation would typically include, e.g.: peripheral blood mononuclear cells (monocytes, T cells, NK cells, granulocytes, B cells), resting (T100); peripheral blood mononuclear cells, activated with anti-CD3 for 2, 6, 12 h pooled (T101); T cell, TH0 clone Mot 72, resting (T102); T cell, TH0 clone Mot 72, activated with anti-CD28 and anti-CD3 for 3, 6, 12 h pooled (T103); T cell, TH0 clone Mot 72, anergic treated with specific peptide for 2, 7, 12 h pooled (T104); T cell, TH1 clone HY06, resting (T107); T cell, TH1 clone HY06, activated with anti-CD28 and anti-CD3 for 3, 6, 12 h pooled (T108); T cell, TH1 clone HY06, anergic treated with specific peptide for 2, 6, 12 h pooled (T109); T cell, TH2 clone HY935, resting (T110); T cell, TH2 clone HY935, activated with anti-CD28 and anti-CD3 for 2, 7, 12 h pooled (T111); T cells CD4+CD45RO- T cells polarized 27 days in anti-CD28, IL-4, and anti IFN-γ, TH2 polarized, activated with anti-CD3 and anti-CD28 4 h (T116); T cell tumor lines Jurkat and Hut78, resting (T117); T cell clones, pooled AD130.2, Tc783.12, Tc783.13, Tc783.58, Tc782.69, resting (T118); T cell random γδ T cell clones, resting (T119); Splenocytes, resting (B100); Splenocytes, activated with anti-CD40 and IL-4 (B101); B cell EBV lines pooled WT49, RSB, JY, CVIR, 721.221, RM3, HSY, resting (B102); B cell line JY, activated with PMA and ionomycin for 1, 6 h pooled (B103); NK 20 clones pooled, resting (K100); NK 20 clones pooled, activated with PMA and ionomycin for 6 h (K101); NKL clone, derived from peripheral blood of LGL leukemia patient, IL-2 treated (K106); NK cytotoxic clone 640-A30-1, resting (K107); hematopoietic precursor line TF1, activated with PMA and ionomycin for 1, 6 h pooled (C100); U937 premonocytic line, resting (M100); U937 premonocytic line, activated with PMA and ionomycin for 1, 6 h pooled (M101); elutriated monocytes, activated with LPS, IFNγ, anti-IL-10 for 1, 2, 6, 12, 24 h pooled (M102); elutriated monocytes, activated with LPS, IFNγ, IL-10 for 1, 2, 6, 12, 24 h pooled (M103); elutriated monocytes, activated with LPS, IFNγ, anti-IL-10 for 4, 16 h pooled (M106); elutriated monocytes, activated with LPS, IFNγ, IL-10 for 4, 16 h pooled (M107); elutriated monocytes, activated LPS for 1 h (M108); elutriated monocytes, activated LPS for 6 h (M109); DC 70% CD1a+, from CD34+ GM-CSF, TNFα 12 days, resting (D101); DC 70% CD1a+, from CD34+ GM-CSF, TNFα 12 days, activated with PMA and ionomycin for 1 hr (D102); DC 70% CD1a+, from CD34+ GM-CSF, TNFα 12 days, activated with PMA and ionomycin for 6 hr (D103); DC 95% CD1a+, from CD34+ GM-CSF, TNFα 12 days FACS sorted, activated with PMA and ionomycin for 1, 6 h pooled (D104); DC 95% CD14+, ex CD34+ GM-CSF, TNFα 12 days FACS sorted, activated with PMA and ionomycin 1, 6 hr pooled (D105); DC CD1a+ CD86+, from CD34+ GM-CSF, TNFα 12 days FACS sorted, activated with PMA and ionomycin for 1, 6 h pooled (D106); DC from monocytes GM-CSF, IL-4 5 days, resting (D107); DC from monocytes GM-CSF, IL-4 5 days, resting (D108); DC from monocytes GM-CSF, IL-4 5 days, activated LPS 4, 16 h pooled (D109); DC from monocytes GM-CSF, IL-4 5 days, activated TNFα, monocyte supe for 4, 16 h pooled (D110); leiomyoma L11 benign tumor (X101); normal myometrium M5 (0115); malignant leiomyosarcoma GS1 (X103); lung fibroblast sarcoma line MRC5, activated with PMA and ionomycin for 1, 6 h pooled (C101); kidney epithelial carcinoma cell line CHA, activated with PMA and ionomycin for 1, 6 h pooled (C102); kidney fetal 28 wk male (O100); lung fetal 28 wk male (O101); liver fetal 28 wk male (O102); heart fetal 28 wk male (O103); brain fetal 28 wk male (O104); gallbladder fetal 28 wk male (O106); small intestine fetal 28 wk male (O107); adipose tissue fetal 28 wk male (O108); ovary fetal 25 wk female (O109); uterus fetal 25 wk female (O110); testes fetal 28 wk male (O111); spleen fetal 28 wk male (O112); adult placenta 28 wk (O113); and tonsil inflamed, from 12 year old (X100).

Samples for mouse mRNA isolation can include, e.g.: resting mouse fibroblastic L cell line (C200); Braf:ER (Braf fusion to estrogen receptor) transfected cells, control (C201); T cells, TH1 polarized (Mel14 bright, CD4+ cells from spleen, polarized for 7 days with IFN-γ and anti IL-4; T200); T cells, TH2 polarized (Mel14 bright, CD4+ cells from spleen, polarized for 7 days with IL-4 and anti-IFN-γ; T201); T cells, highly TH1 polarized (see Openshaw, et al. (1995) J. Exp. Med. 182:1357-1367; activated with anti-CD3 for 2, 6, 16 h pooled; T202); T cells, highly TH2 polarized (see Openshaw, et al. (1995) J. Exp. Med. 182:1357-1367; activated with anti-CD3 for 2, 6, 16 h pooled; T203); CD44- CD25+ pre T cells, sorted from thymus (T204); TH1 T cell clone D1.1, resting for 3 weeks after last stimulation with antigen (T205); TH1 T cell clone D1.1, 10 µg/ml ConA stimulated 15 h (T206); TH2 T cell clone CDC35, resting for 3 weeks after last stimulation with antigen (T207); TH2 T cell clone CDC35, 10 µg/ml ConA stimulated 15 h (T208); Mel14+ naive T cells from spleen, resting (T209); Mel14+ T cells, polarized to Th1 with IFN-γ/IL-12/anti-IL-4 for 6, 12, 24 h pooled (T210); Mel14+ T cells, polarized to Th2 with IL-4/anti-IFN-γ for 6, 13, 24 h pooled (T211); unstimulated mature B cell leukemia cell line A20 (B200); unstimulated B cell line CH12 (B201); unstimulated large B cells from spleen (B202); B cells from total spleen, LPS activated (B203); metrizamide enriched dendritic cells from spleen, resting (D200); dendritic cells from bone marrow, resting (D201); monocyte cell line RAW 264.7 activated with LPS 4 h (M200); bone-marrow macrophages derived with GM and M-CSF (M201); macrophage cell line J774, resting (M202); macrophage cell line J774 + LPS + anti-IL-10 at 0.5, 1, 3, 6, 12 h pooled (M203); macrophage cell line J774 + LPS + IL-10 at 0.5, 1, 3, 5, 12 h pooled(M204); aerosol challenged mouse lung tissue, Th2 primers, aerosol OVA challenge 7, 14, 23 h pooled (see Garlisi, et al. (1995) Clinical Immunology and Immunopathology 75:75-83; X206); Nippostrongulus-infected lung tissue (see Coffman, et al. (1989) Science 245:308-310; X200); total adult lung, normal (O200); total lung, rag-1 (see Schwarz, et al. (1993) Immunodeficiency 4:249-252; O205); IL-10 K.O. spleen (see Kuhn, et al. (1991) Cell 75:263-274; X201); total adult spleen, normal (O201); total spleen, rag-1 (0207); IL-10 K.O. Peyer's patches (0202); total Peyer's patches, normal (0210); IL-10 K.O. mesenteric lymph nodes (X203); total mesenteric lymph nodes, normal (O211); IL-10 K.O. colon (X203); total colon, normal (O212); NOD mouse pancreas (see Makino, et al. (1980) Jikken Dobutsu 29:1-13; X205); total thymus, rag-1 (O208); total kidney, rag-1 (O209); total heart, rag-1 (O202); total brain, rag-1 (O203); total testes, rag-1 (O204); total liver, rag-1 (O206); rat normal joint tissue (0300); and rat arthritic joint tissue (X300).

### V. Cloning of species counterparts of DTLRs

Various strategies are used to obtain species counterparts of these DTLRs, preferably from other primates. One method is by cross hybridization using closely related species DNA probes. It may be useful to go into evolutionarily similar species as intermediate steps. Another method is by using specific PCR primers based on the identification of blocks of similarity or difference between particular species, e.g., human, genes, e.g., areas of highly conserved or nonconserved polypeptide or nucleotide sequence. Alternatively, antibodies may be used for expression cloning.

### VI. Production of mammalian DTLR protein

An appropriate, e.g., GST, fusion construct is engineered for expression, e.g., in E. coli. For example, a mouse IGIF pGex plasmid is constructed and transformed into E. coli. Freshly transformed cells are grown in LB medium containing 50 µg/ml ampicillin and induced with IPTG (Sigma, St. Louis, MO). After overnight induction, the bacteria are harvested and the pellets containing the DTLR protein are isolated. The pellets are homogenized in TE buffer (50 mM Tris-base pH 8.0, 10 mM EDTA and 2 mM pefabloc) in 2 liters. This material is passed through a microfluidizer (Microfluidics, Newton, MA) three times. The fluidized supernatant is spun down on a Sorvall GS-3 rotor for 1 h at 13,000 rpm. The resulting supernatant containing the DTLR protein is filtered and passed over a glutathione-SEPHAROSE column equilibrated in 50 mM Tris-base pH 8.0. The fractions containing the DTLR-GST fusion protein are pooled and cleaved with thrombin (Enzyme Research Laboratories, Inc., South Bend, IN). The cleaved pool is then passed over a Q-SEPHAROSE column equilibrated in 50 mM Tris-base. Fractions containing DTLR are pooled and diluted in cold distilled H₂O, to lower the conductivity, and passed back over a fresh Q-Sepharose column, alone or in succession with an immunoaffinity antibody column.. Fractions containing the DTLR protein are pooled, aliquoted, and stored in the -70° C freezer.

Comparision of the CD spectrum with DTLR1 protein may suggest that the protein is correctly folded. See Hazuda, et al. (1969) J. Biol. Chem. 264:1689-1693.

### VII. Biological Assays with DTLRs

Biological assays will generally be directed to the ligand binding feature of the protein or to the kinase/phosphatase activity of the receptor. The activity will typically be reversible, as are many other enzyme actions.mediate phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738.

The family of interleukins 1 contains molecules, each of which is an important mediator of inflammatory disease. For a comprehensive review, see Dinarello (1996) "Biologic basis for interleukin-1 in disease" Blood 87:2095-2147. There are suggestions that the various Toll ligands may play important roles in the initiation of disease, particularly inflammatory responses. The finding of novel proteins related to the IL-1 family furthers the identification of molecules that provide the molecular basis for initiation of disease and allow for the development of therapeutic strategies of increased range and efficacy.

### VIII. Preparation of antibodies specific for, e.g., DTLR4

Inbred Balb/c mice are immunized intraperitoneally with recombinant forms of the protein, e.g., purified DTLR4 or stable transfected NIH-3T3 cells. Animals are boosted at appropriate time points with protein, with or without additional adjuvant, to further stimulate antibody production. Serum is collected, or hybridomas produced with harvested spleens.

Alternatively, Balb/c mice are immunized with cells transformed with the gene or fragments thereof, either endogenous or exogenous cells, or with isolated membranes enriched for expression of the antigen. Serum is collected at the appropriate time, typically after numerous further administrations. Various gene therapy techniques may be useful, e.g., in producing protein in situ, for generating an immune response.

Monoclonal antibodies may be made. For example, splenocytes are fused with an appropriate fusion partner and hybridomas are selected in growth medium by standard procedures. Hybridoma supernatants are screened for the presence of antibodies which bind to the desired DTLR, e.g., by ELISA or other assay. Antibodies which specifically recognize specific DTLR embodiments may also be selected or prepared.

In another method, synthetic peptides or purified protein are presented to an immune system to generate monoclonal or polyclonal antibodies. See, e.g., Coligan (1991) Current Protocols in Immunology Wiley/Greene; and Harlow and Lane (1989) Antibodies: A Laboratory Manual Cold Spring Harbor Press. In appropriate situations, the binding reagent is either labeled as described above, e.g., fluorescence or otherwise, or immobilized to a substrate for panning methods. Nucleic acids may also be introduced into cells in an animal to produce the antigen, which serves to elicit an immune response. See, e.g., Wang, et al. (1993) Proc. Nat'l. Acad. Sci. 90:4156-4160; Barry, et al. (1994) BioTechniques 16:616-619; and Xiang, et al. (1995) Immunity 2: 129-135.

### IX. Production of fusion proteins with, e.g., DTLR5

Various fusion constructs are made with DTLR5. This portion of the gene is fused to an epitope tag, e.g., a FLAG tag, or to a two hybrid system construct. See, e.g., Fields and Song (1989) Nature 340:245-246.

The epitope tag may be used in an expression cloning procedure with detection with anti-FLAG antibodies to detect a binding partner, e.g., ligand for the respective DTLR5. The two hybrid system may also be used to isolate proteins which specifically bind to DTLR5.

### X. Chromosomal mapping of DTLRs

Chromosome spreads are prepared. In situ hybridization is performed on chromosome preparations obtained from phytohemagglutinin-stimulated lymphocytes cultured for 72 h. 5-bromodeoxyuridine is added for the final seven hours of culture (60 µg/ml of medium), to ensure a posthybridization chromosomal banding of good quality.

An appropriate fragment, e.g., a PCR fragment, amplified with the help of primers on total B cell cDNA template, is cloned into an appropriate vector. The vector is labeled by nick-translation with ³H. The radiolabeled probe is hybridized to metaphase spreads as described in Mattei, et al. (1985) Hum. Genet. 69:327-331.

After coating with nuclear track emulsion (KODAK NTB₂), slides are exposed, e.g., for 18 days at 4° C. To avoid any slipping of silver grains during the banding procedure, chromosome spreads are first stained with buffered Giemsa solution and metaphase photographed. R-banding is then performed by the fluorochrome-photolysis-Giemsa (FPG) method and metaphases rephotographed before analysis.

Alternatively, FISH can be performed, as described above. The DTLR genes are located on different chromosomes. DTLR2 and DTLR3 are localized to human chromosome 4; DTLR4 is localized to human chromosome 9, and DTLR5 is localized to human chromosome 1. See Figures 4A-4D.

### XI. Structure activity relationship

Information on the criticality of particular residues is determined using standard procedures and analysis. Standard mutagenesis analysis is performed, e.g., by generating many different variants at determined positions, e.g., at the positions identified above, and evaluating biological activities of the variants. This may be performed to the extent of determining positions which modify activity, or to focus on specific positions to determine the residues which can be substituted to either retain, block, or modulate biological activity.

Alternatively, analysis of natural variants can indicate what positions tolerate natural mutations. This may result from populational analysis of variation among individuals, or across strains or species. Samples from selected individuals are analysed, e.g., by PCR analysis and sequencing. This allows evaluation of population polymorphisms.

### XI. Isolation of a ligand for a DTLR

A DTLR can be used as a specific binding reagent to identify its binding partner, by taking advantage of its specificity of binding, much like an antibody would be used. A binding reagent is either labeled as described above, e.g., fluorescence or otherwise, or immobilized to a substrate for panning methods.

The binding composition is used to screen an expression library made from a cell line which expresses a binding partner, i.e., ligand, preferably membrane associated. Standard staining techniques are used to detect or sort surface expressed ligand, or surface expressing transformed cells are screened by panning. Screening of intracellular expression is performed by various staining or immunofluorescence procedures. See also McMahan, et al. (1991) EMBO J. 10:2821-2832.

For example, on day 0, precoat 2-chamber permanox slides with 1 ml per chamber of fibronectin, 10 ng/ml in PBS, for 30 min at room temperature. Rinse once with PBS. Then plate COS cells at 2-3 x 10⁵ cells per chamber in 1.5 ml of growth media. Incubate overnight at 37° C.

On day 1 for each sample, prepare 0.5 ml of a solution of 66 µg/ml DEAE-dextran, 66 µM chloroquine, and 4 µg DNA in serum free DME. For each set, a positive control is prepared, e.g., of DTLR-FLAG cDNA at 1 and 1/200 dilution, and a negative mock. Rinse cells with serum free DME. Add the DNA solution and incubate 5 hr at 37° C. Remove the medium and add 0.5 ml 10% DMSO in DME for 2.5 min. Remove and wash once with DME. Add 1.5 ml growth medium and incubate overnight.

On day 2, change the medium. On days 3 or 4, the cells are fixed and stained. Rinse the cells twice with Hank's Buffered Saline Solution (HBSS) and fix in 4% paraformaldehyde (PFA)/glucose for 5 min. Wash 3X with HBSS. The slides may be stored at -80° C after all liquid is removed. For each chamber, 0.5 ml incubations are performed as follows. Add HBSS/saponin (0.1%) with 32 µl/ml of 1 M NaN₃ for 20 min. Cells are then washed with HBSS/saponin 1X. Add appropriate DTLR or DTLR/antibody complex to cells and incubate for 30 min. Wash cells twice with HBSS/saponin. If appropriate, add first antibody for 30 min. Add second antibody, e.g., Vector anti-mouse antibody, at 1/200 dilution, and incubate for 30 min. Prepare ELISA solution, e.g., Vector Elite ABC horseradish peroxidase solution, and preincubate for 30 min. Use, e.g., 1 drop of solution A (avidin) and 1 drop solution B (biotin) per 2.5 ml HBSS/saponin. Wash cells twice with HBSS/saponin. Add ABC HRP solution and incubate for 30 min. Wash cells twice with HBSS, second wash for 2 min, which closes cells. Then add Vector diaminobenzoic acid (DAB) for 5 to 10 min. Use 2 drops of buffer plus 4 drops DAB plus 2 drops of H₂O₂ per 5 ml of glass distilled water. Carefully remove chamber and rinse slide in water. Air dry for a few minutes, then add 1 drop of Crystal Mount and a cover slip. Bake for 5 min at 85-90° C.

Evaluate positive staining of pools and progressively subclone to isolation of single genes responsible for the binding.

Alternatively, DTLR reagents are used to affinity purify or sort out cells expressing a putative ligand. See, e.g., Sambrook, et al. or Ausubel, et al.

Another strategy is to screen for a membrane bound receptor by panning. The receptor cDNA is constructed as described above. The ligand can be immobilized and used to immobilize expressing cells. Immobilization may be achieved by use of appropriate antibodies which recognize, e.g., a FLAG sequence of a DTLR fusion construct, or by use of antibodies raised against the first antibodies. Recursive cycles of selection and amplification lead to enrichment of appropriate clones and eventual isolation of receptor expressing clones.

Phage expression libraries can be screened by mammalian DTLRs. Appropriate label techniques, e.g., anti-FLAG antibodies, will allow specific labeling of appropriate clones.

## Claims

1. A substantially pure or recombinant DTLR 6 polypeptide comprising an amino acid sequence which exhibits at least 90% sequence identity over a length of at least 20 amino acids to SEQ ID No:12.

2. The polypeptide of Claim 1 wherein said polypeptide exhibits at least 90% sequence identity over a length of at least 30 amino acids to SEQ ID No:12.

3. The polypeptide of Claim 1 or 2 wherein said polypeptide has Toll-like receptor activity.

4. A substantially pure or recombinant polypeptide comprising an amino acid sequence which exhibits at least 90% sequence identity to SEQ ID No: 12.

5. A substantially pure or recombinant polypeptide comprising an amino acid sequence which exhibits at least 90% sequence identity to SEQ ID No:12, wherein said polypeptide has Toll-like receptor activity.

6. A substantially pure or recombinant polypeptide comprising the amino acid sequence of SEQ ID No:12.

7. A fusion protein comprising the polypeptide of any one of Claims 1-6.

8. An antibody or antibody fragment which specifically binds to the polypeptide of any one of Claims 1-6.

9. A nucleic acid encoding the polypeptide of any one of Claims 1-7.

10. An expression vector comprising the nucleic acid of Claim 9.

11. A host cell comprising the vector of Claim 10.

12. A process for recombinantly producing a polypeptide comprising culturing the host cell of Claim 11 under conditions in which the polypeptide is expressed.

## Patentansprüche

1. Im wesentlichen reines oder rekombinantes DTLR 6-Polypeptid, das eine Aminosäuresequenz umfaßt, die mindestens 90% Sequenzidentität über eine Länge von mindestens 20 Aminosäuren zu SEQ ID NO.: 12 aufweist.

2. Polypeptid nach Anspruch 1, wobei das Polypeptid mindestens 90% Sequenzidentität über eine Länge von mindestens 30 Aminosäuren zu SEQ ID NO.: 12 aufweist.

3. Polypeptid nach Anspruch 1 oder 2, wobei das Polypeptid eine Toll-ähnliche Rezeptoraktivität aufweist.

4. Im wesentlichen reines oder rekombinantes Polypeptid, das eine Aminosäuresequenz umfaßt, die mindestens 90% Sequenzidentität zu SEQ ID NO.: 12 aufweist.

5. Im wesentlichen reines oder rekombinantes Polypeptid, das eine Aminosäuresequenz umfaßt, die mindestens 90% Sequenzidentität zu SEQ ID NO.: 12 aufweist und wobei das Polypeptid eine Toll-ähnliche Rezeptoraktivität aufweist.

6. Im wesentlichen reines oder rekombinantes Polypeptid, das die Aminosäuresequenz nach SEQ ID NO.: 12 umfaßt.

7. Fusionsprotein, welches das Polypeptid nach einem der Ansprüche 1 bis 6 umfaßt.

8. Antikörper oder Antikörperfragment, das spezifisch an das Polypeptid nach einem der Ansprüche 1 bis 6 bindet.

9. Nukleinsäure, die für das Polypeptid nach einem der Ansprüche 1 bis 7 kodiert.

10. Expressionsvektor, der die Nukleinsäure nach Anspruch 9 umfaßt.

11. Wirtszelle, die den Vektor nach Anspruch 10 umfaßt.

12. Verfahren zur rekombinanten Herstellung eines Polypeptids, bei dem man die Wirtszelle nach Anspruch 11 unter Bedingungen kultiviert, bei denen das Polypeptid exprimiert wird.

## Revendications

1. Polypeptide DTLR6 sensiblement pur ou recombinant, comprenant une séquence d'acides aminés qui est identique, pour au moins 90 % sur une longueur d'au moins 20 résidus d'acides aminés, à la Séquence n° 12.

2. Polypeptide conforme à la revendication 1, lequel polypeptide présente une séquence identique, pour au moins 90 % sur une longueur d'au moins 30 résidus d'acides aminés, à la Séquence n° 12.

3. Polypeptide conforme à la revendication 1 ou 2, lequel polypeptide possède une activité de récepteur de type Toll.

4. Polypeptide sensiblement pur ou recombinant, comprenant une séquence d'acides aminés qui est identique, pour au moins 90 %, à la Séquence n° 12.

5. Polypeptide sensiblement pur ou recombinant, comprenant une séquence d'acides aminés qui est identique, pour au moins 90 %, à la Séquence n° 12, lequel polypeptide possède une activité de récepteur de type Toll.

6. Polypeptide sensiblement pur ou recombinant, comprenant la séquence d'acides aminés présentée en tant que Séquence n° 12.

7. Protéine de fusion, comprenant un polypeptide conforme à l'une des revendications 1 à 6.

8. Anticorps ou fragment d'anticorps qui se lie spécifiquement à un polypeptide conforme à l'une des revendications 1 à 6.

9. Acide nucléique codant un polypeptide conforme à l'une des revendications 1 à 7.

10. Vecteur d'expression comprenant un acide nucléique conforme à la revendication 9.

11. Cellule hôte comprenant un vecteur conforme à la revendica-tion 10.

12. Procédé de production d'un polypeptide par voie recombinante, comportant le fait de cultiver une cellule hôte conforme à la revendication 11, dans des conditions dans lesquelles le polypeptide est exprimé.
